(19) Europäisches Patentamt / European Patent Office / Office européen des brevets

(11) EP 1 915 122 B1

(12) **EUROPÄISCHE PATENTSCHRIFT**

(45) Veröffentlichungstag und Bekanntmachung des Hinweises auf die Patenterteilung:
**04.12.2013 Patentblatt 2013/49**

(21) Anmeldenummer: **06764296.7**

(22) Anmeldetag: **02.08.2006**

(51) Int Cl.:
***A61Q 19/00*** (2006.01) ***A61Q 1/02*** (2006.01)
***A61Q 5/00*** (2006.01) ***A61Q 5/02*** (2006.01)
***A61Q 5/04*** (2006.01) ***A61Q 5/06*** (2006.01)
***A61Q 5/10*** (2006.01) ***A61Q 5/12*** (2006.01)
***A61Q 9/02*** (2006.01) ***A61Q 11/00*** (2006.01)
***A61Q 15/00*** (2006.01) ***A61Q 17/04*** (2006.01)
***A61K 8/81*** (2006.01) ***A61Q 19/10*** (2006.01)
***A61K 8/02*** (2006.01) ***A61K 8/04*** (2006.01)
***C08F 220/06*** (2006.01) ***C08F 220/14*** (2006.01)
***C08F 220/18*** (2006.01)

(86) Internationale Anmeldenummer:
**PCT/EP2006/064952**

(87) Internationale Veröffentlichungsnummer:
**WO 2007/017434 (15.02.2007 Gazette 2007/07)**

(54) **COPOLYMERE FÜR KOSMETISCHE ANWENDUNGEN**

COPOLYMERS FOR COSMETIC APPLICATIONS

COPOLYMERES POUR APPLICATIONS COSMETIQUES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **11.08.2005 EP 05107388**

(43) Veröffentlichungstag der Anmeldung:
**30.04.2008 Patentblatt 2008/18**

(73) Patentinhaber: **BASF SE**
**67056 Ludwigshafen (DE)**

(72) Erfinder:
- **NGUYEN KIM, Son**
  **69502 Hemsbach (DE)**
- **PIEROBON, Marianna**
  **67063 Ludwigshafen (DE)**
- **WINTER, Gabi**
  **67061 Ludwigshafen (DE)**
- **LAUBENDER, Matthias**
  **67105 Schifferstadt (DE)**
- **SCHWALM, Reinhold**
  **67157 Wachenheim (DE)**

(74) Vertreter: **Reinhardt, Jürgen et al**
**BASF Personal Care and Nutrition GmbH**
**Postfach 13 01 64**
**40551 Düsseldorf (DE)**

(56) Entgegenhaltungen:
**EP-A2- 0 100 890** **WO-A-97/00664**
**DE-A1- 19 732 902** **DE-A1- 19 838 852**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 915 122 B1

Printed by Jouve, 75001 PARIS (FR)

## Beschreibung

Gegenstand der vorliegenden Erfindung sind wässrige haut- oder haarkosmetische Zubereitungen, die wenigstens ein Polymer A enthalten, welches wenigstens einen Ester der (Meth)acrylsäure, wenigstens eine olefinisch ungesättigte, radikalisch polymerisierbare anionogene oder anionische Verbindung, wenigstens eine, keine Silikongruppen enthaltende, radikalisch polymerisierbare, olefinisch ungesättigte urethangruppenhaltige Verbindung und gegebenenfalls weitere radikalisch polymerisierbare Verbindungen einpolymerisiert enthält.

Stand der Technik

Strengere Umweltauflagen und wachsendes ökologisches Bewusstsein fordern zunehmend immer geringere Anteile an flüchtigen organischen Komponenten (englisch: volatile organic compounds, VOC) in beispielsweise Haarsprays.

Der VOC-Gehalt in Haarsprays wird im wesentlichen durch die nicht-wässrigen Lösungsmittel und die Treibmittel bestimmt. Daher wird gegenwärtig verstärkt anstelle von nicht-wässrigen Lösungsmittel auf Wasser als Lösungsmittel zurückgegriffen. Dieser Ersatz der organischen Lösungsmitteln bringt aber insbesondere auf dem Gebiet der Haarspray-Formulierungen einige Probleme mit sich.

So sind Formulierungen der aus dem Stand der Technik bekannten filmbildenden Polymere, die entsprechende VOC-Auflagen erfüllen, beispielsweise nicht oder erst nach weiterer Verdünnung sprühbar und somit nur bedingt für die Verwendung in Haarsprays geeignet. Dies wiederum führt zu Filmen, die mitunter nicht die notwendige mechanische Qualität und somit ungenügende Festigungswirkung und schlechten Halt für das Haar mit sich bringen.

Aufgabe und Lösung

Eine Aufgabe der vorliegenden Erfindung bestand darin, Polymere für haut- oder haarkosmetische Zubereitungen bereitzustellen, die als Pump- oder Aerosolspray in Lösungsmitteln oder Lösungsmittelgemischen mit erhöhtem Wasseranteil gut formulierbar sind, deren Formulierungen in Form von kleinen gleichmäßigen Tröpfchen gut sprühbar sind und während und nach dem Aufbringen möglichst wenig zur Schaumbildung neigen und deren dann gebildete Filme nicht klebrig sind und gute mechanische Eigenschaften aufweisen.

Neben der guten Verträglichkeit mit den üblichen haarkosmetischen Inhaltsstoffen sollen die aufgetragenen Polymere schnell trocknen und dem Haar gute Festigung und längeren Halt auch bei erhöhter Luftfeuchtigkeit verleihen, gute Auswaschbarkeit aufweisen und sich als optisch klare VOC-55-Aerosole (d.h. mit einem VOC-Anteil von höchstens 55 Gew.-%) formulieren lassen. Weiterhin soll das behandelte Haar gute haptische Eigenschaften wie insbesondere ein gutes Anfaßgefühl aufweisen.

Diese Aufgaben wurden überraschenderweise gelöst von wässrigen haut- oder haar-kosmetischen Zubereitungen gemäß Patentanspruch 1.

WO 97/000664 beschreibt Nagellack-Zubereitungen auf Basis von teilvernetzten Acrylharzen, die herstellbar sind durch Polymerisation von 0.1-15 Gew.-% difunktionellen Urethan(meth)acrylaten, 2-20 Gew.-% einer $\alpha,\beta$-olefinisch ungesättigten $C_3$-$C_{10}$-Carbonsäure, jeweils 8-75 Gew.-% eines Acryl- und eines Methacrylsäureesters.

DE-A 198 38 852 beschreibt haarkosmetische Zubereitungen enthaltend Polymere, die als Monomerbaustein radikalisch polymerisierbare, siloxangruppenhaltige Urethan(meth)acrylate, die a) wenigstens eine Verbindung, die mindestens ein aktives Wasserstoffatom und mindestens eine radikalisch polymerisierbare, $\alpha,\beta$-olefinisch ungesättigte Doppelbindung pro Molekül enthält, b) wenigstens ein Diisocyanat, c) wenigstens eine Verbindung, die zwei aktive Wasserstoffatome pro Molekül enthält, c) wenigstens eine Verbindung, die mindestens ein aktives Wasserstoffatom und mindestens eine Siloxangruppe pro Molekül enthält, eingebaut enthalten.

Im Rahmen der vorliegenden Erfindung umfasst der Ausdruck Alkyl geradkettige und verzweigte Alkylgruppen. Geeignete kurzkettige Alkylgruppen sind z.B. geradkettige oder verzweigte $C_1$-$C_{12}$-Alkyl-, bevorzugt $C_1$-$C_6$-Alkyl- und besonders bevorzugt $C_1$-$C_4$-Alkylgruppen. Dazu zählen insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, Octyl etc..

Geeignete längerkettige $C_8$-$C_{30}$-Alkyl- bzw. $C_8$-$C_{30}$-Alkenylgruppen sind geradkettige und verzweigte Alkyl- bzw. Alkenylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen, die gegebenenfalls zusätzlich einfach, zweifach oder mehrfach ungesättigt sein können. Dazu zählen z.B. n-Hexyl(en), n-Heptyl(en), n-Octyl(en), n-Nonyl (en), n-Decyl(en), n-Undecyl(en), n-Dodecyl(en), n-Tridecyl(en), n-Tetradecyl(en), n-Pentadecyl(en), n-Hexadecyl(en),

n-Heptadecyl(en), n-Octadecyl(en), n-Nonadecyl(en) etc..

Cycloalkyl steht vorzugsweise für $C_5$-$C_8$-Cycloalkyl, wie Cyclopentyl, Cyclohexyl, Cycloheptyl oder Cyclooctyl.

Der Ausdruck Heterocycloalkyl im Sinne der vorliegenden Erfindung umfasst gesättigte, cycloaliphatische Gruppen mit im Allgemeinen 4 bis 7, vorzugsweise 5 oder 6 Ringatomen, in denen 1 oder 2 der Ringkohlenstoffatome durch Heteroatome, ausgewählt aus den Elementen Sauerstoff, Stickstoff und Schwefel, ersetzt sind und die gegebenenfalls substituiert sein können, wobei im Falle einer Substitution, diese heterocycloaliphatischen Gruppen 1, 2 oder 3, vorzugsweise 1 oder 2, besonders bevorzugt 1 Substituenten, ausgewählt aus Alkyl, Aryl, COOR, $COO^-M^+$ und $NE^1E^2$, bevorzugt Alkyl, tragen können. Beispielhaft für solche heterocycloaliphatischen Gruppen seien Pyrrolidinyl, Piperidinyl, 2,2,6,6-Tetramethyl-piperidinyl, Imidazolidinyl, Pyrazolidinyl, Oxazolidinyl, Morpholidinyl, Thiazolidinyl, Isothiazolidinyl, Isoxazolidinyl, Piperazinyl-, Tetrahydrothiophenyl, Tetrahydrofuranyl, Tetrahydropyranyl, Dioxanyl genannt.

Aryl umfasst unsubstituierte und substituierte Arylgruppen und steht vorzugsweise für Phenyl, Tolyl, Xylyl, Mesityl, Naphthyl, Fluorenyl, Anthracenyl, Phenanthrenyl, Naphthacenyl und insbesondere für Phenyl, Tolyl, Xylyl oder Mesityl.

Substituierte Arylreste weisen vorzugsweise 1, 2, 3, 4 oder 5, insbesondere 1, 2 oder 3 Substituenten, ausgewählt unter Alkyl, Alkoxy, Carboxyl, Carboxylat, Trifluormethyl, - $SO_3H$, Sulfonat, $NE^1E^2$, Alkylen-$NE^1E^2$, Nitro, Cyano oder Halogen auf.

Hetaryl steht vorzugsweise für Pyrrolyl, Pyrazolyl, Imidazolyl, Indolyl, Carbazolyl, Pyridyl, Chinolinyl, Acridinyl, Pyridazinyl, Pyrimidinyl oder Pyrazinyl.

Arylalkyl steht für Gruppen, die sowohl Alkyl- als auch Arylreste enthalten, wobei diese Arylalkyl-Gruppen entweder über den Aryl- oder über den Alkylrest mit der sie tragenden Verbindung verknüpft sind.

Komponente a)

Komponente a) ist beispielsweise ausgewählt aus der Gruppe bestehend aus Methyl(meth)acrylat, Ethyl(meth) acrylat, n-Propyl(meth)acrylat, i-Propyl(meth)acrylat, Methylethacrylat, Ethylethacrylat, n-Propylethacrylat, i-Propylethacrylat, n-Butylethacrylat, tert.-Butylethacrylat, i-Butylethacrylat, n-Butyl(meth)acrylat, tert.-Butyl(meth)acrylat, i-Butyl(meth)acrylat, sec- Butyl(meth)acrylat, 2-Pentyl(meth)acrylat, 3-Pentyl(meth)acrylat, Isopentylacrylat, Neopentylacrylat, n-Octyl(meth)acrylat, 1,1,3,3-Tetramethylbutyl(meth)acrylat, Ethylhexyl(meth)acrylat, n-Nonyl(meth)acrylat, n-Decyl (meth)acrylat, n-Undecyl(meth)acrylat, Tridecyl(meth)acrylat, Myristyl(meth)acrylat, Pentadecyl(meth)acrylat, Palmityl (meth)acrylat, Heptadecyl(meth)acrylat, Nonadecyl(meth)acrylat, Arrachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat, Phenoxyethylacrylat, 4-t-Butylcyclohexylacrylat, Cyclohexyl(meth)acrylat, Ureido(meth)acrylat, Tetrahydrofurfuryl(meth)acrylat und deren Mischungen.

Komponente a) bevorzugt ausgewählt aus den Estern der (Meth)acrylsäure.

Komponente a) ist besonders bevorzugt ausgewählt aus Mischungen aus Methacrylaten und Acrylaten. Bevorzugte (Meth)acrylate sind $C_1$-$C_{10}$-Alkyl(meth)acrylate und insbesondere $C_1$-$C_4$- Alkyl(meth)acrylate.

Komponente a) ist ganz besonders bevorzugt ausgewählt aus der Gruppe bestehend aus Methylmethacrylat, Ethylmethacrylat, n-Propylmethacrylat, i-Propylmethacrylat, n-Butylmethacrylat, tert.-Butylmethacrylat, i-Butylmethacrylat, sec-Butylmethacrylat und deren Mischungen.

Noch weiter bevorzugt ist Komponente a) ausgewählt aus der Gruppe bestehend aus Methylmethacrylat, Ethylmethacrylat und deren Mischungen.

Am meisten bevorzugt als Komponente a) ist Methylmethacrylat (MMA).

Komponente b)

Komponente b) ist ausgewählt aus der Gruppe bestehend aus Acrylsäure, Methacrylsäure, und Mischungen daraus.

Polymer A enthält bevorzugt 4-30, besonders bevorzugt 10-30 und insbesondere 15-28 Gew.-% der Komponente b) einpolymerisiert.

Komponente c)

Als Komponente c) wird wenigstens eine, keine Silikongruppen enthaltende, olefinisch ungesättigte urethangruppenhaltige Verbindung verwendet. Unter olefinisch ungesättigten urethangruppenhaltigen Verbindungen werden im Rahmen der vorliegenden Erfindung Verbindungen verstanden, die wenigstens eine Urethangruppe und wenigstens eine polymerisierbare, bevorzugt radikalisch polymerisierbare olefinische Doppelbindung enthalten.

Als Komponente c) für die erfindungsgemäßen Polymere A geeignete olefinisch ungesättigte, Urethangruppen

enthaltende Prepolymere sind beispielsweise in P. K. T. Oldring (Hrsg.), Chemistry and Technology of UV- and EB-Formulations for Coatings, Inks and Paints, Vol. 11, SITA Technology, London, 1991, S. 73-123 genannt, worauf hiermit in vollem Umfang Bezug genommen wird.

Urethan(meth)acrylate sind dem Fachmann bekannt. Sie können erhalten werden durch Umsetzung eines Di- oder Polyisocyanates mit einem Kettenverlängerungsmittel aus der Gruppe der Diole/ Polyole und/oder Diamine/Polyamine und/oder Dithiole/Polythiole und/oder Alkanolamine und anschließende Umsetzung der restlichen freien Isocyanatgruppen mit mindestens einem Hydroxyalkyl(meth)acrylat oder Hydroxyalkylester anderer ethylenisch ungesättigter Carbonsäuren.Die Mengen an Kettenverlängerungsmittel, Di- bzw. Polyisocyanat und Hydroxyalkylester werden dabei bevorzugt so gewählt, dass

1. das Äquivalentverhältnis der NCO-Gruppen zu den reaktiven Gruppen des Kettenverlängerungsmittels (Hydroxyl-, Amino- bzw. Mercaptylgrupppen) zwischen 3:1 und 1:2, bevorzugt bei 2:1, liegt und

2. die OH-Gruppen der Hydroxyalkylester der ethylenisch ungesättigten Carbonsäuren in stöchiometrischen Mengen in Bezug auf die noch freien Isocyanatgruppen des Präpolymeren aus Isocyanat und Kettenverlängerungsmittel vorliegen. Außerdem ist es möglich, die Polyurethan(meth)acrylate herzustellen, indem zunächst ein Teil der Isocyanatgruppen eines Di- oder Polyisocyantes mit mindestens einem Hydroxyalkylester umgesetzt wird und die restlichen Isocyanatgruppen anschließend mit einem Kettenverlängerungsmittel umgesetzt werden. Auch in diesem Fall werden die Mengen an Kettenverlängerungsmittel, Isocyanat und Hydroxyalkylester so gewählt, dass das Äquivalentverhältnis der NCO-Gruppen zu den reaktiven Gruppen des Kettenverlängerungsmittels zwischen 3:1 und 1:2, bevorzugt bei 2:1 liegt und das Äquivalentverhältnis der restlichen NCO-Gruppen zu den OH-Gruppen des Hydroxyalkylesters 1:1 beträgt. Selbstverständlich sind auch sämtliche Zwischenformen dieser beiden Verfahren möglich. Beispielsweise kann ein Teil der Isocyanatgruppen eines Diisocyanates zunächst mit einem Diol umgesetzt werden, anschließend kann ein weiterer Teil der Isocyanatgruppen mit dem Hydroxalkylester und im Anschluß hieran die restlichen Isocyanatgruppen mit einem Diamin umgesetzt werden. Diese verschiedenen Herstellverfahren der Polyurethan(meth)acrylate sind bekannt (z.B. aus EP-A 0 203 161) und bedürfen daher keiner genauen Beschreibung.

Als Komponente c) geeignete Urethan(meth)acrylate sind in der DE-A 198 38 852 S.3, Z.45 bis S.9, Z.20 beschrieben, worauf hier in vollem Umfang Bezug genommen wird. Die dort mit d) bezeichnete Si-Komponente ist allerdings nicht Bestandteil derjenigen Urethan(meth)acrylate, die im Rahmen der vorliegenden Erfindung als Komponente c) eingesetzt werden.

Unter Urethan(meth)acrylaten werden Verbindungen verstanden, die

I. wenigstens eine Verbindung, die mindestens ein aktives Wasserstoffatom und mindestens eine radikalisch polymerisierbare, $\alpha,\beta$-ethylenisch ungesättigte Doppelbindung pro Molekül enthält,

II. wenigstens ein Diisocyanat und

III. wenigstens eine Verbindung, die zwei aktive Wasserstoffatome pro Molekül enthält,

eingebaut enthalten, und die Salze davon.

Komponente I)

Geeignete Verbindungen I) sind z.B. die üblichen, dem Fachmann bekannten Vinylverbindungen, die zusätzlich wenigstens eine gegenüber Isocyanatgruppen reaktive Gruppe aufweisen, die vorzugsweise ausgewählt ist unter Hydroxylgruppen sowie primären und sekundären Aminogruppen. Dazu zählen z.B. die Ester $\alpha,\beta$-ethylenisch ungesättigter Mono- und Dicarbonsäuren mit mindestens zweiwertigen Alkoholen. Als $\alpha,\beta$-ethylenisch ungesättigte Mono- und/oder Dicarbonsäuren können z. B. Acrylsäure, Methacrylsäure, Fumarsäure, Maleinsäure, Crotonsäure, Itaconsäure etc. und Gemische davon eingesetzt werden. Geeignete Alkohole sind übliche Diole, Triole und Polyole, z. B. 1,2-Ethandiol, 1,3-Propandiol, 1,4-Butandiol, 1,5-Pentandiol, 1,6-Hexandiol, 1,10-Decandiol, Diethylenglykol, 2,2,4-Trimethylpentandiol-1,5, 2,2-Dimethylpropandiol-1,3, 1,4-Dimethylolcyclohexan, 1,6-Dimethylolcyclohexan, Glycerin, Trimethylolpropan, Erythrit, Pentaerythrit, Sorbit etc. Bei den Verbinden I) handelt es sich dann z. B. um Hydroxymethyl(meth)acrylat, Hydroxyethylethacrylat, 2-Hydroxy-ethyl(meth)acrylat, 2-Hydroxypropyl(meth)acrylat, 3-Hydroxypropyl(meth)acrylat, 3-Hydroxybutyl(meth)acrylat, 4-Hydroxybutyl(meth)acrylat, 6-Hydroxyhexyl(meth)acrylat, 3-Hydroxy-2-ethylhexyl(meth) acrylat sowie um Di(meth)acrylsäureester des 1,1,1-Trimethylolpropans oder des Glycerins. Geeignete Verbindungen I sind weiterhin die Ester und Amide der zuvor genannten $\alpha,\beta$-ethylenisch ungesättigten Mono- und Dicarbonsäuren mit

$C_2$- bis $C_{12}$-Aminoalkoholen, die eine primäre oder sekundäre Aminogruppe aufweisen. Dazu zählen Aminoalkylacrylate und Aminoalkylmethacrylate und deren N-Monoalkylderivate, die z. B. einen N-$C_1$- bis $C_8$-Monoalkylrest tragen, wie Aminomethyl(meth)acrylat, Aminoethyl(meth)acrylat, N-Methylaminomethyl(meth)acrylat, N-Ethylaminomethyl(meth) acrylat, N-Ethylaminoethyl(meth)acrylat, N-(n-propyl)aminomethyl(meth)acrylat, N-Isopropylaminomethyl(meth)acrylat und bevorzugt tert.-Butylaminoethylacrylat und tert.-Butylaminoethylmethacrylat. Dazu zählen weiterhin N-(Hydroxy-$C_1$- bis $C_{12}$-alkyl)(meth)acrylamide, wie N-Hydroxymethyl-(meth)acrylamid, N-Hydroxyethyl(meth)acrylamid etc.

Geeignete Verbindungen I sind auch die Amide der zuvor genannten $\alpha,\beta$-ethylenisch ungesättigten Mono- und Dicarbonsäuren mit Di- und Polyaminen, die mindestens zwei primäre oder zwei sekundäre oder eine primäre und eine sekundäre Aminogruppe(n) aufweisen. Dazu zählen z. B. die entsprechenden Amide der Acrylsäure und Methacrylsäure, wie Aminomethyl(meth)acrylamid, Aminoethyl(meth)acrylamid, Aminopropyl(meth)acrylamid, Amino-n-butyl(meth) acrylamid, Methylaminoethyl(meth)acrylamid, Ethylaminoethyl(meth)acrylamid, Methylaminopropyl(meth)acrylamid, Ethylaminopropyl(meth)acrylamid, Methylamino-n-butyl(meth)acrylamid etc.

Geeignete Verbindungen I sind auch die Reaktionsprodukte von Epoxidverbindungen, die mindestens eine Epoxidgruppe aufweisen, mit den zuvor genannten $\alpha,\beta$-ethylenisch ungesättigten Mono- und/oder Dicarbonsäuren und deren Anhydriden. Geeignete Epoxidverbindungen sind z. B. Glycidylether, wie Bisphenol-A-diglycidylether, Resorcindiglycidylether, 1,3-Propandioldiglycidylether, 1,4-Butandioldiglycidylether, 1,5-Pentandioldiglycidylether, 1,6-Hexandioldiglycidylether etc..

Komponente II

Bei der Komponente II handelt es sich um übliche aliphatische, cycloaliphatische und/oder aromatische Diisocyanate, wie Tetramethylendiisocyanat, Hexamethylendiisocyanat, Methylendiphenyldiisocyanat, 2,4- und 2,6-Toluylendiisocyanat und deren Isomerengemische, o- und m-Xylylendiisocyanat, 1,5-Naphthylendiisocyanat, 1,4-Cyclohexylendiisocyanat, Dicyclohexylmethandiisocyanat und Mischungen davon. Bevorzugt handelt es sich bei der Komponente II um Hexamethylendiisocyanat, Isophorondiisocyanat, o- und m-Xylylendiisocyanat, Dicyclohexylmethandiisocyanat und Mischungen davon. Gewünschtenfalls können bis zu 3 Mol-% der genannten Verbindungen durch Triisocyanate ersetzt sein.

Komponente III

Geeignete Verbindungen der Komponente III sind z. B. Diole, Diamine, Aminoalkohole, und Mischungen davon. Das Molekulargewicht dieser Verbindungen liegt vorzugsweise in einem Bereich von etwa 56 bis 280. Gewünschtenfalls können bis zu 3 Mol-% der genannten Verbindungen durch Triole oder Triamine ersetzt sein.

Geeignete Diole III sind z. B. Ethylenglykol, Propylenglykol, Butylenglykol, Neopentylglykol, Cyclohexandimethylol, Di-, Tri-, Tetra-, Penta- oder Hexaethylenglykol und Mischungen davon. Bevorzugt werden Neopentylglykol und/oder Cyclohexandimethylol eingesetzt.

Geeignete Aminoalkohole III sind z. B. 2-Aminoethanol, 2-(N-Methylamino)ethanol, 3-Aminopropanol, 4-Aminobutanol, 1-Ethylaminobutan-2-ol, 2-Amino-2-methyl-1-propanol, 4-Methyl-4-aminopentan-2-ol etc..

Geeignete Diamine III sind z. B. Ethylendiamin, Propylendiamin, 1,4-Diaminobutan, 1,5-Diaminopentan und 1,6-Diaminohexan.

Bevorzugte Verbindungen der Komponente III sind Polymerisate mit einem zahlenmittleren Molekulargewicht im Bereich von etwa 300 bis 5000, bevorzugt etwa 400 bis 4000, insbesondere 500 bis 3000. Dazu zählen z. B. Polyesterdiole, Polyetherole, $\alpha,\omega$-Diaminopolyether und Mischungen davon. Vorzugsweise werden ethergruppenhaltige Polymerisate eingesetzt.

Bei den Polyetherolen III handelt es sich vorzugsweise um Polyalkylenglykole, z. B. Polyethylenglykole, Polypropylenglykole, Polytetrahydrofurane etc., Blockcopolymerisate aus Ethylenoxid und Propylenoxid oder Blockcopolymerisate aus Ethylenoxid, Propylenoxid und Butylenoxid, die die Alkylenoxideinheiten statistisch verteilt oder in Form von Blöcken einpolymerisiert enthalten.

Geeignete $\alpha,\omega$-Diaminopolyether III sind z. B. durch Aminierung von Polyalkylenoxiden mit Ammoniak herstellbar.

Geeignete Polytetrahydrofurane III können durch kationische Polymerisation von Tetrahydrofuran in Gegenwart von sauren Katalysatoren, wie z. B. Schwefelsäure oder Fluoroschwefelsäure, hergestellt werden. Derartige Herstellungsverfahren sind dem Fachmann bekannt.

Brauchbare Polyesterdiole III weisen vorzugsweise ein zahlenmittleres Molekulargewicht im Bereich von etwa 400 bis 5000, bevorzugt 500 bis 3000, insbesondere 600 bis 2000, auf.

Als Polyesterdiole kommen alle diejenigen in Betracht, die üblicherweise zur Herstellung von Polyurethanen eingesetzt werden, insbesondere solche auf Basis aromatischer Dicarbonsäuren, wie Terephthalsäure, Isophthalsäure, Phthalsäure, Na- oder K-Sulfoisophthalsäure etc., aliphatischer Dicarbonsäuren, wie Adipinsäure oder Bernsteinsäure

etc., und cycloaliphatischer Dicarbonsäuren, wie 1,2-, 1,3- oder 1,4-Cyclohexandicarbonsäure. Als Diole kommen insbesondere aliphatische Diole in Betracht, wie Ethylenglykol, Propylenglykol, 1,6-Hexandiol, Neopentylglykol, Diethylenglykol, Polyethylenglykole, Polypropylenglykole, 1,4-Dimethylolcyclohexan, sowie Poly(meth)acrylatdiole der Formel

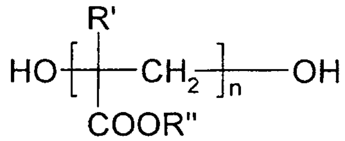

worin R' für H oder $CH_3$ steht und R" für $C_1$-$C_{18}$-Alkyl (insbesondere $C_1$-$C_{12}$- oder $C_1$-$C_8$-Alkyl) steht, die eine Molmasse von bis zu etwa 3000 aufweisen. Derartige Diole sind auf übliche Weise herstellbar und im Handel erhältlich (Tegomer®-Typen MD, BD und OD der Fa. Goldschmidt).

Bevorzugt sind Polyesterdiole auf Basis von aromatischen und aliphatischen Dicarbonsäuren und aliphatischen Diolen, insbesondere solche, bei denen die aromatische Dicarbonsäure 10 bis 95 Mol-%, insbesondere 40 bis 90 Mol-% und bevorzugt 50 bis 85 Mol-%, des gesamten Dicarbonsäureanteils (Rest aliphatische Dicarbonsäuren) ausmacht.

Besonders bevorzugte Polyesterdiole sind die Umsetzungsprodukte aus Phthalsäure/Diethylenglykol, Isophthalsäure/1,4-Butandiol, Isophthalsäure/Adipinsäure/1,6-Hexandiol, 5-$NaSO_3$-Isophthalsäure/Phthalsäure/Adipinsäure/1,6-Hexandiol, Adipinsäure/Ethylenglykol, Isophthalsäure/Adipinsäure/Neopentylglykol, Isophthalsäure/Adipinsäure/Neopentylglykol/Diethylenglykol/Dimethylolcyclohexan und 5-$NaSO_3$-Isophthalsäure/Isophthalsäure/Adipinsäure/Neopentylglykol/Diethylenglykol/ Dimethylolcyclohexan.

Die Verbindungen der Komponente III können einzeln oder als Mischungen eingesetzt werden. Weitere mögliche Bestandteile dieser Urethan(meth)acrylate sind der DE-A 198 38 852 S.5, Z.40 bis S.9, Z.20 zu entnehmen.

Als geeignete Komponente c) seien weiter genannt:

c1) Reaktionsprodukte der Umsetzung von Hydroxy(Meth)Acrylaten mit Diolen und/oder OH-terminierten Polyolen und/oder OH-terminierten Polyestern und/oder Diaminen und Diisocyanaten. Solche difunktionellen Urethanacrylat-Oligomere und ihre Herstellung sind beispielsweise beschrieben in WO 97/00664, S.5,Z.17 bis S.6, Z.8 und den entsprechenden Beispielen, worauf hier vollumfänglich Bezug genommen wird.

c2) Carbamoyloxycarboxylate der allgemeinen Formel I

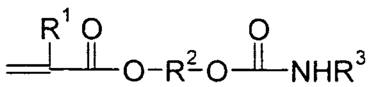

(I)

wobei

- $R^1$ H, Halogen oder C1-C8-Alkyl,
- $R^2$ gegebenenfalls subsituiertes $C_1$-$C_{12}$-Alkylen, -Arylen, -Alkylarylen oder-Arylalkylen, Polyoxyalkylen,
- $R^3$ $C_1$-$C_8$-Alkyl bedeuten.

Solche Carbamoyloxycarboxylate der allgemeinen Formel I sind in US 3,479,328 und US 3,674,838 offenbart, worauf hiermit in vollem Umfang Bezug genommen wird.

c3) die in der GB 1 443 715 offenbarten Divinylurethane der allgemeinen Formel II

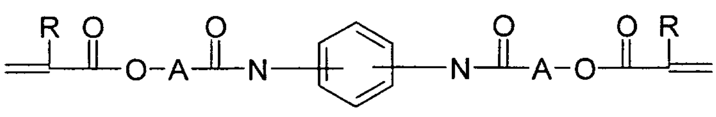

(II)

wobei

- R H oder Methyl
- A (Poly)Alkylenoxy bedeuten

und Vinylurethane der allgemeinen Formel III

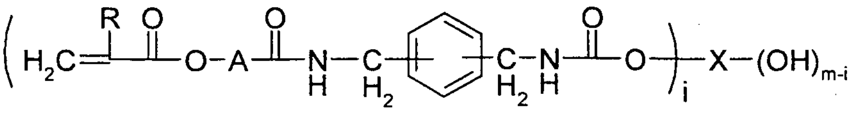 (III)

wobei R und A wie in Formel II und X und n wie in GB 1 443 715 S.2, Z.9-13 definiert. Die ebenfalls in der GB 1 443 715, auf die hiermit in vollem Umfang Bezug genommen wird, beschriebenen Vinylurethane sind weitere mögliche Komponenten c) der erfindungsgemäßen Polymere.

c4) die in der EP-A 0 036 813, auf die hiermit in vollem Umfang Bezug genommen wird, beschriebenen N-substituierten Carbamoyloxyalkylenoxyalkyl(meth)acrylate der allgemeinen Formel IV

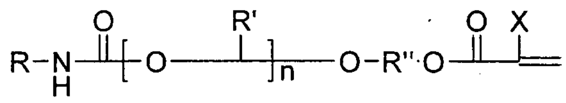 (IV)

wobei R, R', R" und X wie in EP-A 0 036 813, S.2, Z.13-28 definiert und n eine ganze Zahl von 0 bis 20, bevorzugt von 1 bis 6 und besonders bevorzugt von 1 bis 4 bedeutet.

c5) die aus der DE-A-4 007 146, auf die hiermit in vollem Umfang Bezug genommen wird, bekannten Urethanacrylat-Verbindungen, die erhältlich sind durch Umsetzung von Polyisocyanaten mit Hydroxyalkylacrylaten, gefolgt von einer Umsetzung mit primären oder sekundären Aminen.

c6) Produkte der Umsetzung von Isocyanaten mit Polyolen und Hydroxyalkylacrylaten wie beispielsweise in DE 27 26 041 A, US 4,260,703 und US 4,481,093 beschrieben und Produkte der Umsetzung von Isocyanaten mit Hydroxyalkylacrylaten wie in JP 63297369 und JP 59157112 beschrieben, worauf hiermit in vollem Umfang Bezug genommen wird.

c7) die in der EP-A 0 903 363, auf die hier hiermit in vollem Umfang Bezug genommen wird, beschriebenen Urethangruppen enthaltende Prepolymere, die durch ein Verfahren herstellbar sind, wobei man eine Isocyanatgruppen enthaltende Komponente A mit einer OH-Gruppen enthaltenden Komponente B umsetzt, wobei die Komponente A wenigstens eine trifunktionelle Isocyanatverbindung A1 und gegebenenfalls eine oder mehrere difunktionelle Isocyanatverbindungen A2 umfasst und die OH-Gruppen enthaltende Komponente B wenigstens eine olefinisch ungesättigte Verbindung B1 mit wenigstens einer reaktiven OH-Gruppe und gegebenenfalls davon verschiedene OH-Gruppen enthaltende Verbindungen B2 umfasst, wobei entweder die Komponente A zwei verschiedene Isocyanatverbindungen A1 oder eine Isocyanatverbindung A1 und wenigstens eine Isocyanatverbindung A2 umfasst oder die Komponente B wenigstens zwei verschiedene Verbindungen B2 umfasst.

c8) Polyurethanpolymerisate, welche A) 40 bis 80 Gew.-%, bezogen auf das Gesamtgewicht der Komponenten A) bis F), wenigstens eines hydroxyl gruppenhaltigen Präpolymers mit mindestens einer radikalisch oder photochemisch polymerisierbaren $\alpha,\beta$-ethylenisch ungesättigten Doppelbindung, wobei das Präpolymer

A) ein Reaktionsprodukt oder eine Mischung aus a) wenigstens einem Polyesteractylat und/oder Polyetheractylat und/oder Polyurethanacrylat und b) wenigstens einem Epoxyactylat ist,

B) 0,1 bis 20 Gew.-%, bezogen auf das Gesamtgewicht der Komponenten A) bis F), wenigstens einer Verbindung mit mindestens einer gegenüber Isocyanatgruppen reaktiven Hydroxyl- und/oder primären oder sekundären Aminogruppe und zusätzlich wenigstens einer polaren funktionellen Gruppe,

C) 0,1 bis 10 Gew.-%, bezogen auf das Gesamtgewicht der Komponenten A) bis F), wenigstens einer Verbindung, ausgewählt unter Diaminen, Polyaminen und Mischungen davon,

D) 0 bis 20 Gew.-%, bezogen auf das Gesamtgewicht der Komponenten A) bis F), wenigstens einer weiteren von A), B), C) und E) verschiedenen Verbindung mit mindestens zwei gegenüber Isocyanatgruppen reaktiven

Gruppen, wobei es sich um Hydroxylgruppen und Mischungen von Hydroxylgruppen und/oder primären oder sekundären Aminogruppen handelt,

E) 0 bis 20 Gew.-%, bezogen auf das Gesamtgewicht der Komponenten A) bis F), wenigstens einer Verbindung mit einer gegenüber Isocyanatgruppen reaktiven Gruppe,

F) 10 bis 50 Gew.-%, bezogen auf das Gesamtgewicht der Komponenten A) bis F), wenigstens eines Polyisocyanates einpolymerisiert enthält, und die Salze davon, die dadurch gekennzeichnet sind, dass die Summe der Hydroxylzahlen der Komponenten A) und D) in einem Bereich von 121 bis 300 mg KOH/g liegt.

Diese Polyurethanpolymerisate sind in der EP-A 0 942 022 beschrieben, auf die hier hiermit in vollem Umfang Bezug genommen wird.

c9) die in der EP-A 1 002 818 (auf die hier hiermit in vollem Umfang Bezug genommen wird) beschriebenen Umsetzungsprodukte von

a) Isocyanat-Trimeren(gemische) auf der Basis aliphatischer oder cycloaliphatischer Diisocyanate, welche zu bis 100 Mol-% aus Verbindungen vom Iminooxadiazindion-Strukturtyp der Formel A bestehen,

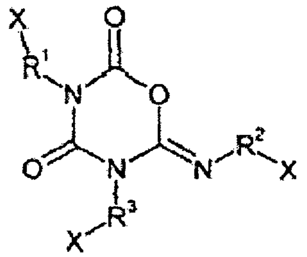

(A)

in welcher $R^1$, $R^2$ und $R^3$ unabhängig voneinander für gegebenenfalls verzweigte $C_4$-$C_{20}$-(Cyclo)Alkylen stehen, und
X für gleiche oder verschiedene Reste von Isocyanat oder von Isocyanat-Folgeprodukten, welche vom Iminooxadiazindion-, Isocyanurat-, Uretdion-, Urethan-, Allophanat-, Biuret- oder Oxadiazintrion-Strukturtyp steht und N-ständig die obengenannten Reste $R^1$, $R^2$ und $R^3$ tragen, mit

b) einer Alkoholkomponente, welche mindestens einen einwertig Hydroxyfunktionellen gegebenenfalls verzweigten $C_1$-$C_{12}$-Alkyl-Ester der (Meth)acrylsäure enthält.

c10) die Allylgruppen enthaltenden Polyurethane der allgemeinen Formel V, wie in der WO 01/72862, auf die hiermit in vollem Umfang Bezug genommen wird, offenbart:

$$R^1\text{-}[NHCO(OR)_y\,(OCH_2CH{=}CH_2)_m]_n \qquad (V)$$

Die Bedeutungen von R, $R^1$, y, m und n sind in der WO 01/72862 auf S.3, Z.29 bis S.4, Z. 10 beschrieben.

c11) die in der WO 04/050888, auf die hiermit in vollem Umfang Bezug genommen wird, beschriebenen urethangruppenhaltigen (Meth)acrylsäureester, die herstellbar sind durch die Umsetzung eines urethangruppenhaltigen Alkohols mit (Meth)acrylsäure oder einem Ester von (Meth)acrylsäure mit einem gesättigten Alkohol und gegebenenfalls Aufreinigung des Reaktionsgemisches, wobei man die Umsetzung in Gegenwart eines Enzyms (E) durchführt.

c12) die in der WO 98/06783, worauf an dieser Stelle in vollem Umfang Bezug genommen wird, insbesondere auf S.1, Z.22 bis S.2, Z.6 beschriebenen Urethan(meth)-acrylat-Oligomere.

c13) die in der DE 44 34 554 A1, worauf an dieser Stelle in vollem Umfang Bezug genommen wird, insbesondere auf S.2, Z.42 bis S.4, Z.27 beschriebenen Polyurethane.

c14) die in der WO 04/067599, auf die hiermit in vollem Umfang Bezug genommen wird, insbesondere auf S.10, Z. 24 bis S.12, Z.13 beschriebenen Urethan-(Meth)acrylat-Oligomere.

c15) die in der US 5240835, worauf an dieser Stelle in vollem Umfang Bezug genommen wird, beschriebenen Urethanacrylate, die herstellbar sind durch die Umesterung von Alkylacrylaten mit Alkoholen unter Katalyse eines Biokatalysators aus Corynebacterium oxydans.

c16) die in der WO 04/052843, worauf an dieser Stelle in vollem Umfang Bezug genommen wird, beschriebenen Carbamoyloxy(meth)acrylate, die herstellbar sind durch ein Verfahren, wie es auf S.3, Z.34 bis S.10, Z.28 der WO 04/052843 beschrieben ist.

c17) die Carbamyloxy(meth)acrylate, die beschrieben sind in der WO 94/25537 S.8, Z.29 bis S.9, Z.32, worauf an dieser Stelle in vollem Umfang Bezug genommen wird.

c18) die in der DE-A 102 46 112, worauf hiermit in vollem Umfang Bezug genommen wird, beschriebenen Polyisocyanatfolgeprodukte enthaltend mindestens eine Allophanatgruppe, die an dem über zwei Einfachbindungen gebundenen Sauerstoffatom der Allophanatgruppe mindestens eine Acrylat-, Methacrylat- oder Vinylether-Doppelbindung trägt, dadurch gekennzeichnet, dass ein Polyisocyanat oder Polyisocyanatfolgeprodukt enthaltend mindestens eine Oxadiazintriongruppe mit einem Acrylat-, Methacrylat- oder Vinylether-Doppelbindung enthaltenden Alkohol bei Temperaturen zwischen -20 bis 100°C reagiert.

c19) Die WO 00/39183, worauf hiermit in vollem Umfang Bezug genommen wird, beschreibt Verbindungen mit Isocyanatgruppen oder verkappten Isocyanatgruppen, Allophanatgruppen und radikalisch polymerisierbaren C-C-Doppelbindungen, wobei die C-C-Doppelbindungen durch eine direkt daran gebundene Carbonylgruppe oder ein O-Atom in Etherfunktion aktiviert sind (Aktivierte Doppelbindungen), abgeleitet von Polyisocyanaten und Alkoholen A, die neben der Alkoholgruppe noch eine Aktivierte Doppelbindung tragen.
Erfindungsgemäß werden diese Verbindungen bevorzugt mit Alkoholen ROH, die nur eine OH-Gruppe tragen oder mit Aminen $RNH_2$ oder RR'NH in mindestens der
Menge umgesetzt, die ausreicht, um alle Isocyanatgruppen und verkappten Isocyanatgruppen in Urethan- oder Harnstoffgruppen zu überführen.
Dabei bedeuten R und R' unabhängig voneinander $C_1$-$C_{12}$-Alkyl, -Aryl, -Alkylaryl oder -Arylalkyl, Polyoxyalkylen, wobei die Reste gegebenenfalls mit Hydroxylgruppen funktionalisiert sein können.
Bevorzugte Alkohole für diese Umsetzung sind $C_1$-$C_{12}$-, insbesondere $C_1$-$C_4$-Alkanole wie beispielsweise Methanol, Ethanol, n-Propanol, i-Propanol, n-Butanol, i-Butanol, sek-Butanol, tert.-Butanol.
Bevorzugte Amine für diese Umsetzung sind $C_1$-$C_{12}$-, insbesondere $C_1$-$C_4$-(Di)Alkylamine, (Di)Alkanolamine, Alkylalkanolamine wie beispielsweise Ethylamin, Butylamin, Diethylamin, Ethanolamin, Diethanolamin, 2-Amino-2-Methylpropanol.
Es werden auf diese Weise beispielsweise Verbindungen der folgenden allgemeinen Formeln erhalten:
Aus der Umsetzung mit Alkoholen:

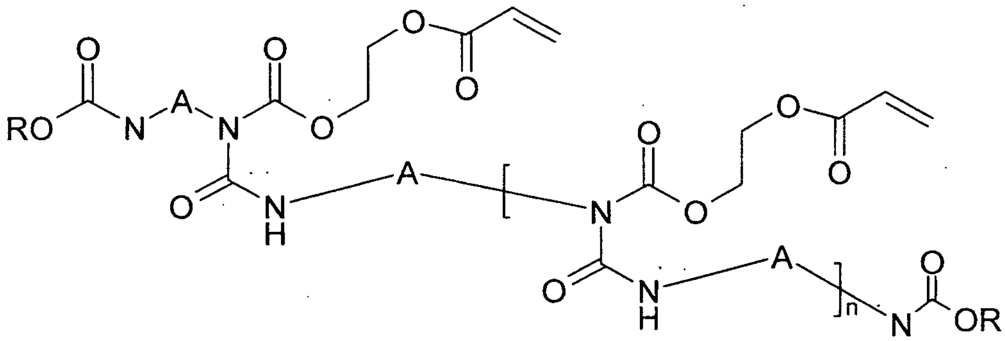

Aus der Umsetzung mit Aminen:

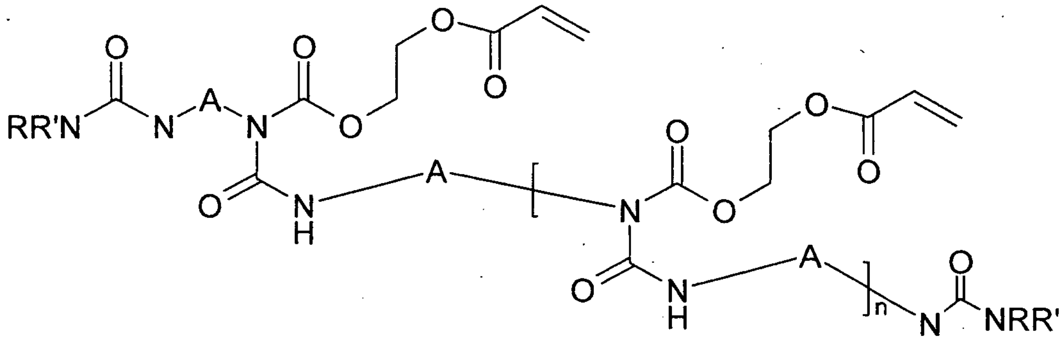

wobei

| | |
|---|---|
| R | $C_1$-$C_{12}$-Alkyl, -Aryl, -Alkylaryl oder -Arylalkyl, Polyoxyalkylen, gegebenenfalls mit Hydroxylgruppen funktionalisiert |
| R' | H, $C_1$-$C_{12}$-Alkyl, -Aryl, -Alkylaryl oder -Arylalkyl, Polyoxyalkylen, gegebenenfalls mit Hydroxylgruppen funktionalisiert |
| n | 0 bis 10, bevorzugt 0 bis 5, besonders bevorzugt 0 bis 2 |
| A | $C_1$-$C_{12}$-Alkylen, -Arylen, -Alkylarylen oder -Arylalkylen, Polyoxyalkylen |

bedeuten und Mischungen davon.
Selbstverständlich können auch die entsprechenden Methacrylat-Derivate dieser Verbindungen als Komponente c) eingesetzt werden.
Geeignete Komponenten c) sind beispielsweise auch

c20) N-butyl-2-hydroxyethyl-carbamate (CAS 63225-53-6) der Formel

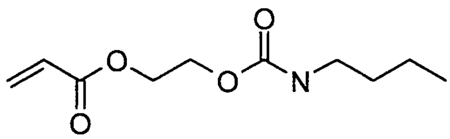

(wie beispielsweise als Ebecryl®CL 1039 (UCB) kommerziell erhältlich) und das entsprechende Methacrylsäure-Derivat,

c21) N-methyl-2-hydroxyethyl-carbamate (CAS 52607-81-5) der Formel

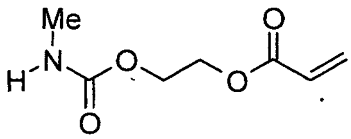

und das entsprechende Methacrylsäure-Derivat,

c22) eine der oder eine Mischung der beiden Komponenten der folgenden Formeln (die Mischung wird hierin als Monomer C22 bezeichnet (siehe auch die Beispiele)):

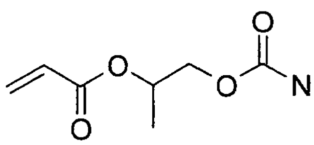

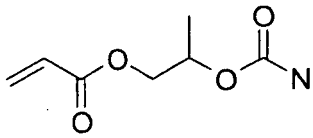

und die entsprechenden Methacrylsäure-Derivate,

c23) eine der oder eine Mischung der beiden Komponenten der folgenden Formeln

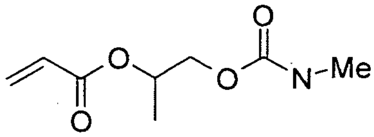

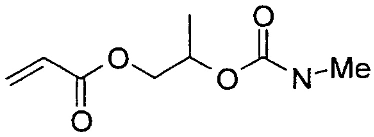

und die entsprechenden Methacrylsäure-Derivate,

c24) Verbindung der folgenden Formel

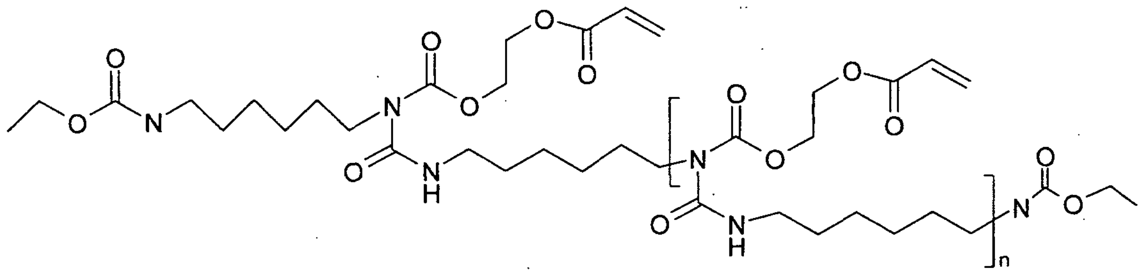

wobei n 0 bis 10, bevorzugt 0 bis 4, besonders bevorzugt 0 bis 2 bedeutet, und die entsprechenden Methacrylsäure-Derivate.

c25) Diurethandimethacrylat 7,7,9-(bzw. 7,9,9-)Trimethyl-4,13-dioxo-3,14-dioxa-5,12-diaza-hexadecan-1,16-dioldimethacrylat (CAS 72869-86-4), das beispielsweise als PLEX®6661-O (Degussa) kommerziell erhältlich ist.
Als Komponente c) geeignete Polyurethan(meth)acrylate wie Polyurethan-mono-, -di-, -tri-, -tetra, -penta oder -hexa-(meth)acrylate sind kommerziell erhältlich unter den Marken Laromer® (BASF), Photomer® (Cognis), Sartomer® (Sartomer) oder Ebecryl® (UCB).
Sie können in reiner Form (ohne Verdünnungsmittel), als Lösungen in Lösungsmitteln wie Ethanol oder Butylacetat oder als Lösungen in reaktiven Verdünnungsmitteln (wie beispielsweise Tripropylenglykoldiacrylat (TPGDA), Hexandioldiacrylat (HDDA), Dipropylenglykoldiacrylat (DPGDA), Trimethylolpropanformalmonoacrylat (Laromer®LR 8887), Trimethylolpropantriacrylat (TMPTA), propoxyliertes Glyceryltriacrylat (GPTA), Ethoxyliertes Trimethylolpropantriacrylat (EO3TMPTA), Ethoxyethoxyethylacrylat (EOEOEA), PEG 400 diacrylat (PEG400DA), Isobornylacrylat (IBOA), Propoxyliertes Neopentylglycoldiacrylat (P02NPGDA), 2-Phenoxyethylacrylat (POEA), Butandioldiacrylat (BDDA), Butandiolacrylat (BDMA), Dihydrodicyclopentadienylacrylat (DCPA), Triethylenglycoldivinylether, Ethyldigycolacrylat (EDGA), Laurylacrylat (LA), 4-t-butylcyclohexylacrylat (TBCH) oder als wässrige Emulsionen eingesetzt werden.
Solche Polyurethan(meth)acrylate sind:

Laromer®-Typen Laromer-Typen LR 8949, LR 9005, LR 8983, UA 19 T, UA 9030V, UA 9028V, UA 9029V, UA 9033V, UA 9031V und LR 8987,

Photomer®-Typen 6891, 6892, 6893-20R, 6572, 6010, 6019, 6184, 6210, 6217, 6230, 6363 und 6008

Sartomer®CN-Typen wie beispielsweise

die aliphatischen Urethanacrylate CN 934 CN 934X50, CN 944B85, CN 945A60, CN 945B85, CN 953B70, CN 961 E75, CN 961 H81, CN 962, CN 963A80, CN 963B80, CN 963E75, CN 963E80, CN 963J85, CN 964, CN 964A85, CN 964B85, CN 964H90, CN 964E75, CN 965, CN 965A80, CN 966A80, CN 966B85, CN 966H90, CN 966I80, CN 966J75, CN 966R60, CN 968, CN 982E75, CN 982P90, CN 983, CN 983B88, CN 984, CN 985B88

und die aromatischen Urethanacrylate CN 970A60, CN 970E60, CN 970H75, CN 971A80, CN 972, CN 973A80, CN 973H85, CN 973J75, CN 975, CN 977C70, CN 978, CN 980, CN 980M50, CN 981, CN 981A75, CN 981B88, CN 982A75, CN 982B88

Ebecryl®-Typen wie beispielsweise 220, 230, 244, 264, 265, 270.

Als Komponente c) sind besonders bevorzugt Carbamoyloxycarboxylate der allgemeinen Formel VIII

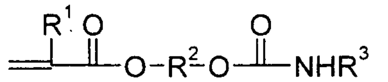 (VIII)

wobei
$R^1$ H, Halogen, $C_1$-$C_8$-Alkyl, bevorzugt H oder Methyl,
$R^2$ gegebenenfalls subsituiertes $C_1$-$C_{12}$-Alkylen, -Arylen, -Alkylarylen oder-Arylalkylen, gegebenenfalls hydroxysubstituiertes Polyoxyalkylen,
$R^3$ H, $C_1$-$C_8$-Alkyl bedeuten.
Generell sind solche Komponenten c) bevorzugt, die höchstens 4, bevorzugt höchstens 3 und besonders bevorzugt höchstens 2 radikalische polymerisierbare Doppelbindungen pro Molekül enthalten.
Polymer A enthält bevorzugt 0,1-20, besonders bevorzugt 0,5-10 und am meisten bevorzugt 0,5-5 Gew.-% der Komponente c) einpolymerisiert.
In einer Ausführungsform der Erfindung enthält Polymer A

a) Methylmethacrylat,
b) Methacrylsäure und/oder Acrylsäure,
c) N-butyl-2-hydroxyethyl-carbamate der allgemeinen Formel

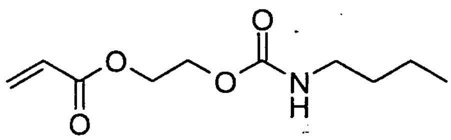

einpolymerisiert.

In einer weiteren Ausführungsform der Erfindung enthält Polymer A

a) Ethylmethacrylat,
b) Methacrylsäure und/oder Acrylsäure,
c) N-butyl-2-hydroxyethyl-carbamate der allgemeinen Formel

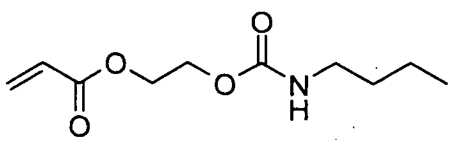

einpolymerisiert.

In einer weiteren Ausführungsform der Erfindung enthält Polymer A

a) Methylmethacrylat,
b) Methacrylsäure und/oder Acrylsäure,
c) eine Mischung der beiden Komponenten der folgenden Formeln

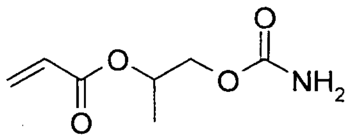

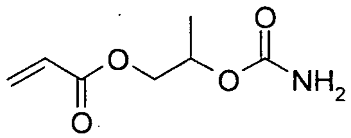

einpolymerisiert.

In einer weiteren Ausführungsform der Erfindung enthält Polymer A

a) Ethylmethacrylat,
b) Methacrylsäure und/oder Acrylsäure,
c) eine Mischung der beiden Komponenten der folgenden Formeln

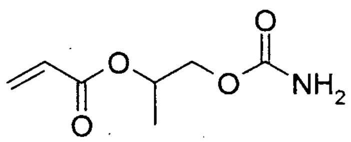

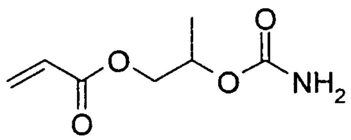

einpolymerisiert.

Komponente d)

Als Komponente d) kommen generell alle von den Komponenten a) bis c) verschiedenen, radikalisch polymerisierbaren ungesättigten Verbindungen in Betracht, die mit den Komponenten a) bis c) copolymerisiert werden können.

Bevorzugte Komponenten d) sind

d1) von d2) verschiedene amidgruppenhaltige Verbindungen,
d2) (Meth)acrylamide,
d3) kationogene Monomere,
d4) kationische Monomere und
d5) Verbindungen mit wenigstens 2 polymerisierbaren Doppelbindungen, die üblicherweise auch als Vernetzer bezeichnet werden.

Komponenten d1)

Die amidgruppenhaltigen Verbindungen d1) sind bevorzugt ausgewählt aus von d2) verschiedenen Verbindungen der allgemeinen Formel VI

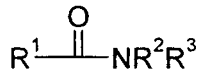 (VI)

wobei $R^1$ für eine Gruppe der Formel $CH_2=CR^4$- mit $R^4$ = H oder $C_1$-$C_4$-Alkyl steht und $R^2$ und $R^3$ unabhängig voneinander für H, Alkyl, Cycloalkyl, Heterocycloalkyl, Aryl oder Hetaryl stehen oder $R^2$ und $R^3$ gemeinsam mit dem Stickstoffatom, an das sie gebunden sind, für einen fünf- bis achtgliedrigen Stickstoff-Heterocyclus stehen oder
$R^2$ für eine Gruppe der Formel $CH_2=CR^4$- steht und $R^1$ und $R^3$ unabhängig voneinander für H, Alkyl, Cycloalkyl, Heterocycloalkyl, Aryl oder Hetaryl stehen oder $R^1$ und $R^3$ gemeinsam mit der Amidgruppe, an die sie gebunden sind für ein Lactam mit 5 bis 8 Ringatomen stehen.

Bevorzugt als Komponente d1) sind N-Vinyllactame. Als Komponente d1) eignen sich unsubstituierte N-Vinyllactame und N-Vinyllactamderivate, die z.B. einen oder mehrere $C_1$-$C_6$-Alkylsubstituenten, wie Methyl, Ethyl, n-Propyl, Isopropyl, n-Butyl, sec.-Butyl, tert.-Butyl etc., aufweisen können. Dazu zählen z.B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam etc. sowie deren Mischungen.

Bevorzugte Komponenten d1) sind diejenigen, für die in Formel VI $R^2$ für $CH_2=CH$- und $R^1$ und $R^3$ gemeinsam mit der Amidgruppe, an die sie gebunden sind für ein Lactam mit 5 Ringatomen stehen.

Besonders bevorzugt werden N-Vinylpyrrolidon, N-Vinylcaprolactam, N-Vinylformamid, Acrylamid oder deren Mischungen eingesetzt, wobei N-Vinylpyrrolidon am meisten bevorzugt ist.

Komponente d2)

Geeignete Komponenten d2) sind die von d3) und d4) verschiedenen Amide der (Meth)acrylsäure. Solche Amide sind beispielsweise (Meth)acrylamid, N-Methyl-(meth)acrylamid, N-Ethyl(meth)acrylamid, N-n-Propyl-(meth) acrylamid, N-i-Propyl-(meth)acrylamid, N-(n-Butyl)methacrylamid, N-(sek.-Butyl)methacrylamid, N-(tert.-Butyl)methacrylamid, N-(n-Pentyl)(meth)acrylamid, N-(n-Hexyl)(meth)acrylamid, N-(n-Heptyl)(meth)acrylamid, N-(n-Octyl)(meth) acrylamid, N-(tert.-Octyl)(meth)acrylamid N-(1,1,3,3-Tetramethylbutyl)(meth)acrylamid, N-Ethylhexyl(meth)acrylamid, N-(n-Nonyl)(meth)acrylamid, N-(n-Decyl)(meth)acrylamid, N-(n-Undecyl)(meth)acryl-amid, N-Tridecyl(meth)acrylamid, N-Myristyl(meth)acrylamid, N-Pentadecyl(meth)acrylamid, N-Palmityl(meth)acrylamid, N-Heptadecyl(meth)acrylamid, N-Nonadecyl(meth)-acrylamid, N-Arrachinyl(meth)acrylamid, N-Behenyl(meth)acrylamid, N-Lignocerenyl-(meth)acrylamid, N-Cerotinyl(meth)acrylamid, N-Melissinyl(meth)acrylamid, N-Palmitoleinyl(meth)acrylamid, N-Oleyl(meth)acrylamid, N-Linolyl(meth)acrylamid, N-Linolenyl(meth)acrylamid, N-Stearyl(meth)acrylamid, N-Lauryl(meth)acrylamid.

Geeignete Komponenten d2) sind weiterhin 2-Hydroxyethylacrylamid, 2-Hydroxyethylmethacrylamid, 2-Hydroxyethylethacrylamid, 2-Hydroxypropylacrylamid, 2-Hydroxypropylmethacrylamid, 3-Hydroxypropylacrylamid, 3-Hydroxypropylmethacrylamid, 3-Hydroxybutylacrylamid, 3-Hydroxybutylmethacrylamid, 4-Hydroxybutylacrylamid, 4-Hydroxybutylmethacrylamid, 6-Hydroxyhexylacrylamid,
6-Hydroxyhexylmethacrylamid, 3-Hydroxy-2-ethylhexylacrylamid und
3-Hydroxy-2-ethylhexylmethacrylamid.

Komponenten d3) und d4)

Die Komponenten d3) und d4) sind Monomere, die wenigstens eine kationogene und/oder kationische Gruppe pro Molekül enthalten.

Bevorzugt handelt es sich bei den kationogenen bzw. kationischen Gruppen um stickstoffhaltige Gruppen, wie primäre, sekundäre und tertiäre Aminogruppen sowie quaternäre Ammoniumgruppen. Vorzugsweise handelt es sich bei den stickstoffhaltigen Gruppen um tertiäre Aminogruppen.

Bevorzugt werden die Komponenten d3) und d4) in nicht geladener Form zur Polymerisation eingesetzt. Geeignet ist jedoch auch ein Einsatz in geladener Form.

Geladene kationische Gruppen lassen sich beispielsweise aus den Aminstickstoffatomen durch Protonierung, beispielsweise mit einwertigen oder mehrwertigen Carbonsäuren, wie Milchsäure oder Weinsäure, oder Mineralsäuren, wie Phosphorsäure, Schwefelsäure und Salzsäure erzeugen.

Vorzugsweise sind die Komponenten d3) und d4) ausgewählt ist unter

- Estern α,β-olefinisch ungesättigter Mono- und Dicarbonsäuren mit Aminoalkoholen, welche am Aminstickstoff mono- oder dialkyliert sein können,
- Amiden α,β-olefinisch ungesättigter Mono- und Dicarbonsäuren mit Diaminen, welche mindestens eine primäre oder sekundäre Aminogruppe aufweisen,
- N,N-Diallylamin,
- N,N-Diallyl-N-alkylaminen und deren Derivaten,
- vinyl- und allylsubstituierten Stickstoffheterocyclen,
- vinyl- und allylsubstituierten heteroaromatischen Verbindungen und
- Mischungen davon.

Geeignet als Komponenten d3) und d4) sind auch die Ester von α,β-olefinisch ungesättigten Mono- und Dicarbonsäuren mit Aminoalkoholen. Bevorzugte Aminoalkohole sind $C_2$-$C_{12}$-Aminoalkohole, welche am Aminstickstoff $C_1$-$C_8$-mono- oder -dialkyliert sind. Als Säurekomponente dieser Ester eignen sich z. B. Acrylsäure, Methacrylsäure, Fumarsäure, Maleinsäure, Itaconsäure, Crotonsäure, Maleinsäureanhydrid, Monobutylmaleat und Gemische davon. Bevorzugt werden Acrylsäure, Methacrylsäure und deren Gemische eingesetzt.

Besonders bevorzugt als Komponenten d3) und d4) sind N-Methylaminoethyl-(meth)acrylat, N-Ethylaminoethyl (meth)acrylat, N-(n-Propyl)aminoethyl(meth)acrylat, N-(n-Butyl)aminoethyl(meth)acrylat, N-(tert.-Butyl)aminoethyl(meth)acrylat, N,N-Dimethylaminomethyl(meth)acrylat, N,N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat und N,N-Dimethylaminocyclohexyl(meth)acrylat. Insbesondere werden als Komponenten d3) und d4) N-(tert.-Butyl)aminoethylacrylat und N-(tert.-Butyl)aminoethylmethacrylat eingesetzt.

Geeignete Komponenten d3) und d4) sind weiterhin die Amide der zuvor genannten α,β-olefinisch ungesättigten Mono- und Dicarbonsäuren mit Diaminen, welche mindestens eine primäre oder sekundäre Aminogruppe aufweisen.

Bevorzugt sind Diamine, die eine tertiäre und eine primäre oder sekundäre Aminogruppe aufweisen. Bevorzugt werden als Komponenten d3) und d4) N-[2-(dimethylamino)ethyl]acrylamid, N-[2-(dimethylamino)ethyl]methacrylamid, N-[3-(dimethylamino)propyl]acrylamid, N-[3-(dimethylamino)propyl]methacrylamid, N-[4-(dimethylamino)butyl]acrylamid, N-[4-(dimethylamino)-butyl]methacrylamid, N-[2-(diethylamino)ethyl]acrylamid, N-[4-(dimethylamino)cyclohexyl] acrylamid und N-[4-(dimethylamino)cyclohexyl]methacrylamid eingesetzt.

Besonders bevorzugt werden N-[3-(dimethyl-amino)propyl]acrylamid und/oder N-[3-(dimethylamino)propyl] methacrylamid eingesetzt.

Geeignete Komponenten d3) und d4) sind weiterhin N,N-Diallylamine und N,N-Diallyl-N-alkylamine und deren Säureadditionssalze. Alkyl steht dabei vorzugsweise für $C_1$-$C_{24}$-Alkyl. Bevorzugt ist N,N-Diallyl-N-methylamin.

Geeignete Komponenten d3) und d4) sind weiterhin vinyl- und allylsubstituierte Stickstoffheterocyclen, wie N-Vinylimidazol, N-Vinylimidazol-Derivate, z. B. N-Vinyl-2-methylimidazol, vinyl- und allylsubstituierte heteroaromatische Verbindungen, wie 2-und 4-Vinylpyridin, 2- und 4-Allylpyridin, und die Salze davon.

Geeignete Komponenten d3) und d4) sind auch N-Vinylimidazole der allgemeinen Formel VII, worin $R^1$ bis $R^3$ für Wasserstoff, $C_1$-$C_4$-Alkyl oder Phenyl steht

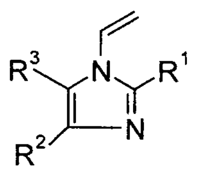

(VII)

Beispiele für Verbindungen der allgemeinen Formel VII sind folgender Tabelle 1 zu entnehmen:

Tabelle 1

| $R^1$ : | $R^2$ : | $R^3$ : |
|---|---|---|
| H | H | H |

(fortgesetzt)

| $R^1$ : | $R^2$ : | $R^3$ : |
|---|---|---|
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |
| Me = Methyl; Ph = Phenyl | | |

Besonders bevorzugt sind die Komponenten d3) und d4) ausgewählt unter N-(tert.-Butylamino)ethyl(meth)acrylat, N,N-Dimethylaminoethyl(meth)acrylat, N-[3-(dimethylamino)propyl](meth)acrylamid, Vinylimidazol und deren Mischungen.

Enthalten die erfindungsgemäßen Polymere A Komponenten d3) und/oder d4) einpolymerisiert, so enthalten sie wenigstens 0.1 Gew.-%, bevorzugt wenigstens 1 Gew.-%, besonders bevorzugt wenigstens 2 Gew.-% und insbesondere wenigstens 3 Gew.-% und höchstens 30 Gew.-%, bevorzugt höchstens 20 Gew.-%, besonders bevorzugt höchstens 15 Gew.-% und insbesondere höchstens 10 Gew.-% der Komponenten d3) und/oder d4), bezogen auf das Gesamtgewicht eingesetzten Komponenten a) bis d).

Die geladenen kationischen Gruppen lassen sich aus den Aminstickstoffen durch Quaternisierung mit sogenannten Alkylierungsmitteln erzeugen. Beispiele für geeignete Alkylierungsmittel sind $C_1$-$C_4$-Alkylhalogenide oder -sulfate, wie Ethylchlorid, Ethylbromid, Methylchlorid, Methylbromid, Dimethylsulfat und Diethylsulfat. Eine Quaternisierung kann im Allgemeinen sowohl vor als auch nach der Polymerisation erfolgen.

Komponente d5)

Komponente d5) sind Verbindungen mit wenigstens 2 radikalisch polymerisierbaren nichtkonjugierten Doppelbindungen pro Molekül.

Geeignete Komponenten d5) sind zum Beispiel Acrylester, Methacrylester, Allylether oder Vinylether von mindestens zweiwertigen Alkoholen. Die OH-Gruppen der zugrundeliegenden Alkohole können dabei ganz oder teilweise verethert oder verestert sein; die Komponenten d5) enthalten aber mindestens zwei radikalisch polymerisierbare ungesättigte Gruppen.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypiva-linsäure-neopentylglykolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000.

Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten,

eingesetzt werden.

Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Bevorzugte mehrwertige Alkohole in diesem Zusammenhang sind auch Di- und Trisaccaride.

Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Komponenten d5) sind die Vinylester oder die Ester einwertiger, ungesättigter Alkohole mit olefinisch ungesättigten $C_3$- bis $C_6$-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Komponenten d5) sind Ester ungesättigter Carbonsäuren mit den oben be-schriebenen mehrwertigen Alkoholen, beispielsweise der Ölsäure, Crotonsäure, Zimtsäure oder 10-Undecensäure.

Geeignet als Komponenten d5) sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht kon-jugiert sein dürfen, z.B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Als Komponenten d5) sind ferner geeignet die Amide von (Meth)Acrylsäure, Itaconsäure und Maleinsäure sowie N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z.B. Triallylmethylammoniumchlorid oder -methylsulfat, als Komponente d5) geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z.B. N,N'-Divinylethylenharnstoff oder N,N'-Divinylpropylenharnstoff.

Geeignet sind auch Alkylenbisacrylamide wie Methylenbisacrylamid und N,N'-(2,2)butan und 1,1'-bis-(3,3'-vinylbenzimidazolith-2-on)-1,4-butan.

Andere geeignete Komponenten d5) sind beispielsweise Alkylenglykoldi(meth)acrylate wie E-thylenglykoldiacrylat, Ethylenglykoldimethacrylat, Tetraethlyenglykolacrylat, Tetraethy-lenglykoldimethacrylat, Diethylenglykolacrylat, Diethylenglykolmethacrylat, Vinylacrylat, Allylacrylat, Allylmethacrylat, Divinyldioxan, Pentaerythritallylether sowie Gemische dieser Komponenten d5).

Weitere geeignete Komponenten d5) sind Divinyldioxan, Tetraallylsilan oder Tetravinylsilan.

Besonders bevorzugt eingesetzte Komponenten d5) sind beispielsweise Methylenbisacrylamid, Triallylamin und Triallylalkylammoniumsalze, Divinylimidazol, Pentaerythrittriallylether, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Ganz besonders bevorzugt als Komponente d5) sind Pentaerythrittriallylether, Methylenbisacrylamid, N,N'-Divinylethylenharnstoff, Triallylamin und Triallylmonoalkylammoniumsalze, und Acrylsäureester von Glykol, Butandiol, Trimethylolpropan oder Glycerin oder Acrylsäureester von mit Ethylenoxid und/oder Epichlorhydrin umgesetzten Glykol, Butandiol, Trimethylolpropan oder Glycerin.

Selbstverständlich können auch Mischungen der vorgenannten Verbindungen eingesetzt werden. Die Komponente d5) ist vorzugsweise im Reaktionsmedium löslich. Ist die Löslichkeit der Komponente d5) im Reaktionsmedium gering, so kann sie in einem Monomeren oder in einer Monomerenmischung gelöst werden oder aber in einem Lösungsmittel gelöst zudosiert werden, das sich mit dem Reaktionsmedium mischt. Besonders bevorzugt sind solche Komponenten d5), die in der Monomermischung löslich sind.

Wird die Komponente d5) zur Herstellung des erfindungsgemäßen Polymers A eingesetzt, so beträgt die eingesetzte Menge wenigstens 0,01, bevorzugt wenigstens 0,05, besonders bevorzugt wenigstens 0,1 und höchstens 5, bevorzugt höchstens 2 und besonders bevorzugt höchstens 1 Gew.-%, bezogen auf die Gesamtmenge der Komponenten a) bis d).

Sollen die erfindungsgemäßen Polymere A eine Komponente d5) einpolymerisiert enthalten, so ist es besonders vorteilhaft, Mischungen der Komponenten c) und d5) einzusetzen. Solche Mischungen sind kommerziell erhältlich und enthalten neben der Komponente c) üblicherweise als Reaktivverdünner bezeichnete Substanzen wie beispielsweise

Tripropylenglykoldiacrylat (TPGDA), Hexandioldiacrylat (HDDA), Dipropylenglykoldiacrylat (DPGDA), Trimethylolpropanformalmonoacrylat (Laromer®LR 8887), Trimethylolpropantriacrylat (TMPTA), propoxyliertes Glyceryltriacrylat (GPTA), Ethoxyliertes Trimethylolpropantriacrylat (EO3TMPTA), Ethoxyethoxyethylacrylat (EOEOEA), PEG 400 diacrylat (PEG400DA), Isobornylacrylat (IBOA), Propoxyliertes Neopentylglycoldiacrylat (PO2NPGDA), 2-Phenoxyethylacrylat (POEA), Butandioldiacrylat (BDDA), Butandiolacrylat (BDMA), Dihydrodicyclopentadienylacrylat (DCPA), Triethylenglycoldivinylether, Ethyldigycolacrylat (EDGA), Laurylacrylat (LA), 4-t-butylcyclohexylacrylat (TBCH).

Als Komponente d) können auch Vinylacetat, Vinylpropionat, Vinylbutyrat, Ethylen, Propylen, Isobutylen, Butadien, Styrol, α-Methylstyrol, Acrylnitril, Methacrylnitril, Vinylchlorid, Vinylidenchlorid, Vinylfluorid, Vinylidenfluorid und Mischungen davon eingesetzt werden.

Die Komponenten d) können auch silikonhaltige Verbindungen sein, insbesonder auch Silikongruppen (Poly) urethanacrylate.

Die in den erfindungsgemäßen Zubereitungen enthaltenen Polymere A enthalten bevorzugt

a) 50-95 Gew.-% der Komponente a),
b) 4-30 Gew.-% der Komponente b),
c) 0,1-20 Gew.-% der Komponente c) und
d) 0-30 Gew.-% der Komponente d)
einpolymerisiert, mit der Maßgabe, dass sich die Mengen der Komponenten a) bis d) zu 100 Gew.-% addieren.

Die in den erfindungsgemäßen Zubereitungen enthaltenen Polymere A enthalten besonders bevorzugt

a) 65-85 Gew.-% der Komponente a),
b) 10-30 Gew.-% der Komponente b),
c) 0,5-10 Gew.-% der Komponente c) und
d) 0-30 Gew.-% der Komponente d)
einpolymerisiert enthält, mit der Maßgabe, dass sich die Mengen der Komponenten a) bis d) zu 100 Gew.-% addieren.

In einer Ausführungsform der Erfindung enthalten die in den erfindungsgemäßen Zubereitungen enthaltenen Polymere A

a) 70-80 Gew.-% der Komponente a), ausgewählt aus der Gruppe bestehend aus Methylmethacrylat, Ethylmethacrylat und deren Mischungen,
b) 15-28 Gew.-% der Komponente b), ausgewählt aus der Gruppe bestehend aus Acrylsäure, Methacrylsäure und deren Mischungen,
c) 0,5-5 Gew.-% urethangruppenhaltige (Meth)acrylate
d) 0-30 Gew.-% der Komponente d)
einpolymerisiert mit der Maßgabe, dass sich die Mengen der Komponenten a) bis d) zu 100 Gew.-% addieren.

Herstellung der erfindungsgemäßen Polymere A

Die Herstellung der erfindungsgemäßen Polymere A kann z.B. durch Lösungs-, Fällungs-, Suspensions- oder Emulsionspolymerisation erfolgen. Derartige Verfahren sind dem Fachmann prinzipiell bekannt. Bevorzugt ist die Herstellung durch Lösungspolymerisation. Bevorzugt ist es, die Polymere A durch radikalische Lösungspolymerisation herzustellen.

Bevorzugte Lösemittel zur Polymerisation sind alkoholische oder alkoholisch/wässrige Lösungsmittel wie Ethanol und Gemische aus Ethanol mit Wasser und/oder weiteren Alkoholen wie Methanol, n-Propanol, Isopropanol, n-Butanol, sek.-Butanol, tert.-Butanol, n-Hexanol und Cyclohexanol sowie Glykolen wie Ethylenglykol, Propylenglykol und Butylenglykol sowie den Methyl- oder Ethylethern der zweiwertigen Alkohole wie Diethylenglykol, Triethylenglykol, Polyethylenglykolen mit zahlenmittleren Molekulargewichten bis etwa 3000, Glycerin und Dioxan.

Besonders bevorzugt ist die Polymerisation in Alkohol, beispielsweise in Ethanol oder in einem Alkohol/Wasser-Gemisch, beispielsweise in einem Ethanol/Wasser-Gemisch.

Die Polymerisationstemperaturen liegen vorzugsweise in einem Bereich von etwa 30 bis 120°C, besonders bevorzugt 40 bis 100°C. Die Polymerisation erfolgt üblicherweise unter atmosphärischem Druck, sie kann jedoch auch unter vermindertem oder erhöhtem Druck ablaufen. Ein geeigneter Druckbereich liegt zwischen 1 und 5 bar.

Zur Copolymerisation können die Monomeren mit Hilfe von Radikale bildenden Initiatoren polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid,

Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azobisisobutyronitril, 2,2'-Azobis(2-amidinopropan)hydrochlorid (Wako V-50®), 2,2'-Azobis[2-(2-imidazolin-2-yl)propan] (Wako VA-061®), 2,2'-Azobis(2-methyl-butyronitril) (Wako V-59®), Dimethyl 2,2'-azobis(2-methylpropionate) (Wako V-601®), 2,2'-Azobis(2,4-dimethylvaleronitril), 1,1'-Azo-bis-(1-cyclohexancarbonitril), 4,4'-Azobis(4-Cyanovaleriansäure) oder 2-(Carbamoylazo)-isobutyronitril.

Geeignet sind auch Initiatormischungen oder Redox-Initiator-Systeme, wie z. B. Ascorbinsäure/Eisen(II)sulfat/Natriumperoxodisulfat, tert.-Butylhydroperoxid/Natriumdisulfit, tert.-Butylhydroperoxid/ Natriumhydroxymethansulfinat, $H_2O_2/Cu^I$.

Als Oxidationsmittel für Redoxinitiatorsysteme kommen im wesentlichen die oben genannten Peroxide in Betracht. Als entsprechende Reduktionsmittel können Schwefelverbindungen mit niedriger Oxidationsstufe, wie Alkalisulfite, beispielsweise Kalium- und/oder Natriumsulfit, Alkalihydrogensulfite, beispielsweise Kalium- und/oder Natriumhydrogensulfit, Alkalimetabisulfite, beispielsweise Kalium- und/oder Natriummetabisulfit, Formaldehydsulfoxylate, beispielsweise Kalium- und/oder Natriumformaldehydsulfoxylat, Alkalisalze, speziell Kalium- und/oder Natriumsalze aliphatischer Sulfinsäuren und Alkalimetallhydrogensulfide, wie beispielsweise Kalium- und/oder Natrium-hydrogensulfid, Salze mehrwertiger Metalle, wie Eisen-(II)-sulfat, Eisen-(II)-Ammoniumsulfat, Eisen-(II)-phosphat, Endiole, wie Dihydroxymaleinsäure, Benzoin und/oder Ascorbinsäure sowie reduzierende Saccharide, wie Sorbose, Glucose, Fructose und/oder Dihydroxyaceton eingesetzt werden.

Geeignete Initiatoren sind beschrieben in den Kapiteln 20 und 21 von Macromolecules, Vol. 2, 2nd Ed., H. G. Elias, Plenum Press, 1984, New York, worauf hier vollumfänglich Bezug genommen wird. Weiterhin sind geeignete Photoinitiatoren beschrieben in S.P. Pappas, J. Rad. Cur., July 1987, S.6, worauf hier vollumfänglich Bezug genommen wird.

Die Initiatoren werden üblicherweise in Mengen bis zu 10, vorzugsweise 0,02 bis 5 Gew.-%, bezogen auf die zu polymerisierenden Monomeren eingesetzt.

Zur Einstellung des Molekulargewichts kann die Polymerisation in Gegenwart wenigstens eines Kettenübertragungsreagens (Reglers) erfolgen. Als Kettenübertragungsreagens können die üblichen, dem Fachmann bekannten Verbindungen, wie z. B. Schwefelverbindungen, z. B. Mercaptoethanol, 2-Ethylhexylthioglycolat, Thioglycolsäure, Alkanthiole sowie Tribromchlormethan oder andere Verbindungen, die regelnd auf das Molekulargewicht der erhaltenen Polymere wirken, eingesetzt werden.

Als Alkanthiole werden lineare und verzweigte Alkanthiole mit einer C-Kettenlänge von $C_{10}$ bis $C_{22}$ eingesetzt. Besonders bevorzugt sind lineare Alkanthiole, weiterhin bevorzugt sind Alkanthiole mit einer Kettenlänge von $C_{12}$ bis $C_{22}$, insbesondere von $C_{12}$ bis $C_{18}$. Als bevorzugte Alkanthiole seien genannt n-Decanthiol, n-Dodecanthiol, tert.-Dodecanthiol, n-Tetradecanthiol, n-Pentadecanthiol, n-Hexadecanthiol, n-Heptadecanthiol, n-Octadecanthiol, n-Nonadecanthiol, n-Eicosanthiol, n-Docosanthiol. Besonders bevorzugt sind lineare, geradzahlige Alkanthiole. Die Alkanthiole können auch in Mischungen eingesetzt werden.

Die Alkanthiole werden üblicherweise in Mengen von 0,1 bis 5 Gew.-%, insbesondere 0,25 bis 2 Gew.-% bezogen auf die zu polymerisierenden Monomere eingesetzt. Üblicherweise werden die Alkanthiole zusammen mit den Monomeren der Polymerisation zugesetzt.

Werden bei der Polymerisation Alkanthiole mit einer C-Kettenlänge von $C_{10}$ bis $C_{13}$ eingesetzt, ist eine anschließende Wasserstoffperoxidbehandlung erforderlich, um geruchlich neutrale Polymere zu erhalten. Für diese sich an die Polymerisation anschließende Wasserstoffperoxidbehandlung werden üblicherweise 0,01 bis 2,0 Gew.-%, insbesondere 0,02 bis 1,0 Gew.-%, bevorzugt 0,3 bis 0,8 Gew.-%, weiterhin bevorzugt 0,03 bis 0,15 Gew.-% Wasserstoffperoxid, bezogen auf die zu polymerisierenden Monomere eingesetzt. Es hat sich als vorteilhaft erwiesen, die Wasserstoffperoxidbehandlung bei einer Temperatur von 20 bis 100°C, insbesondere von 30 bis 80°C durchzuführen. Die Wasserstoffperoxidbehandlung wird üblicherweise für eine Dauer von 30 min bis 240 min, insbesondere für eine Dauer von 45 min bis 90 min durchgeführt.

Werden Alkanthiole mit einer C-Kettenlänge von C14 bis C22 eingesetzt, kann die Wasserstoffperoxidbehandlung entfallen. In einer weiteren Ausführungsform der Erfindung kann jedoch auch bei der Verwednung von Alkanthiolen mit einer Kettenlänge von C14 bis C22 eine Wasserstoffperoxidbehandlung angeschlossen werden.

Zur Erzielung möglichst reiner Polymere A mit geringem Restmonomergehalt kann sich an die Polymerisation (Hauptpolymerisation) wenigstens ein Nachpolymerisationsschritt anschließen. Die Nachpolymerisation kann in Gegenwart desselben oder eines anderen Initiatorsystems wie die Hauptpolymerisation erfolgen. Vorzugsweise erfolgt die Nachpolymerisation mindestens bei der gleichen, vorzugsweise bei einer höheren Temperatur als die Hauptpolymerisation. Gewünschtenfalls kann der Reaktionsansatz im Anschluss an die Polymerisation oder zwischen dem ersten und dem zweiten Polymerisationsschritt einem Strippen mit Wasserdampf oder einer Wasserdampf-Destillation unterzogen werden.

Die Copolymerisation erfolgt nach den üblichen Verfahrenstechniken der Lösungspolymerisation, z.B. nach

der sogenannten Batchpolymerisation, bei der man die Monomeren sowie gegebenenfalls Polymerisationsregler und Initiator in einem Lösemittel vorlegt und auf die Polymerisationstemperatur erwärmt. Das Reaktionsgemisch wird bevorzugt solange bei der Polymerisationstemperatur gerührt, bis die Umsetzung der Monomeren mehr als 99,9% beträgt. Die Zugabe der Initiatoren kann bei diesen Verfahren gegebenenfalls auch erst nach Erreichen der Polymerisationstemperatur erfolgen.

Weitere Verfahrensvarianten sind Zulaufmethoden, die bevorzugt angewendet werden. Dabei werden einzelne oder alle Reaktionsteilnehmer ganz oder teilweise, absatzweise oder kontinuierlich, gemeinsam oder in getrennten Zuläufen zu einer Reaktionsmischung gegeben. So kann man beispielsweise zu einem Gemisch der Monomeren und eines Lösemittels bei der Polymerisationstemperatur innerhalb einer gegebenen Zeit gegebenenfalls eine Lösung des Polymerisationsreglers und eine Initiatorlösung kontinuierlich oder absatzweise zugeben. Es.ist jedoch auch möglich, eine Mischung aus Initiator und gegebenenfalls Regler der auf Polymerisationstemperatur erwärmten Vorlage zuzudosieren. Eine andere Variante besteht darin, den Initiator unterhalb oder bei der Polymerisationstemperatur in die Vorlage zu geben und, falls ein Regler verwendet werden soll, nur den Regler oder eine Lösung des Reglers nach Erreichen der Polymerisationstemperatur innerhalb eines vorgegebenen Zeitraums dem Reaktionsgemisch zuzuführen.

Das bei der Herstellung der Polymere eingesetzte organische Lösungsmittel kann durch übliche, dem Fachmann bekannte Verfahren, z. B. durch Destillation bei vermindertem Druck, entfernt werden. Die bei der Polymerisation entstandenen Mischungen können im Anschluß an den Polymerisationsprozeß einer physikalischen oder chemischen Nachbehandlung unterworfen werden. Solche Verfahren sind beispielsweise die bekannten Verfahren zur Restmonomerenreduzierung wie z.B. die Nachbehandlung durch Zusatz von Polymerisationsinitiatoren oder Mischungen mehrerer Polymerisationsinitiatoren bei geeigneten Temperaturen oder Erhitzen der Polymerisationslösung auf Temperaturen oberhalb der Polymerisationstemperatur, eine Nachbehandlung der Polymerlösung mittels Wasserdampf oder Strippen mit Stickstoff oder Behandeln der Reaktionsmischung mit oxidierenden oder reduzierenden Reagenzien, Adsorptionsverfahren wie die Adsorption von Verunreinigung an ausgewählten Medien wie z.B. Aktivkohle oder eine Ultrafiltration. Es können sich auch die bekannten Aufarbeitungsschritte anschließen, beispielsweise geeignete Trockenverfahren wie Sprüh-, Gefrier- oder Walzentrocknung oder an die Trocknung anschließende Agglomerationsverfahren. Die nach dem erfindungsgemäßen Verfahren erhaltenen restmonomerenarmen Mischungen können auch direkt in den Handel gebracht werden.

Pulverförmige Polymere haben den Vorteil einer besseren Lagerfähigkeit, einer einfacheren Transportmöglichkeit und zeigen in der Regel eine geringere Neigung für Keimbefall.

Neutralisation

Die erfindungsgemäßen Polymere A können teilweise oder vollständig neutralisiert werden. Insbesondere zur Verwendung der Polymere in haarkosmetischen Zubereitungen ist eine teilweise oder vollständige Neutralisation vorteilhaft. In bevorzugten Ausführungsformen sind die Polymere beispielsweise zu mindestens 10, bevorzugt zu mindestens 30, weiter bevorzugt zu mindestens 40, besonders bevorzugt zu mindestens 50, ganz besonders bevorzugt zu mindestens 70 und insbesondere zu mindestens 90 bis 100 % neutralisiert. Es kann auch vorteilhaft sein, die Polymere zu mindestens 99 % und insbesondere zu mindestens 100 % zu neutralisieren.

Die Neutralisation kann während oder nach der Polymerisation erfolgen.

Es ist weiterhin vorteilhaft, wenn das Neutralisationsmittel in mehr als äquivalenter Menge zugesetzt wird, wobei unter äquivalenter Menge die Menge verstanden wird, die benötigt wird, um alle neutralisierbaren Gruppen der Polymere zu neutralisieren.

Die Neutralisation kann beispielsweise erfolgen mit

- einem Mono-, Di- oder Trialkanolamin mit 2 bis 5 Kohlenstoffatomen im Alkanolrest, der gegebenenfalls in veretherter Form vorliegt, beispielsweise Mono-, Di- und Trietha-nolamin, Mono-, Di- und Tri-n-propanolamin, Mono-, Di- und Triisopropanolamin, 2-Amino-2-methylpropanol und Di(2-methoxyethyl)amin,

- einem Alkandiolamin mit 2 bis 5 Kohlenstoffatomen, beispielsweise 2-Amino-2-methylpropan-1,3-diol und 2-Amino-2-ethylpropan-1,3-diol, oder

- einem primären, sekundären oder tertiären Alkylamin mit insgesamt 5 bis 10 Kohlen-stoffatomen, beispielsweise N,N-Diethylpropylamin oder 3-Diethylamino-1-propylamin.

Als Alkalimetallhydroxide eignen sich zur Neutralisation vor allem Natrium-, oder Kalium- sowie Ammoniumhydroxid.

Häufig werden gute Neutralisationsergebnisse mit 2-Amino-2-methylpropanol, Triisopropanolamin, 2-Amino-2-ethylpropan-1,3-diol, N,N-Dimethylaminoethanol oder 3-Diethylamino-1-propylamin erhalten.

Zur Neutralisation der Polymere in den erfindungsgemäßen Zubereitungen und Mitteln sind inbesondere Aminogruppen enthaltende Silikonpolymere geeignet. Geeignete Aminogruppen enthaltende Silikonpolymere sind beispielsweise die Silikon-Aminopoly-alkylenoxid-Blockcopolymere der WO 97/32917, die Produkte Silsoft®A-843 (Dimethicone Bisamino Hydroxypropyl Copolyol) und Silsoft®A-858 (Trimethylsilyl A-modimethicone Copolymer) (beide Fa. Witco). Weiterhin sind auch die Neutralisationspolymere der EP-A 1 035 144 und insbesondere die silikonhaltigen Neutralisationspolymere gemäß Anspruch 12 der EP-A 1 035 144 geeignet.

Haarkosmetische Zubereitungen

Die zuvor beschriebenen Polymere A eignen sich hervorragend zur Herstellung haarkosmetischer Zubereitungen, insbesondere als low-VOC-Zubereitungen. Sie dienen dabei z.B. als polymere Filmbildner. Sie sind universell in den verschiedensten haarkosmetischen Zubereitungen einsetzbar und einformulierbar und mit den üblichen Komponenten verträglich.

Der Begriff VOC ist dem Fachmann bekannt. VOC (engl.: volatile organic compounds - VOC) sind organisch chemische Verbindungen, welche bei Normaldruck in einem Bereich bis ca. 260°C sieden und somit gasförmig in die Raumluft gelangen können. Zu den flüchtigen organischen Verbindungen zählen zahlreiche Lösemittel und Treibmittel.

| Klassifizierung von organischen Verbindungen im Innenraum (nach: WHO - World Health Organization 1989) | | |
|---|---|---|
| Verbindungen | Abkürzung | Siedepunktsbereich [°Celsius] |
| leichtflüchtige organische Verbindungen (engl.: very volatile organic compounds) | VVOC | < 0 bis 50°C (bis 100°C) |
| flüchtige organische Verbindungen (engl.: volatile organic compounds) | VOC | 50 bis 250°C (100 bis 260°C) |
| Mittel- bis schwerflüchtige organische Verbindungen (engl.: semi volatile organic compounds) | SVOC | 250 bis 380°C (260 bis 400°C) |

Die Polymere A eignen sich vorteilhaft zur Erzeugung elastischer Frisuren bei gleichzeitig starker Festigung (auch bei hoher Luftfeuchtigkeit). Die erfindungsgemäßen Polymere A zeichnen sich durch gute Treibgasverträglichkeit, gute Löslichkeit in wässrig/alkoholischen Lösungsmittelgemischen, insbesondere durch die Eignung für einen Einsatz als optisch klare Low-VOC-Formulierungen und durch gute Auswaschbarkeit aus. Zudem haben sie in der Regel auch gute konditionierende Eigenschaften, d.h. sie verbessern damit behandeltes Haar in seinen sensorisch erfassbaren Eigenschaften wie Griff, Volumen, Handhabbarkeit usw.. Haarsprayformulierungen auf Basis der erfindungsgemäßen Polymere A zeichnen sich durch gute Sprühbarkeit und gute rheologische Eigenschaften und äußerst geringe Klebrigkeit der resultierenden Filme aus. Die die Polymer A enthaltenden erfindungsgemäßen haarkosmetischen Zubereitungen neigen nach dem Aufbringen nicht zur Schaumbildung. Neben der guten Verträglichkeit mit den üblichen haarkosmetischen Inhaltsstoffen trocknen die aufgetragenen Polymere A schnell.

Kosmetisch akzeptabler Träger B)

Die erfindungsgemäßen wässrigen Zubereitungen weisen weiterhin wenigstens einen kosmetisch akzeptablen Träger B) auf, der ausgewählt ist unter

- i) wassermischbaren organischen Lösungsmitteln, vorzugsweise $C_2$-$C_4$-Alkanolen, insbesondere Ethanol,
- ii) Ölen, Fetten, Wachsen,
- iii) von iii) verschiedenen Estern von $C_6$-$C_{30}$-Monocarbonsäuren mit ein-, zwei- oder dreiwertigen Alkoholen,
- iv) gesättigten acyclischen und cyclischen Kohlenwasserstoffen,
- v) Fettsäuren,
- vi) Fettalkoholen,
- vii) Treibmitteln (Treibgasen)

und Mischungen davon.

Geeignete Träger B und weitere, vorteilhaft zu verwendende Wirk- und Zusatzstoffe sind im Folgenden detailliert beschrieben.

Geeignete kosmetisch und pharmazeutisch verträgliche Öl- bzw. Fettkomponenten B) sind in Karl-Heinz

Schrader, Grundlagen und Rezepturen der Kosmetika, 2. Auflage, Verlag Hüthig, Heidelberg, S. 319 - 355 beschrieben, worauf hier Bezug genommen wird. Die erfindungsgemäßen Zubereitungen können z.B. eine Öl- bzw. Fettkomponente B) aufweisen, die ausgewählt ist unter:Kohlenwasserstoffen geringer Polarität, wie Mineralölen; linearen gesättigten Kohlenwasserstoffen, vorzugsweise mit mehr als 8 C-Atomen, wie Tetradecan, Hexadecan, Octadecan etc.; cyclischen Kohlenwasserstoffen, wie Decahydronaphthalin; verzweigten Kohlenwasserstoffen; tierischen und pflanzlichen Ölen; Wachsen; Wachsestern; Vaselin; Estern, bevorzugt Estern von Fettsäuren, wie z. B. die Ester von $C_1$-$C_{24}$-Monoalkoholen mit $C_1$-$C_{22}$-Monocarbonsäuren, wie Isopropylisostearat, n-Propylmyristat, iso-Propylmyristat, n-Propylpalmitat, isoPropylpalmitat, Hexacosanylpalmitat, Octacosanylpalmitat, Triacontanylpalmitat, Dotriacontanylpalmitat, Tetratriacontanylpalmitat, Hexacosanylstearat, Octacosanylstearat, Triacontanylstearat, Dotriacontanylstearat, Tetratriacontanylstearat; Salicylaten, wie $C_1$-$C_{10}$-Salicylaten, z. B. Octylsalicylat; Benzoatestern, wie $C_{10}$-$C_{15}$-Alkylbenzoaten, Benzylbenzoat; anderen kosmetischen Estern, wie Fettsäuretriglyceriden, Propylenglykolmonolaurat, Polyethylenglykolmonolaurat, $C_{10}$-$C_{15}$-Alkyllactaten, etc. und Mischungen davon.

Geeignete Siliconöle B) sind z. B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Bevorzugte Öl- bzw. Fettkomponenten B) sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkernöl, Ricinusöl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Geeignete hydrophile Träger B) sind ausgewählt unter Wasser, 1-, 2- oder mehrwertigen Alkoholen mit vorzugsweise 1 bis 8 Kohlenstoffatomen, wie Ethanol, n-Propanol, iso-Propanol, Propylenglykol, Glycerin, Sorbit, etc..

Vorzugsweise liegen die erfindungsgemäßen Zubereitungen, die die Polymere A enthalten, in Form eines Sprays, Gels, Schaums, einer Salbe, Creme, Emulsion, Suspension, Lotion, Milch oder Paste vor. Gewünschtenfalls können auch Liposomen oder Mikrosphären eingesetzt werden.

Die erfindungsgemäßen Zubereitungen besitzen bevorzugt in pH-Wert von 2,0 bis 9,3. Besonders bevorzugt ist der pH-Bereich zwischen 4 und 8. Als zusätzliche Co-Solventien können organische Lösungsmittel oder ein Gemisch aus Lösungsmitteln mit einem Siedepunkt unter 400°C in einer Menge von 0,1 bis 15 Gew.-%, bevorzugt von 1 bis 10 Gew.-% enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol in einer Menge bis 30 Gew.-%.

In einer bevorzugten Ausführungsform der Erfindung weisen die erfindungsgemäßen Zubereitungen einen Anteil an flüchtigen organischen Komponenten von höchstens 80 Gew.-%, bevorzugt höchstens 55 Gew.-% und insbesondere höchstens 35 Gew.-% auf. Ein bevorzugter Gegenstand sind somit haarkosmetische Zubereitungen, die dem low-VOC-Standard, also VOC-80- bzw. VOC-55-Standard entsprechen.

Bevorzugt ist die Verwendung der Polymere A insbesondere in Haarsprayzubereitungen, welche die folgenden Bestandteile enthalten:

- teilweise oder vollständig neutralisiertes Polymer A;
- Wasser;
- kosmetisch übliches organisches Lösungsmittel wie beispielsweise Ethanol, Isopropanol und Dimethoxymethan, daneben auch Aceton, n-Propanol, n-Butanol, 2Methoxypropan-1-ol, n-Pentan, n-Hexan, Cyclohexan, n-Heptan, n-Octan oder Dichlormethan oder deren Gemische;
- kosmetisch übliches Treibmittel wie beispielsweise n-Propan, iso-Propan, n-Butan, i-Butan, 2,2-Dimethylbutan, n-Pentan, Isopentan, Dimethylether, Difluorethan, Fluortrichlormethan, Dichlordifluormethan oder Dichlortetrafluorethan, HFC-152 A (1,1-Difluorethan), HFC-134a (1,1,2,2-Tetrafluorethan), $N_2$, $N_2O$ und CO oder deren Gemische.

Zur Neutralisation der erfindungsgemäßen Polymere A und damit der Komponente b) und zur Einstellung des pH-Werts der haarkosmetischen Zubereitungen werden vorteilhaft Alkanolamine eingesetzt. Beispiele (INCI) sind Aminomethylpropanol, Diethanolamine, Diisopropanolamine, Ethanolamine, Methylethanolamine, N-Lauryl Diethanolamine, Triethanolamine, Triisopropanolamine, usw.. Es können sowohl primäre Aminogruppen tragende als auch sekundäre Aminogruppen tragende Alkanolamine verwendet werden.

Außerdem können Alkalihydroxide (z.B. NaOH, bevorzugt KOH) und andere Basen zur Neutralisation verwendet werden (z.B. Histidin, Arginin, Lysin oder Ethylenediamine, Diethylentriamin, Melamin, Benzoguanamin). Alle angegebenen Basen können allein oder als Gemisch mit anderen Basen zur Neutralisation säurehaltiger kosmetischer Produkte eingesetzt werden.

Ein Gegenstand der vorliegenden Erfindung sind demnach wässrige haarkosmetische Zubereitungen die neben dem wenigstens einen Polymer A und dem Träger B mindestens noch einen Wirk- oder Zusatzstoff ausgewählt aus der Gruppe bestehend aus viskositätsmodifizierenden Stoffen, haarpflegenden Stoffen, haarfestigenden Stoffen, Siliconverbindungen, Lichtschutzstoffen, Fetten, Ölen, Wachsen, Konservierungsmitteln, Pigmenten, löslichen Farbstoffen, partikelförmigen Stoffen, und Tensiden enthält.

Treibmittel (Treibgase)

Als Treibmittel (Treibgase) kommen von den genannten Verbindungen vor allem die Kohlenwasserstoffe, insbesondere Propan, n-Butan, n-Pentan und Gemische hieraus sowie Dimethylether und Difluorethan zur Anwendung. Gegebenenfalls werden einer oder mehrere der genannten chlorierten Kohlenwasserstoffe in Treibmittelmischungen mitverwendet, jedoch nur in geringen Mengen, etwa bis zu 20 Gew.-%, bezogen auf die Treibmittelmischung.

Die erfindungsgemäßen haarkosmetischen Zubereitungen eignen sich auch besonders für Pumpsprayzubereitungen ohne den Zusatz von Treibmitteln oder auch für Aerosolsprays mit üblichen Druckgasen wie Stickstoff, Druckluft oder Kohlendioxid als Treibmittel.

Eine wasserhaltige Standard-Aerosol-Sprayformulierung weist beispielsweise die folgende Zusammensetzung auf:

- zu 100 % mit 2-Amino-2-methylpropanol neutralisiertes Polymer
- Ethanol
- Wasser
- Dimethylether und/oder Propan/ n-Butan und/oder Propan/iso-Butan,

Dabei beträgt die Gesamtmenge der flüchtigen organischen Komponenten bevorzugt höchstens 80, besonders bevorzugt höchstens 55 Gew.-% der Zubereitung.

Vorzugsweise enthalten die erfindungsgemäßen haarkosmetischen Zubereitungen wenigstens ein Polymer A, wenigstens einen wie vorstehend definierten kosmetisch akzeptablen Träger B) und wenigstens einen weiteren, davon verschiedenen Wirk- oder Zusatzstoff, der ausgewählt ist unter kosmetisch aktiven Wirkstoffen, Emulgatoren, Tensiden, Konservierungsmitteln, Parfümölen, Verdickern, Haarpolymeren, Haarconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweisshydrolysaten, Lipiden, Antioxidantien, Entschäumern, Antistatika, Emollentien, Lanolin-Komponenten, Proteinhydrolysaten und Weichmachern.

Weitere Polymere

Zur gezielten Einstellung der Eigenschaften von haarkosmetischen Zubereitungen kann es von Vorteil sein, die erfindungsgemäßen Polymere in Mischung mit weiteren (haar)kosmetisch üblichen Polymeren einzusetzen.

In einer weiteren bevorzugten Ausführungsform enthält das erfindungsgemäße Mittel 0,01 bis 15 Gew.-%, vorzugsweise 0,5 bis 10 Gew.-% mindestens eines synthetischen oder natürlichen nichtionischen, bevorzugt eines filmbildenden Polymers. Unter natürlichen Polymeren werden auch chemisch modifizierte Polymere natürlichen Ursprungs verstanden. Unter filmbildenden Polymeren werden solche Polymere verstanden, die bei Anwendung in 0,01 bis 5%iger wässriger, alkoholischer oder wässrig-alkoholischer Lösung in der Lage sind, auf dem Haar einen Polymerfilm abzuscheiden.

Als solche weiteren üblichen Polymere eignen sich dazu beispielsweise anionische, kationische, amphotere, zwitterionische und neutrale Polymere.

Beispiele für solche weiteren Polymere sind

- Copolymere aus Ethylacrylat und Methacrylsäure
- Copolymere aus N-tert.-Butylacrylamid, Ethylacrylat und Acrylsäure
- Polyvinylpyrrolidone
- Polyvinylcaprolactame
- Polyurethane

- Copolymere aus Acrylsäure, Methylmethacrylat, Octylacrylamid, Butylaminoethylmethacrylat und Hydroxypropylmethacrylat,
- Copolymere aus Vinylacetat und Crotonsäure und/oder (Vinyl)-Neodecanoat,
- Copolymere aus Vinylacetat und/oder Vinylpropionat und N-Vinylpyrrolidon,
- carboxyfunktionelle Copolymere aus Vinylpyrrolidon, t-Butylacrylat, Methacrylsäure,
- Copolymere aus tert.-Butylacrylat, Methacrylsäure und Dimethicone Copolyol.

Überraschenderweise wurde gefunden, dass haarkosmetische Zubereitungen, welche die Polymere A in Kombination mit weiteren Polymeren enthalten, unerwartete Eigenschaften aufweisen. Die erfindungsgemäßen haarkosmetischen Zubereitungen sind insbesondere hinsichtlich der Gesamtheit ihrer haarkosmetischen Eigenschaften den Zubereitungen aus dem Stand der Technik überlegen.

Copolymere aus Ethylacrylat und Methacrylsäure (INCI Bezeichnung: Acrylates Copolymer), sind beispielsweise als Handelsprodukte Luviflex®Soft (BASF) erhältlich.

Copolymere aus N-tert.-Butylacrylamid, Ethylacrylat und Acrylsäure (INCI Bezeichnung: Acrylates/Acrylamide Copolymer) sind beispielsweise als Handelsprodukte Ultrahold Strong®, Ultrahold 8® (BASF) erhältlich.

Polyvinylpyrrolidone (INCI Bezeichnung: PVP) sind beispielsweise unter den Handelsnamen Luviskol®K, Luviskol®K30 (BASF) und PVP K® (ISP) erhältlich.

Polyvinylcaprolactame (INCI: Polyvinylcaprolactame) sind beispielsweise unter dem Handelsnamen Luviskol Plus® (BASF) erhältlich.

Polyurethane (INCI: Polyurethane -1) sind beispielsweise unter dem Handelsnamen Luviset®PUR erhältlich.

Copolymere aus Acrylsäure, Methylmethacrylat, Octylacrylamid, Butylaminoethylmethacrylat, Hydroxypropylmethacrylat (INCI: Octylacrylamide/ Acrylates/ Butylaminoethyl Methacrylate Copolymer) sind beispielsweise unter den Handelsnamen Amphomer®28-4910 und Amphomer®LV-71 (National Starch) bekannt.

Copolymere aus Vinylacetat und Crotonsäure (INCI: VA/Crotonate/Copolymer) sind beispielsweise unter den Handelsnamen Luviset®CA 66 (BASF), Resyn®28-1310 (National Starch), Gafset® (GAF) oder Aristoflex®A (Celanese) erhältlich.

Copolymere aus Vinylacetat, Crotonsäure und (Vinyl)neodecanoate (INCI: VA/Crotonates/Neodecanoate Copolymer) sind beispielsweise unter den Handelsnamen Resyn®28-2930 (National Starch) und Luviset®CAN (BASF) erhältlich.

Copolymere aus Vinylacetat und N-Vinylpyrrolidon (INCI: PVP/VA) sind beispielsweise unter den Handelsnamen Luviskol VA® (BASF) und PVP/VA (ISP) erhältlich.

Carboxyfunktionelle Copolymere aus Vinylpyrrolidon, t-Butylacrylat, Methacrylsäure sind beipielsweise unter dem Handelsnamen Luviskol®VBM (BASF) erhältlich.

Copolymere aus tert.-Butylacrylat, Methacrylsäure und Dimethicone Copolyol sind beipielsweise unter dem Handelsnamen Luviflex®Silk (BASF) erhältlich.

Geeignete anionische Polymere sind von den Polymeren A verschiedene Homo- und Copolymerisate von Acrylsäure und Methacrylsäure oder deren Salzen, Copolymere von Acrylsäure und Acrylamid und deren Salzen, Natriumsalze von Polyhydroxycarbonsäuren, Copolymere von Acrylsäure und Methacrylsäure mit beispielsweise hydrophoben Monomeren, z.B. $C_4$-$C_{30}$-Alkylester der (Meth)acrylsäure, $C_4$-$C_{30}$-Alkylvinylester, $C_4$-$C_{30}$-Alkylvinylether und Hyaluronsäure so wie weitere unter den Handelsnamen Amerhold®DR-25, Ultrahold®, Luviset®P.U.R., Acronal®, Acudyne®, Lovocryl®, Versatyl®, Amphomer® (28-4910, LV-71), Placise®L53, Gantrez®ES 425, Advantage Plus®, Omnirez®2000, Resyn®28-1310, Resyn®28-2930, Balance®(0/55), Acudyne®255, Aristoflex®A oder Eastman AQ® bekannte Polymere.

Weiterhin umfasst die Gruppe der geeigneten Polymere beispielhaft Balance®CR (National Starch), Balance®47 (National Starch; Octylacrylamid/Acrylate/Butylaminoethyl-methacrylate-Copolymer), Aquaflex®FX 64 (ISP; Isobutylen/ Ethylmaleimid/ Hydroxye-thylmaleimid-Copolymer), Aquaflex®SF-40 (ISP / National Starch; VP/Vinyl Caprolactam/ DMAPA Acrylate Copolymer), Allianz®LT-120 (ISP / Rohm & Haas; Acrylat/C1-2 Succinat/Hydroxyacrylat Copolymer), Aquarez® HS (Eastman; Polyester-1).

Geeignet sind auch die Polymere unter den Handelsnamen Diaformer®Z-400 (Clariant; Methacryloylethylbetain/ Methacrylat-Copolymer), Diaformer®Z-711 (Clariant; Methacry-loylethyl N-oxid/Methacrylat-Copolymer), Diaformer®Z-712 (Clariant; Methacryloylethyl N-oxide/Methacrylat-Copolymer), Omnirez®2000 (ISP; Monoethylester von Poly(Methylvinylether/ Maleinsäure in Ethanol), Amphomer®HC (National Starch; Acrylat/ Octylacrylamid-Copolymer), Amphomer®28-4910 (National Starch; Octylacrylamid/Acrylat/ Butylaminoethylmethacrylat-Copolymer), Advantage®HC 37 (ISP; Terpolymer aus Vinylcaprolactam/Vinylpyrrolidon/ Dimethylaminoethylmethacrylat), Advantage®LC55 und LC80 oder LC A und LC E, Advantage®Plus (ISP; VA/Butyl Maleate/Isobornyl Acrylate Copolymer), Aculyne®258 (Rohm & Haas; Acrylat/ Hydroxyesteracrylat-Copolymer), Luviset®P.U.R. (BASF, Polyurethane-1), Eastman®AQ 48 (Eastman), Styleze®CC-10 (ISP; VP/DMAPA Acrylates Copolymer), Styleze® 2000 (ISP; VP/Acrylates/Lauryl Methacrylate Copolymer), DynamX® (National Starch; Polyurethane-14 AMP-Acrylates Copolymer), Resyn®XP (National Starch; Acrylates/Octylacrylamide Copolymer), Fixomer® A-30 (Ondeo Nalco; Polymethacrylsäure (und) Acrylamidomethylpropansulfonsäure), Fixate® G-1 00 (Noveon; AMP-Acrylates/Allyl Methacrylate Copolymer).

Geeignete Polymere sind auch Copolymere aus (Meth)acrylsäure und Polyetheracrylaten, wobei die Polyetherkette mit einem $C_8$-$C_{30}$-Alkylrest terminiert ist. Dazu zählen z.B. Acrylat/Beheneth-25-methacrylat-Copolymere, die unter der Bezeichnung Aculyn® (Rohm + Haas) erhältlich sind. Besonders geeignete Polymere sind weiterhin Copolymere aus t-Butylacrylat, Ethylacrylat, Methacrylsäure (z.B. Luvimer®100P, Luvimer®Pro55) und Copolymere aus Ethylacrylat und Methacrylsäure (z.B. Luvimer®MAE).

Geeignet sind auch vernetzte Polymere der Acrylsäure, wie sie unter der INCI-Bezeichnung Carbomer erhältlich sind. Derartige vernetzte Homopolymere der Acrylsäure sind kommerziell beispielsweise als Carbopol® (Noveon) erhältlich. Bevorzugt sind auch hydrophob modifizierte vernetzte Polyacrylat-Polymere, wie Carbopol®Ultrez 21 (Noveon). Solche weiteren Polymere können auch zur Rheologiemodifizierung der Zubereitungen, also als Verdicker eingesetzt werden.

Als zusätzliche Polymere weiterhin geeignet sind wasserlösliche oder wasserdispergierbare Polyester, Polyharnstoffe, Polyurethane, Polyurethanharnstoffe, gegebenenfalls mit Alkoholen umgesetzte Maleinsäureanhydridcopolymere oder anionische Polysiloxane.

Weiterhin zur Verwendung gemeinsam mit den Polymeren A geeignete Polymere sind z.B. auch kationische und kationogene Polymere. Dazu zählen beispielsweise

- Copolymere aus N-Vinylpyrrolidon/N-Vinylimidazoliumsalzen (erhältlich beispielsweise unter den Handelsnamen Luviquat®FC, Luviquat®HM, Luviquat®MS, Luviquat®Care, Luviquat® UltraCare (BASF),
- Copolymere aus N-Vinylcaprolactam/N-Vinylpyrrolidon/N-Vinylimidazoliumsalzen (erhältlich beispielsweise unter dem Handelsnamen Luviquat®Hold),
- Copolymere aus N-Vinylpyrrolidon/Dimethylaminoethylmethacrylat, quaternisiert mit Diethylsulfat (erhältlich beispielsweise unter dem Handelsnamen Luviquat®PQ11),
- kationische Cellulosederivate (Polyquaternium-4 und -10),
- Acrylamidcopolymere (Polyquaternium-7),
- Guar-hydroxypropyltrimethylammoniumchlorid (INCI: Hydroxypropyl Guar Hydroxypropyltrimonium Chloride),
- Polyethylenimine und deren Salze,
- Polyvinylamine und deren Salze,
- Polymere auf Basis von Dimethyldiallylammoniumchlorid (Merquat®),
- Polymere, die durch Reaktion von Polyvinylpyrrolidon mit quaternären Ammoniumverbindungen entstehen (Gafquat®),
- Hydroxyethylcellulose mit kationischen Gruppen (Polymer®JR) und
- kationische Polymere auf pflanzlicher Basis, z.B. Guarpolymere, wie die Jaguar®-Marken der Fa. Rhodia.

Als weitere Haarkosmetik-Polymere sind auch neutrale Polymere geeignet wie

- Polyvinylpyrrolidone,
- Copolymere aus N-Vinylpyrrolidon und Vinylacetat und/oder Vinylpropionat,
- Polysiloxane,
- Polyvinylcaprolactame und
- Copolymere mit N-Vinylpyrrolidon,

- Cellulosederivate,
- Polyasparaginsäuresalze und Derivate,
- Polyamide, z.B. auf Basis von Itaconsäure und aliphatischen Diaminen, wie in der DE-A-43 33 238 beschrieben,

Zu den vorgenannten Polymerarten gehören die unter den Handelsnamen bekannten Luviskol® (K, VA, Plus), PVP K, PVP/VA, Advantage®HC, Luviflex®Swing, Kollicoat®IR, $H_2$OLD®EP-1.

Außerdem geeignet als weitere Polymere sind auch Biopolymere, d.h. Polymere, die aus natürlich nachwachsenden Rohstoffen gewonnen werden und aus natürlichen Monomerbausteinen aufgebaut sind, z.B. Cellulosederivate, Chitin-, Chitosan-, DNA-, Hyaluronsäure- und RNA-Derivate.

Geeignete Mischungspartner für die erfindungsgemäßen Polymere sind auch zwitterionische Polymere, wie sie beispielsweise in den deutschen Patentanmeldungen DE 39 29 973, DE 21 50 557, DE 28 17 369 und DE 37 08 451 offenbart sind sowie Methacroylethylbetain/Methacrylat-Copolymere, die unter der Bezeichnung Amersette® (Amerchol) im Handel erhältlich sind oder Copolymere aus Hydroxyethylmethacrylat, Methylmethacrylat, N,N-Dimethylaminoethylmethacrylat und Acrylsäure (Jordapon®).

Weitere geeignete Polymere sind auch betainische Polymere wie Yukaformer (R205, SM) und Diaformer.

Als Mischungspartner geeignete Polymere sind auch nichtionische, siloxanhaltige, wasserlösliche oder -dispergierbare Polymere, z.B. Polyethersiloxane, wie Tegopren® (Goldschmidt) oder Belsil® (Wacker).

Kosmetisch und/oder dermatologisch aktive Wirkstoffe

Geeignete kosmetisch und/oder dermatologisch aktive Wirkstoffe sind z. B. färbende Wirkstoffe, Haut- und Haarpigmentierungsmittel, Tönungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, antierythimatös oder antiallergisch aktive Wirkstoffe und Mischungen davon.

Bevorzugte haarkosmetische Pflege- und Wirkstoffe sind AHA-Säuren, Fruchtsäuren, Ceramide, Phytantriol, Collagen, Vitamine und Provitamine, beispielsweise Vitamin A, E und C, Retinol, Bisabolol und Panthenol. Besonders bevorzugt als haarkosmetischer Pflegestoff in den erfindungsgemäßen Zubereitungen ist Panthenol, das beispielsweise als D-Panthenol®USP, D-Panthenol®50 P, D-Panthenol®75 W, D,L-Panthenol®50 W kommerziell erhältlich ist.

Künstlich hautbräunende Wirkstoffe, die geeignet sind, die Haut ohne natürliche oder künstliche Bestrahlung mit UV-Strahlen zu bräunen, sind z. B. Dihydroxyaceton, Alloxan und Walnussschalenextrakt.

Geeignete keratinhärtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat, etc..

Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z.B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure, etc.. Derartige desodorierend wirkende Stoffe sind z. B. Zinkricinoleat, Triclosan, Undecylensäurealkylolamide, Citronensäuretriethylester, Chlorhexidin etc.. Die erfindungsmäßen Zubereitungen enthalten bevorzugt 0,01 bis 5, besonders bevorzugt 0,05 bis 1 Gew.-% mindestens eines Konservierungsmittels. Geeignete weitere Konservierungsmittel sind die im International Cosmetic Ingredient Dictionary and Handbook, 9. Auflage mit der Funktion "Preservatives" aufgeführten Stoffe, z.B . Phenoxyethanol, Benzylparaben, Butylparaben, Ethylparaben, Isobuty-lparaben, Isopropylparaben, Methylparaben, Propylparaben, lodopropinylbutylcarbamat, Methyldibromoglutaronitril, DMDM Hydantoin.

UV-Filtersubstanzen

Die erfindungsgemäßen Zubereitungen können in einer Ausführungsform öllösliche und/oder wasserlösliche UVA- und/oder UVB-Filter enthalten.

Die Gesamtmenge der Filtersubstanzen beträgt vorzugsweise 0,01 bis 10 Gew.-% oder von 0,1 bis 5 Gew.-%, besonders bevorzugt von 0,2 bis 2 Gew.-%, bezogen auf das Gesamtgewicht der Zubereitungen.

Der größte Teil der Lichtschutzmittel in den zum Schutz der menschlichen Epidermis dienenden Zubereitungen besteht aus Verbindungen, die UV-Licht im UV-B-Bereich absorbieren. Beispielsweise beträgt der Anteil der erfindungsgemäß zu verwendenden UV-A-Absorber 10 bis 90 Gew.-%, bevorzugt 20 bis 50 Gew.-% bezogen auf die Gesamtmenge von UV-B und UV-A absorbierenden Substanzen.

Die UVB-Filter können öllöslich oder wasserlöslich sein. Vorteilhafte UVB-Filtersubstanzen sind z.B.:

i) Benzimidazolsulfonsäure-Derivate wie z.B. 2-Phenylbenzimidazol-5-sulfonsäure und deren Salze

ii) Benzotriazol-Derivate wie z.B. 2,2'-Methylen-bis-(6-(2H-benzotriazol-2-yl)-4-(1,1,3,3-tetramethylbutyl)-phenol);

iii) 4-Aminobenzoesäure-Derivate, vorzugsweise 4-(Dimethylamino)-benzoesäure(2-ethylhexyl)ester, 4-(Dimethy1amino)benzoesäureamylester;

iv) Ester der Benzalmalonsäure, vorzugsweise 4-Methoxybenzalmalonsäuredi(2-ethylhexyl)ester;

v) Ester der Zimtsäure, vorzugsweise 4-Methoxyzimtsäure(2-ethylhexyl)ester, 4-Methoxyzimtsäureisopentylester;

vi) Derivate des Benzophenons, vorzugsweise 2-Hydroxy-4-methoxybenzophenon, 2-Hydroxy-4-methoxy-4'-methyl benzophenon, 2,2'-Dihydroxy-4-methoxybenzophenon;

vii) Methylidencampherderivate, vorzugsweise 4-Methylbenzylidencampher, Benzyl idencampher;

viii) Triazinderivate, vorzugsweise 4,4',4"-(1,3,5-Triazin-2,4,6-triylimino)-tris-benzoesäure-tris-(2-ethylhexylester) [INCI: Diethylhexyl Butamido Triazine, UVA-Sorb® HEB (Sigma 3V)] und 2,4,6-Tris-[anilino-(p-carbo-2'-ethyl-1'-hexyloxy)]-1 ,3,5-triazin [INCI: Octyl Triazone, Uvinul®T 150 (BASF)].

Vorteilhaft zu verwendende wasserlösliche UVB-Filtersubstanzen sind z.B. Sulfonsäure-Derivate des 3-Benzylidencamphers, wie z.B. 4-(2-Oxo-3-bornylidenmethyl) benzol-sulfonsäure, 2-Methyl-5-(2-oxo-3-bornylidenmethyl) sulfonsäure und deren Salze.

Vorteilhaft zu verwendende UVA-Filter sind z.B.:

- 1 ,4-Phenylendimethincamphersulfonsäurederivate wie z.B. 3,3'-(1,4-phenylendimethin)-bis-(7,7-dimethyl-2-oxobicyclo[2.2.1]-heptan-1-methansulfonsäure und ihre Salze

- 1,3,5-Triazinderivate wie 2,4-Bis{[(2-ethylhexyloxy)-2-hydroxy)-phenyl}-6-(4-methoxyphenyl)-l,3,5)-triazin (z.B. Tinosorb®S (Ciba))

- Dibenzoylmethanderivate, vorzugsweise 4-Isopropyldibenzoylmethan, 4-(tert.-Butyl)-4'-methoxydibenzoylmethan

- Benzoxazol-Derivate, beispielsweise das 2,4-Bis-[4-[5-(1,1-dimethylpropyl)benzoxazol-2-yl]phenylimino]-6-[(2-ethylhexyl)imino]-1,3,5- triazin (CAS Nr. 288254-1 6-0, Uvasorb®K2A (3V Sigma))

- Hydroxybenzophenone, beispielsweise der 2-(4'-Diethylamino-2'-hydroxybenzoyl)-benzoesäurehexylester (auch: Aminobenzophenon) (Uvinul®A Plus (BASF))

Ferner kann erfindungsgemäß gegebenenfalls von Vorteil sein, Zubereitungen mit weiteren UVA- und/oder UVB-Filtern zu versehen, beispielsweise bestimmten Salicylsäurederivaten wie 4-Isopropylbenzylsalicylat, 2-Ethylhexylsalicylat, Octylsalicylat, Homo-menthylsalicylat. Die Gesamtmenge an Salicylsäurederivaten in den kosmetischen Zubereitungen wird vorteilhaft aus dem Bereich von 0,1-15,0, bevorzugt 0,3-10,0 Gew.- % gewählt, bezogen auf das Gesamtgewicht der Zubereitungen. Ein weiterer erfindungsgemäß vorteilhaft zu verwendender Lichtschutzfilter ist Ethylhexyl-2-cyano-3,3-diphenylacrylat (Octocrylen, Uvinul®N 539 (BASF)).

Die folgende Tabelle stellt beispielhaft einige für die Verwendung in den erfindungsgemäßen Zubereitungen geeignete Lichtschutzfilter zusammen:

| Nr. | Stoff | CAS-Nr. |
|---|---|---|
| 1 | 4-Aminobenzoesäure | 150-13-0 |
| 2 | 3-(4'-Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |

(fortgesetzt)

| Nr. | Stoff | CAS-Nr. |
|---|---|---|
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfonsäure-(Sulisobenzonum) und das Natriumsalz | 4065-45-6 |
| 13 | 3-(4'-Sulfobenzyliden)-bornan-2-on und Salze | 58030-58-6 |
| 14 | 3-Benzylidenbornan-2-on | 16087-24-8 |
| 15 | 1-(4'-Isopropylphenyl)-3-phenylpropan-1,3-dion | 63260-25-9 |
| 16 | 4-Isopropylbenzylsalicylat | 94134-93-7 |
| 17 | 3-Imidazol-4-yl-acrylsäure und ihr Ethylester | 104-98-3 |
| 18 | 2-Cyano-3,3-diphenylacrylsäureethylester | 5232-99-5 |
| 19 | 2-Cyano-3,3-diphenylacrylsäure-2'-ethylhexylester | 6197-30-4 |
| 20 | Menthyl-o-aminobenzoat oder: 5-Methyl-2-(1-methylethyl)-2-aminobenzoat | 134-09-8 |
| 21 | Glyceryl p-aminobenzoat oder: 4-Aminobenzoesäure-1-glyceryl-ester | 136-44-7 |
| 22 | 2,2'-Dihydroxy-4-methoxybenzophenon (Dioxybenzone) | 131-53-3 |
| 23 | 2-Hydroxy-4-methoxy-4-methylbenzophenon (Mexenon) | 1641-17-4 |
| 24 | Triethanolamin Salicylat | 2174-16-5 |
| 25 | Dimethoxyphenylglyoxalsäure oder: 3,4-dimethoxy-phenyl-glyoxal-saures Natrium | 4732-70-1 |
| 26 | 3-(4'Sulfobenzyliden)-bornan-2-on und seine Salze | 56039-58-8 |
| 27 | 4-tert.-Butyl-4'-methoxy-dibenzoylmethan | 70356-09-1 |
| 28 | 2,2',4,4'-Tetrahydroxybenzophenon | 131-55-5 |
| 29 | 2,2'-Methylen-bis-[6-(2H-benzotriazol-2-yl)-4-(1,1,3,3,-tetramethylbutyl)phenol] | 103597-45-1 |
| 30 | 2,2'-(1,4-Phenylen)-bis-1H-benzimidazol-4,6-disulfonsäure, Na-Salz | 180898-37-7 |
| 31 | 2,4-bis-[4-(2-Ethylhexyloxy)-2-hydroxy]phenyl-6-(4-methoxyphenyl)-(1,3,5)-triazin | 187393-00-6 |
| 32 | 3-(4-Methylbenzyliden)-campher | 36861-47-9 |
| 33 | 4-Bis(polyethoxy)paraaminobenzoesäurepolyethoxyethylester | 113010-52-9 |
| 34 | 2,4-Dihydroxybenzophenon | 131-56-6 |
| 35 | 2,2'-Dihydroxy-4,4'-dimethoxybenzophenon-5,5'-dinatriumsulfonat | 3121-60-6 |
| 36 | Benzoesäure, 2-[4-(diethylamino)-2-hydroxybenzoyl]-, hexylester | 302776-68-7 |
| 37 | 2-(2H-Benzotriazol-2-yl)-4-methyl-6-[2-methyl-3-[1,3,3,3-tetramethyl-1-[(trimethylsilyl) oxy]disiloxanyl]propyl]phenol | 155633-54-8 |
| 38 | 1,1-[(2,2'-Dimethylpropoxy)carbonyl]-4,4-diphenyl-1,3-butadien | 363602-15-7 |

Geeignete UV-Lichtschutzfilter mit der CAS-Nr. 113010-52-9 sind beispielsweise unter der Bezeichnung Uvinul®P 25 kommerziell erhältlich.

Auch polymere oder polymergebundene Filtersubstanzen können erfindungsgemäß verwendet werden.

Metalloxide wie Titandioxid oder Zinkoxid können ebenfalls vorteilhaft zum Schutz vor schädlicher Sonnenstrahlung eingesetzt werden. Ihre Wirkung beruht im wesentlichen auf Reflexion, Streuung und Absorption der schädigenden UV-Strahlung und hängt wesentlich von der Primärpartikelgröße der Metalloxide ab. Die erfindungsgemäßen haarkosmetischen Zubereitungen können vorteilhafterweise außerdem anorganische Pigmente auf Basis von Metalloxiden und/oder anderen in Wasser schwerlöslichen oder unlöslichen Metallverbindungen, ausgewählt aus der Gruppe der Oxide des Zinks (ZnO), Eisens (z.B. $Fe_2O_3$), Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden enthalten. Besonders bevorzugt handelt es sich um Pigmente auf der Basis ZnO.

Die anorganischen Pigmente können dabei in gecoateter Form vorliegen, d.h. dass sie oberflächlich behandelt sind. Diese Oberflächenbehandlung kann beispielsweise darin bestehen, dass die Pigmente nach an sich bekannter Weise, wie in DE-A-33 14 742 beschrieben, mit einer dünnen hydrophoben Schicht versehen sind.

Zur Verwendung in den erfindungsgemäßen Zubereitungen geeignete Lichtschutzmittel sind die in der EP-A 1 084 696 in den Absätzen [0036] bis [0053] genannten Verbindungen, worauf an dieser Stelle vollumfänglich Bezug genommen wird. Für die erfindungsgemäße Verwendung geeignet sind alle UV-Lichtschutzfilter, die in Anlage 7 (zu § 3b) der deutschen Kosmetik-Verordnung unter "Ultraviolett-Filter für kosmetische Mittel" genannt sind.

Die Aufzählung der genannten UV-Lichtschutz-Filter, die in den erfindungsgemäßen Zubereitungen verwendet werden können, ist nicht abschließend.

Verdickungsmittel

Geeignete Verdickungsmittel sind in "Kosmetik und Hygiene von Kopf bis Fuß", Hrsg. W. Umbach, 3. Auflage, Wiley-VCH, 2004, S.235-236 genannt, worauf an dieser Stelle vollumfänglich Bezug genommen wird.

Konsistenzregulatoren ermöglichen die Einstellung der gewünschten Viskosität von beispielsweise Shampoos. Verdicker, die durch eine Vergrößerung der Tensidmicellen bzw. durch eine Quellung der Wasserphase viskositätsaufbauend wirken, entstammen chemischsehr unterschiedlichen Stoffklassen.

Geeignete Verdickungsmittel für die erfindungsgemäßen Zubereitungen sind vernetzte Polyacrylsäuren und deren Derivate, Polysaccharide wie Xanthan-gum, Guar-Guar, Agar-Agar, Alginate oder Tylosen, Cellulosederivate, z. B. Carboxymethylcellulose oder Hydroxycarboxymethylcellulose, ferner höhermolekulare Polyethylenglycolmono-und -diester von Fettsäuren, Fettalkohole, Monoglyceride und Fettsäuren, Polyvinylalkohol und Polyvinylpyrrolidon.

Geeignete Verdicker sind weiterhin Polyacrylate wie Carbopol® (Noveon), Ultrez® (Noveon), Luvigel® EM (BASF), Capigel®98 (Seppic), Synthalene® (Sigma), die Aculyn®-Marken der Fa. Rohm und Haas wie Aculyn® 22 (Copolymerisat aus Acrylaten und Methacrylsäureethoxylaten mit Stearylrest (20 EO-Einheiten)) und Aculyn® 28 (Copolymerisat aus Acrylaten und Methacrylsäureethoxilaten mit Behenylrest (25 EO-Einheiten)).

Geeignete Verdickungsmittel sind weiterhin beispielsweise Aerosil-Typen (hydrophile Kieselsäuren), Polyacrylamide, Polyvinylalkohol und Polyvinylpyrrolidon, Tenside wie beispielsweise ethoxylierte Fettsäureglyceride, Ester von Fettsäuren rnit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Besonders bevorzugte Verdickungsmittel zur Herstellung von Gelen sind Ultrez®21, Aculyn®28, Luvigel® EM und Capigel®98.

Insbesondere bei höher konzentrierten Shampoo-Formulierungen können zur Regulierung der Konsistenz auch Stoffe zugesetzt werden, die die Viskosität der Formulierungverringern, wie z. B. Propylenglykol oder Glycerin. Diese Stoffe beeinflussen die Produkteigenschaften nur wenig.

Gelbildner

Wird für die erfindungsgemäßen Zubereitungen der Einsatz von Gelbildnern gewünscht, so können alle in der Kosmetik üblichen Gelbildner eingesetzt werden. Hierzu zählen leicht vernetzte Polyacrylsäure, beispielsweise Carbomer (INCI), Cellulosederivate, z.B. Hydroxypropylcellulose, Hydroxyethylcellulose, kationisch modifizierte Cellulosen, Polysaccharide, z.B. Xanthum Gummi, Caprylic/Capric Triglyceride, Sodium acrylates Copolymer, Polyquaternium-32 (and) Paraffinum Liquidum (INCI), Sodium Acrylates Copolymer (and) Paraffinum Liquidum (and) PPG-1 Trideceth-6, Acrylamidopropyl Trimonium Chloride/Acrylamide Copolymer, Steareth-10 Allyl Ether Acrylates Copolymer, Polyquaternium-37 (and) Paraffinum Liquidum (and) PPG-1 Trideceth-6, Polyquaternium 37 (and) Propylene Glycole Dicaprate Dicaprylate (and) PPG-1 Trideceth-6, Polyquaternium-7, Polyquaternium-44.

Emulgatoren

Als Emulgatoren kommen beispielsweise nichtionogene Tenside aus mindestens einer der folgenden Gruppen in Frage:

o Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;

o C12/18-Fettsäuremono- und -diester von Anlagerungsprodukten von 1 bis 30 Mol Ethylenoxid an Glycerin;

o Glycerinmono- und -diester und Sorbitanmono- und -diester von gesättigten und ungesättigten Fettsäuren mit 6 bis 22 Kohlenstoffatomen und deren Ethylenoxidanlagerungsprodukte;

o Alkylmono- und -oligoglycoside mit 8 bis 22 Kohlenstoffatomen im Alkylrest und deren ethoxylierte Analoga;

o Anlagerungsprodukte von 15 bis 60 Mol Ethylenoxid an Ricinusöl und/oder gehärtetes Ricinusöl;

o Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;

o Anlagerungsprodukte von 2 bis 15 Mol Ethylenoxid an Ricinusöl und/oder gehärtetes Ricinusöl;

o Partialester auf Basis linearer, verzweigter, ungesättigter bzw. gesättigter $C_6/_{22}$-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Me-thylglucosid, Butylglucosid, Laurylglucosid) sowie Polyglucoside (z.B. Cellulose);

o Mono-, Di- und Trialkylphosphate sowie Mono-, Di- und/oder Tri-PEG-alkylphosphate und deren Salze;

o Wollwachsalkohole;

o Polysiloxan-Polyalkyl-Polyether-Copolymere bzw. entsprechende Derivate;

o Mischester aus Pentaerythrit, Fettsäuren, Citronensäure und Fettalkohol gemäß DE-PS 1165574 und/oder Mischester von Fettsäuren mit 6 bis 22 Kohlenstoffatomen, Methylglycose und Polyolen, vorzugsweise Glycerin oder Polyglycerin sowie

o Polyalkylenglykole.

Die Anlagerungsprodukte von Ethylenoxid und/oder von Propylenoxid an Fettalkohole, Fettsäuren, Alkylphenole, Glycerinmono- und -diester sowie Sorbitanmono- und - diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. $C_{12}$ bis $C_{18}$-Fettsäuremono- und -diester von Anlagerungsprodukten von Ethylenoxid an Glycerin sind aus DE-PS 2024051 als Rückfettungsmittel für kosmetische Zubereitungen bekannt. $C_8$ bis $C_{18}$-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidesters gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und/oder eine Sulfonatgruppe tragen. Besonders geeignete zwitterionische Tenside sind die sogenannten Betaine wie die N-Alkyl-N,N-dimethylammonium-glycinate, beispielsweise das Kokosalkyldimethylammoniumglycinat, N-Acylamino-propyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokos-acylaminoethylhydroxyethylcarboxy-methylglycinat.

Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer $C_8$ bis $C_{18}$-Alkyl- oder -Acylgruppe im Molekül mindestens eine freie Aminogruppe und mindestens eine -COOH- und/oder -$SO_3$H-Gruppe enthalten und zur Ausbildung innerer

Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12}$ bis $C_{18}$-Acylsarcosin.

Neben den ampholytischen kommen auch quartäre Emulgatoren in Betracht, wobei solche vom Typ der Esterquats, vorzugsweise methylquaternierte Difettsäuretriethano-laminester-Salze, besonders bevorzugt sind.

Antioxidantien

Ein zusätzlicher Gehalt der Zubereitungen an Antioxidantien kann von Vorteil sein. Erfindungsgemäß können als Antioxidantien alle für kosmetische Anwendungen geeigneten oder gebräuchlichen Antioxidantien verwendet werden. Vorteilhaft werden die Antioxidantien gewählt aus der Gruppe bestehend aus Aminosäuren (z.B. Glycin, Histidin, Tyrosin, Tryptophan) und deren Derivaten, Imidazolen (z.B. Urocaninsäure) und deren Derivaten, Peptiden wie D,L-Carnosin, D-Carnosin, L-Carnosin und deren Derivaten (z.B. Anserin), Carotinoide, Carotinen (z.B. $\alpha$-Carotin, $\beta$-Carotin, $\gamma$-Lycopin) und deren Derivaten, Chlorogensäure und deren Derivaten, Liponsäure und deren Derivaten (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl- und Glycerylester) sowie deren Salzen, Dilaurylthiodipropionat, Distearylthiodipropionat, Thiodipropionsäure und deren Derivaten (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-Hydroxyfettsäuren, Palmitinsäure, Phytinsäure, Lactoferrin), $\alpha$-Hydroxysäuren (z. B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Coniferylbenzoat des Benzoeharzes, Rutinsäure und deren Derivate, $\alpha$-Glycosylrutin, Ferulasäure, Furfurylidenglucitol, Carnosin, Butylhydroxytoluol, Butylhydroxyanisol , Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist es vorteilhaft, diese in Konzentrationen von 0,001 bis 10 Gew.-%, bezogen auf das Gesamtgewicht der Zubereitung, bereitzustellen.

Sofern Vitamin A, bzw. Vitamin-A-Derivate, bzw. Carotine bzw. deren Derivate das oder die Antioxidantien darstellen, ist vorteilhaft, diese in Konzentrationen von 0,001 bis 10 Gew.-%, bezogen auf das Gesamtgewicht der Zubereitung, bereitzustellen.

Parfümöle

Die haut- oder haarkosmetischen Zubereitungen können Parfümöle enthalten. Als Parfümöle seien beispielsweise Gemische aus natürlichen und synthetischen Riechstoffen genannt. Natürliche Riechstoffe sind Extrakte von Blüten (Lilie, Lavendel, Rose, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orange), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, 4-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzy-lethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, cc-Isomethylionen und Methylcedrylketon, zu den Alkoholen Anethol,

Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzeöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen eingesetzt.

Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Silikonverbindungen

In einer Ausführungsform enthalten die erfindungsgemäßen Zubereitungen als haarpflegenden Zusatzstoff mindestens eine Silikonverbindung in einer Menge von vorzugsweise 0,01 bis 15 Gew.-%, besonders bevorzugt von 0,1 bis 5 Gew.-%. Die Silikonverbindungen umfassen flüchtige und nicht-flüchtige Silikone und in dem Mittel lösliche und unlösliche Silikone. Bei einer Ausführungsform handelt es sich um hochmolekulare Silikone mit einer Viskosität von 1.000 bis 2.000.000 cSt bei 25°C, vorzugsweise 10.000 bis 1.800.000 oder 100.000 bis 1.500.000. Die Silikonverbindungen umfassen Polyalkyl- und Polyarylsiloxane, insbesondere mit Methyl-, Ethyl-, Propyl-, Phenyl-, Methylphenyl- und Phenylmethylgruppen. Bevorzugt sind Polydimethylsiloxane, Polydiethylsiloxane, Polymethylphenylsiloxane. Bevorzugt sind auch glanzgebende, arylierte Silikone mit einem Brechungsindex von mindestens 1,46, oder mindestens 1,52. Die Silikonverbindungen umfassen insbesondere die Stoffe mit den INCI-Bezeichnungen Cyclomethicone, Dimethicone, Dimethiconol, Dimethicone Copolyol, Phenyl Trimethicone, Amodimethicone, Trimethylsilylamodimethicone, Stearyl Siloxysilicate, Polymethylsilsesquioxane, Dimethicone Crosspolymer. Geeignet sind auch Silikonharze und Silikonelastomere, wobei es sich um hochvernetzte Siloxane handelt.

Bevorzugte Silikone sind cyclische Dimethylsiloxane, lineare Polydimethylsiloxane, Blockpolymere aus Polydimethylsiloxan.und Polyethylenoxid und/oder Polypropylenoxid, Polydimethylsiloxane mit end- oder seitenständigen Polyethylenoxid- oder Polypropylenoxidresten, Polydimethylsiloxane mit endständigen Hydroxylgruppen, phenylsubstituierte Polydimethylsiloxane, Silikonemulsionen, Silkonelastomere, Silikonwachse, Silikongums und aminosubstituierte Silikone.

Haarkonditioniermittel

In einer Ausführungsform enthalten die erfindungsgemäßen Zubereitungen 0,01 bis 20, bevorzugt von 0,05 bis 10, besonders bevorzugt von 0,1 bis 5 Gew.-% mindestens eines Konditioniermittels.

Erfindungsgemäß bevorzugte Konditionierungsmittel sind beispielsweise alle Verbindungen, welche im International Cosmetic Ingredient Dictionary and Handbook (Volume 4, Herausgeber: R. C. Pepe, J.A. Wenninger, G. N. McEwen, The Cosmetic, Toiletry, and Fragrance Association, 9. Auflage, 2002) unter Section 4 unter den Stichworten Hair Conditioning Agents, Humectants, Skin-Conditioning Agents, Skin-Conditioning Agents-Emollient, Skin-Conditioning Agents-Humectant, Skin-Conditioning Agents-Miscellaneous, Skin-Conditioning Agents-Occlusive und Skin Protectans aufgeführt sind sowie alle in der EP-A 934 956 (S.11-13) unter "water soluble conditioning agent" und "oil soluble conditioning agent" aufgeführten Verbindungen. Weitere vorteilhafte Konditionierungsmittel stellen beispielsweise die nach INCI als Polyquaternium bezeichneten Verbindungen dar (insbesondere Polyquaternium-1 bis Polyquaternium-56).

Zu den geeigneten Konditionierungsmitteln zählen beispielsweise auch polymere quaternäre Ammoniumverbindungen, kationische Cellulosederivate, Chitosanderivate und Polysaccharide.

Das Konditioniermittel ist bevorzugt ausgewählt aus Betain, Panthenol, Panthenylethylether, Sorbitol, Proteinhydrolysaten, Pflanzenextrakten; A-B-Block-Copolymeren aus Alkylacrylaten und Alkylmethacryaten; A-B-Block-Copolymeren aus Alkylmethacrylaten und Acrylnitril; A-B-A-Block-Copolymeren aus Lactid und Ethylenoxid; A-B-A-Block-Copolymeren aus Caprolacton und Ethylenoxid; A-B-C-Block-Copolymeren aus Alky-len- oder Alkadienverbindungen, Styrol und Alkylmethacrylaten; A-B-C-Block-Copolymeren aus Acrylsäure, Styrol und Alkylmethacrylaten, sternförmigen Block-Copolymeren, hyperverzweigten Polymeren, Dendrimeren, intrinsisch elektrisch leitfähigen 3,4-Polyethylendioxythiophenen und intrinsisch elektrisch leitfähigen Polyanilinen.

Weitere erfindungsgemäß vorteilhafte Konditioniermittel stellen Cellulosederivate und quaternisierte Guargum Derivate, insbesondere Guar Hydroxypropylammoniumchlorid (z.B. Jaguar Excel®, Jaguar C 162® (Rhodia), CAS 65497-29-2, CAS 39421-75-5) dar. Auch nichtionische Poly-N-vinylpyrrolidon/Polyvinylacetat-Copolymere (z.B. Luviskol®VA 64 (BASF)), anionische Acrylat-Copolymere (z.B. Luviflex®Soft (BASF)), und/oder amphotere Amid/Acrylat/Methacrylat Copolymere (z.B. Amphomer® (National Starch)) können erfindungsgemäß vorteilhaft als Konditioniermittel eingesetzt werden.

Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind

- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methylolverbindungen, wie insbesondere Trimethylolethan, Trimetylolpropan, Trimetylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit;
- Zucker mit 5 bis 12 Kohlenstoffatomen, wie beispielsweise Glucose oder Saccharose;
- Aminozucker, wie beispielsweise Glucamin.

Öle, Fette und Wachse

Die erfindungsgemäßen haut- oder haarkosmetischen Zubereitungen können auch Öle, Fette oder Wachse enthalten. Solche werde vorteilhaft gewählt aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr. Weitere polare Ölkomponenten können gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat Dicaprylyl Carbonat (Cetiol CC) und Cocoglyceride (Myritol 331), Butylen Glycol Dicaprylat/Dicaprat und Dibutyl Adipat sowie synthetische, halbsynthetische und natürliche Gemische solcher Ester, wie z. B. Jojobaöl.

Ferner können eine oder mehrere Olkomponenten vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige

Lipidkomponente der Ölphase einzusetzen.

Erfindungsgemäß vorteilhaft wird die Olkomponente gewählt aus der Gruppe 2-Ethylhexylisostearat, Octyldodecanol, Isotridecylisononanoat, Isoeicosan, 2-Ethylhexylcocoat, C12-15-Alkylbenzoat, Capryl-Caprinsäure-triglycerid, Dicaprylylether.

Erfindungsgemäß vorteilhaft sind Mischungen aus C12-15-Alkylbenzoat und 2-Ethyl-hexylisostearat, Mischungen aus C12-15-Alkylbenzoat und Isotridecylisononanoat sowie Mischungen aus C12-15-Alkylbenzoat, 2-Ethylhexylisostearat und Isotridecylisononanoat.

Erfindungsgemäß besonders bevorzugt werden als Öle mit einer Polarität von 5 bis 50 mN/m Fettsäuretriglyceride, insbesondere Sojaöl und/oder Mandelöl eingesetzt.

Ferner kann die Ölphase vorteilhaft gewählt werden aus der Gruppe der Guerbet-alkohole. Diese sind selbst bei niederen Temperaturen flüssig und bewirken praktisch keine Hautreizungen. Vorteilhaft können sie als fettende, überfettende und auch rückfettend wirkende Bestandteile in kosmetischen Zusammensetzungen eingesetzt werden.

Die Verwendung von Guerbet-Alkoholen in Kosmetika ist an sich bekannt.

Erfindungsgemäß bevorzugte Guerbet-Alkohole sind 2-Butyloctanol (beispielsweise als Isofol®12 (Condea) kommerziell erhältlich) und 2-Hexyldecanol (beispielsweise als Isofol®16 (Condea) kommerziell erhältlich).

Auch Mischungen von erfindungsgemäßen Guerbet-Alkoholen sind erfindungsgemäß vorteilhaft zu verwenden wie beispielsweise Mischungen aus 2-Butyloctanol und 2-Hexyldecanol (beispielsweise als Isofol®14 (Condea) kommerziell erhältlich).

Auch beliebige Abmischungen solcher Öl- und Wachskomponenten sind vorteilhaft im Sinne der vorliegenden Erfindung einzusetzen. Unter den Polyolefinen sind Polydecene die bevorzugten Substanzen.

Erfindungsgemäß vorteilhaft zu verwendende Fett- und/oder Wachskomponenten können aus der Gruppe der pflanzlichen Wachse, tierischen Wachse, Mineralwachse und petrochemischen Wachse gewählt werden. Vorteilhaft sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse.

Weitere vorteilhafte Fett- und/oder Wachskomponenten sind chemisch modifzierte Wachse und synthetische Wachse, wie beispielsweise Syncrowax®HRC (Glyceryltribehenat), und Syncrowax®AW 1 C ($C_{18-36}$-Fettsäure) sowie Montanesterwachse, Sasolwachse, hydrierte Jojobawachse, synthetische oder modifizierte Bienenwachse (z. B. Dimethicon Copolyol Bienenwachs und/oder $C_{30-50}$-Alkyl Bienenwachs), Cetyl Ricinoleate wie beispielsweise Tegosoft®CR, Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride wie beispielsweise Hydriertes Soy Glycerid, Trihydroxystearin, Fettsäuren, Fettsäureester und Gly kolester wie beispielsweise $C_{20-40}$-Alkylstearat, $C_{20-40}$)-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan.

Erfindungsgemäß können die Fett- und/oder Wachskomponenten sowohl einzeln als auch als Gemisch in den Zusammensetzungen verwendet werden.

Auch beliebige Abmischungen solcher Öl- und Wachskomponenten sind vorteilhaft im Sinne der vorliegenden Erfindung einzusetzen.

Vorteilhaft wird die Ölphase gewählt aus der Gruppe 2-Ethylhexylisostearat, Octyldodecanol, Isotridecylisononanoat, Butylen Glycol Dicaprylat/Dicaprat, 2-Ethylhexylcocoat, $C_{12-15}$-Alkylbenzoat, Capryl-Caprin-säure-triglycerid, Dicaprylylether. Besonders vorteilhaft sind Mischungen aus Octyldodecanol, Capryl-Caprinsäure-triglycerid, Dicaprylylether, Dicaprylyl Carbonat, Cocoglyceriden oder Mischungen aus $C_{12-15}$-Alkylbenzoat und 2-Ethylhexylisostearat, Mischungen aus $C_{12-15}$-Alkylbenzoat und Butylen Glycol Dicaprylat/Dicaprat sowie Mischungen aus $C_{12-15}$-Alkylbenzoat, 2-Ethylhexylisostearat und Isotridecylisononanoat.

Von den Kohlenwasserstoffen sind Paraffinöl, Cycloparaffin, Squalan, Squalen, hydriertes Polyisobuten bzw. Polydecen vorteilhaft im Sinne der vorliegenden Erfindung zu verwenden.

Die Ölkomponente wird ferner vorteilhaft aus der Gruppe der Phospholipide gewählt. Als erfindungsgemäß vorteilhaftes Paraffinöl kann erfindungsgemäß Merkur®Weissoel Pharma 40 von Merkur Vaseline, Shell Ondina® 917, Shell Ondina®927, Shell Oil 4222, Shell Ondina®933 von Shell & DEA Oil, Pionier® 6301 S, Pionier® 2071 (Hansen & Rosenthal) eingesetzt werden.

Geeignete kosmetisch verträgliche Öl- und Fettkomponenten sind in Karl-Heinz Schrader, Grundlagen und Rezepturen der Kosmetika, 2. Auflage, Verlag Hüthig, Heidelberg, S. 319 - 355 beschrieben, worauf hier in vollem Umfang Bezug genommen wird.

Der Gehalt an Ölen, Fetten und Wachsen beträgt höchstens 30, bevorzugt 20, weiter bevorzugt höchstens 10 Gewichts-%, bezogen auf das Gesamtgewicht der Zusammensetzung.

Pigmente

In einer Ausführungsform enthalten die erfindungsmäßen Zubereitungen mindestens ein Pigment. Hierbei kann es sich um farbige Pigmente handeln, welche der Produktmasse oder dem Haar Farbeffekte verleihen oder es kann sich um Glanzeffektpigmente handeln, welche der Produktmasse oder dem Haar Glanzeffekte verleihen. Die Farb-oder Glanzeffekte am Haar sind vorzugsweise temporär, d.h. sie verbleiben bis zur nächsten Haarwäsche auf dem Haar und können durch Waschen der Haare mit üblichen Shampoos wieder entfernt werden.

Die Pigmente liegen in der Produktmasse in ungelöster Form vor und können in einer Menge von 0,01 bis 25 Gew.-%, besonders bevorzugt von 5 bis 15 Gew.-% enthalten sein. Die bevorzugte Teilchengröße beträgt 1 bis 200 μm, insbesondere 3 bis 150 μm, besonders bevorzugt 10 bis 100 μm. Die Pigmente sind im Anwendungsmedium praktisch unlösliche Farbmittel und können anorganisch oder organisch sein. Auch anorganisch-organische Mischpigmente sind möglich. Bevorzugt sind anorganische Pigmente. Der Vorteil der anorganischen Pigmente ist deren ausgezeichnete Licht-, Wetter- und Temperaturbeständigkeit. Die anorganischen Pigmente können natürlichen Ursprungs sein, beispielsweise hergestellt aus Kreide, Ocker, Umbra, Grünerde, gebranntem Terra di Siena oder Graphit. Bei den Pigmenten kann es sich um Weißpigmente wie z.B. Titandioxid oder Zinkoxid, um Schwarzpigmente wie z. B. Eisenoxidschwarz, Buntpigmente wie z.B. Ultramarin oder Eisenoxid rot, um Glanzpigmente , Metalleffekt-Pigmente, Perlglanzpigmente sowie um Fluoreszenz- oder Phosphoreszenzpigmente handeln, wobei vorzugsweise mindestens ein Pigment ein farbiges, nicht-weißes Pigment ist.

Geeignet sind Metalloxide, -hydroxide und -oxidhydrate, Mischphasenpigmente, schwefelhaltige Silicate, Metallsulfide, komplexe Metallcyanide, Metallsulfate, - Chromate und -molybdate sowie die Metalle selbst (Bronze-Pigmente). Geeignet sind insbesondere Titandioxid (CI 77891), schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI 77289), Eisenblau (Ferric Ferro-Cyanide, CI 7751 0), Carmine (Cochineal).

Besonders bevorzugt sind Perlglanz- und Farbpigmente auf Mica- bzw. Glimmerbasis welche mit einem Metalloxid oder einem Metalloxychlorid wie Titandioxid oder Wismutoxychlorid sowie gegebenenfalls weiteren farbgebenden Stoffen wie Eisenoxiden, Eisenblau, Ultramarine, Carmine etc. beschichtet sind und wobei die Farbe durch Variation der Schichtdicke bestimmt sein kann. Derartige Pigmente werden beispielsweise unter den Handelsbezeichnungen Rona®, Colorona®, Dichrona® und Timiron® von der Firma Merck, Deutschland vertrieben.

Organische Pigmente sind beispielsweise die natürlichen Pigmente Sepia, Gummigutt, Knochenkohle, Kasseler Braun, Indigo, Chlorophyll und andere Pflanzenpigmente. Synthetische organische Pigmente sind beispielsweise Azo-Pigmente, Anthrachinoide, Indigoide, Dioxazin-, Chinacridon-, Phtalocyanin-, Isoindolinon-, Perylen- und Perinon-, Metallkomplex-, Alkaliblau- und Diketopyrrolopyrrol-Pigmente.

In einer Ausführungsform enthalten die erfindungsmäßen Zubereitungen 0,01 bis 10, besonders bevorzugt von 0,05 bis 5 Gew.-% mindestens eines partikelförmigen Stoffes. Geeignete Stoffe sind z.B. Stoffe, die bei Raumtemperatur (25°C) fest sind und in Form von Partikeln vorliegen. Geeignet sind etwa Silica, Silikate, Aluminate, Tonerden, Mica, Salze, insbesondere anorganische Metallsalze, Metalloxide, z.B. Titandioxid, Minerale und Polymerpartikel.

Die Partikel liegen in dem Mittel ungelöster, vorzugsweise stabil dispergierter Form vor und können sich nach Aufbringen auf die Anwendungsoberfläche und Verdampfen des Lösungsmittels in fester Form abscheiden.

Bevorzugte partikelförmige Stoffe sind Silica (Kieselgel, Siliciumdioxid) und Metallsalze, insbesondere anorganische Metallsalze, wobei Silica besonders bevorzugt ist. Metallsalze sind z.B. Alkali- oder Erdalkalihalogenide wie Natriumchlorid oder Kaliumchlorid; Alkali- oder Erdalkalisulfate wie Natriumsulfat oder Magnesiumsulfat.

Geeignete Repellentwirkstoffe sind Verbindungen, die in der Lage sind, bestimmte Tiere, insbesondere Insekten, vom Menschen abzuhalten oder zu vertreiben. Dazu gehört z. B. 2-Ethyl-1,3-hexandiol, N,N-Diethyl-m-toluamid etc..

Geeignete hyperemisierend wirkende Stoffe, welche die Durchblutung der Haut anregen, sind z. B. ätherische Öle, wie Latschenkiefer, Lavendel, Rosmarin, Wacholderbeer, Rosskastanienextrakt, Birkenblätterextrakt, Heublumenextrakt, Ethylacetat, Campher, Menthol, Pfefferminzöl, Rosmarinextrakt, Eukalyptusöl, etc..

Geeignete keratolytisch und keratoplastisch wirkende Stoffe sind z. B. Salicylsäure, Kalziumthioglykolat, Thioglykolsäure und ihre Salze, Schwefel, etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion, etc..

Geeignete Antiphlogistika, die Hautreizungen entgegenwirken, sind z. B. Allantoin, Bisabolol, Dragosantol, Kamillenextrakt, Panthenol, etc..

Darreichungsform

Die erfindungsgemäßen Zubereitungen sind in einer bevorzugten Ausführungsform versprühbar, beispielsweise als Aerosol- oder Pumpspray-Zubereitung.

Die erfindungsgemäßen Zubereitungen können in verschiedenen Applikationsformen Anwendung finden, wie

beispielsweise als Lotion, als Non-Aerosol Sprühlotion, welches mittels einer mechanischen Vorrichtung zum Versprühen zum Einsatz kommt, als Aerosol-Spray welches mittels eines Treibmittels versprüht wird, als Aerosol-Schaum oder als Non-Aerosol Schaum, welcher in Kombination mit einer geeigneten mechanischen Vorrichtung zum Verschäumen der Zusammensetzung vorliegt, als Haarcreme, als Haarwachs, als Gel, als Flüssiggel, als versprühbares Gel oder als Schaumgel.

Auch ein Einsatz in Form einer mit einem üblichen Verdicker verdickten Lotion ist möglich.

In einer Ausführungsform liegt das erfindungsgemäße Mittel in Form eines Gels, in Form einer viskosen Lotion oder in Form eines Sprühgels, welches mit einer mechanischen Vorrichtung versprüht wird, vor und enthält mindestens eines der oben genannten Verdickungsmittel in einer Menge von vorzugsweise 0,05 bis 10, besonders bevorzugt von 0,1 bis 2 Gew.-% und weist eine Viskosität von mindestens 250 mPas auf. Die Viskosität des Gels beträgt vorzugsweise von 500 bis 50.000 mPas, besonders bevorzugt von 1.000 bis 15.000 mPas bei 25°C.

In einer anderen Ausführungsform liegt die erfindungsgemäße Zubereitung in Form einer O/W-Emulsion, einer W/O-Emulsion oder einer Mikroemulsion vor und und enthält mindestens eines der oben genannten, in Wasser emulgierten Öle oder Wachse sowie mindestens ein kosmetisch übliches Tensid.

In einer bevorzugten Ausführungsform liegt die erfindungsgemäße Zubereitung in Form eines Sprühproduktes vor, entweder in Kombination mit einer mechanischen Pumpsprühvorrichtung oder in Kombination mit mindestens einem der vorgenannten Treibmittel. Ein bevorzugten Aerosolspray enthält zusätzlich Treibmittel in einer solchen Menge, dass die Gesamtmenge der flüchtigen organischen Komponenten 80, insbesondere 55 Gew.-% der Zubereitung nicht übersteigt und wird in einem Druckbehälter abgefüllt.

Ein Non-Aerosol-Haarspray wird mit Hilfe einer geeigneten mechanisch betriebenen Sprühvorrichtung versprüht. Unter mechanischen Sprühvorrichtungen sind solche Vorrichtungen zu verstehen, welche das Versprühen einer Zusammensetzung ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem die erfindungsgemäße haarkosmetische Zubereitung unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

In einer weiteren Ausführungsform liegt die erfindungsgemäße Zubereitung in Form eines verschäumbaren Produktes (Mousse) in Kombination mit einer Vorrichtungzum Verschäumen vor, enthält mindestens eine übliche, hierfür bekannte schaumgebende Substanz, z.B. mindestens ein schaumbildendes Tensid oder mindestens ein schaumbildendes Polymer. Unter Vorrichtungen zum Verschäumen sind solche Vorrichtungen zu verstehen, welche das Verschäumen einer Flüssigkeit mit oder ohne Verwendung eines Treibmittels ermöglichen. Als geeignete mechanische Schäumvorrichtung kann beispielsweise ein handelsüblicher Pumpschäumer oder ein Aerosolschaumkopf verwendet werden. Das Produkt liegt entweder in Kombination mit einer mechanischen Pumpschäumvorrichtung (Pumpschaum) oder in Kombination mit mindestens einem Treibmittel (Aerosol-Schaum) in einer Menge von vorzugsweise 1 bis 20, insbesondere von 2 bis 10 Gew.-%, vor. Treibmittel sind z.B. ausgewählt aus Propan, Butan, Dimethylether und fluorierten Kohlenwasserstoffen.

Ein Gegenstand der Erfindung ist somit eine haarkosmetische Zubereitung in Form eines Sprühproduktes, wobei die Zubereitung entweder in Kombination mit einer mechanischen Pumpsprühvorrichtung oder in Kombination mit mindestens einem Treibmittel ausgewählt aus der Gruppe bestehend aus Propan, Butan, Dimethylether, fluorierten Kohlenwasserstoffen und deren Mischungen vorliegt.

Das Mittel wird unmittelbar vor der Anwendung verschäumt und als Schaum in das Haar eingearbeitet und kann anschließend ausgespült werden oder ohne Ausspülen im Haar belassen werden.

In einer weiteren Ausführungsform liegt die erfindungsgemäße Zubereitung in Form eines Haarwachses vor, d.h. sie weist wachsartige Konsistenz auf und enthält mindestens eines der oben genannten Wachse in einer Menge von vorzugsweise 0,5 bis 30 Gew.-% sowie gegebenenfalls weitere wasserunlösliche Stoffe. Die wachsartige Konsistenz ist vorzugsweise dadurch gekennzeichnet, dass die Nadelpenetrationszahl (Maßeinheit 0,1 mm, Prüfgewicht 100 g, Prüfdauer 5 s, Prüftemperatur 25°C; nach DIN 51 579) größer oder gleich 10, besonders bevorzugt größer oder gleich 20 ist und dass der Erstarrungspunkt des Produktes vorzugsweise größer oder gleich 30°C und kleiner oder gleich 70°C ist, besonders bevorzugt im Bereich von 40 bis 55°C liegt. Geeignete Wachse und wasserunlösliche Stoffe sind insbesondere Emulgatoren mit einem HLB-Wert unterhalb von 7, Silikonöle, Silikonwachse, Wachse (2.B. Wachsalkohole, Wachssäuren, Wachsester, sowie insbesondere natürliche Wachse wie Bienenwachs, Carnaubawachs, etc.), Fettalkohole, Fettsäuren, Fettsäureester oder hydrophile Wachse wie z.B. hochmolekulare Polyethylenglykole mit einem Molekulargewicht von 800 bis 20.000, vorzugsweise von 2.000 bis 10.000 g/mol.

Wenn die erfindungsgemäße haarkosmetische Zubereitung in Form einer Haarlotion vorliegt, so liegt sie als im wesentlichen nicht-viskose oder gering viskose, fließfähige Lösung, Dispersion oder Emulsion mit einem Gehalt an mindestens 10 Gew.-%, vorzugsweise 20 bis 95 Gew.-% eines kosmetisch verträglichen Alkohols vor. Als Alkohole können insbesondere die für kosmetische Zwecke üblicherweise verwendeten niederen Alkohole mit 1 bis 4 C-Atomen, z.B. Ethanol und Isopropanol verwendet werden.

Wenn die erfindungsgemäße haarkosmetische Zubereitung in Form einer Haarcreme vorliegt, so liegt sie vorzugsweise als Emulsion vor und enthält entweder zusätzlich viskositätsgebende Inhaltsstoffe in einer Menge von 0,1 bis 10 Gew.-% oder die erforderliche Viskosität und cremige Konsistenz wird durch Micellbildung mit Hilfe von geeigneten Emulgatoren, Fettsäuren, Fettalkoholen, Wachsen etc. in üblicher Weise aufgebaut.

Hautkosmetische Mittel

Nach einer weiteren bevorzugten Ausführungsform handelt es sich bei den erfindungsgemäßen Zubereitungen um hautkosmetische Zubereitungen, also Zubereitungen zur Pflege und zum Schutz der Haut oder Zubereitungen für die dekorative Kosmetik.

Zubereitungen für die Nagelpflege sind nicht Gegenstand dieser Erfindung.

Geeignete hautkosmetische Zubereitungen sind z. B. Gesichtswässer, Gesichtsmasken, Deodorantien und andere kosmetische Lotionen. Mittel für die Verwendung in der dekorativen Kosmetik umfassen beispielsweise Abdeckstifte, Theaterfarben, Mascara und Lidschatten, Lippenstifte, Kajalstifte, Eyelinern, Rouges, Puder und Augenbrauenstifte.

Außerdem können die Polymere A in Nose-Strips zur Porenreinigung, in Antiaknemitteln, Repellents, Rasiermitteln, Haarentfernungsmitteln, Intimpflegemitteln, Fußpflegemitteln sowie in der Babypflege verwendet werden.

Bei den erfindungsgemäßen Hautpflegemitteln handelt es sich insbesondere um W/O-oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen.

Hautkosmetische und dermatologische Mittel auf Basis der zuvor beschriebenen Polymere A zeigen vorteilhafte Wirkungen. Die Polymere können unter anderem zur Verbesserung des Hautgefühls beitragenDurch Zusatz der erfindungsgemäßen Polymere kann in bestimmten Formulierungen eine erhebliche Verbesserung der Hautverträglichkeit erreicht werden.

Hautkosmetische und dermatologische Zubereitungen enthalten vorzugsweise wenigstens ein Polymer A in einem Anteil von etwa 0,001 bis 30 Gew.-%, vorzugsweise 0,01 bis 20 Gew.-%, ganz besonders bevorzugt 0,1 bis 12 Gew.-%, bezogen auf das Gesamtgewicht der Zubereitung.

Besonders Lichtschutzmittel auf Basis der Polymere A besitzen die Eigenschaft, die Verweilzeit der UV-absorbierenden Inhaltsstoffe im Vergleich zu gängigen Hilfsmitteln wie Polyvinylpyrrolidon zu erhöhen.

Je nach Anwendungsgebiet können die erfindungsgemäßen Zubereitung in einer zur Hautpflege geeigneten Form, wie z. B. als Creme, Schaum, Gel, Stift, Mousse, Milch, Spray (Pumpspray oder treibmittelhaltiger Spray) oder Lotion appliziert werden.

Die hautkosmetischen Zubereitungen können neben den Polymeren A und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Färbemittel, Tönungsmittel, Bräunungsmittel, Collagen, Eiweißhydrolysate, Stabilisatoren, pH-Wert-Regulatoren, Farbstoffe, Salze, Verdicker, Gelbildner, Konsistenzgeber, Silicone, Feuchthaltemittel, Rückfetter und weitere übliche Additive. Die Inhaltsstoffe für die hautkosmetischen Zubereitungen können auch aus den zuvor für die haarkosmetischen Zubereitungen genannten Inhaltsstoffen gewählt werden.

Bevorzugte Öl- und Fettkomponenten der hautkosmetischen und.dermatologischen Zubereitungen sind die zuvor genannten mineralischen und synthetischen Öle, wie z.B. Paraffine, Siliconöle und aliphatische Kohlenwasserstoffe mit mehr als 8 Kohlenstoffatomen, tierische und pflanzliche Öle, wie z. B. Sonnenblumenöl, Kokosöl, Avocadoöl, Olivenöl, Lanolin, oder Wachse, Fettsäuren, Fettsäureester, wie z. B. Triglyceride von $C_6$-$C_{30}$-Fettsäuren, Wachsester, wie z. B. Jojobaöl, Fettalkohole, Vaseline, hydriertes Lanolin und acetyliertes Lanolin sowie Mischungen davon.

Man kann die erfindungsgemäßen Polymere auch mit herkömmlichen Polymeren abmischen, falls spezielle Eigenschaften eingestellt werden sollen.

Zur Einstellung bestimmter Eigenschaften wie z. B. Verbesserung des Anfassgefühls, des Spreitverhaltens, der Wasserresistenz und/oder der Bindung von Wirk- und Hilfsstoffen, wie Pigmenten, können die hautkosmetischen und dermatologischen Zubereitungen zusätzlich auch konditionierende Substanzen auf Basis von Siliconverbindungen enthalten. Geeignete Siliconverbindungen sind beispielsweise Polyalkylsiloxane, Polyarylsiloxane, Polyarylalkylsiloxane, Polyethersiloxane oder Siliconharze.

Die Herstellung der hautkosmetischen oder dermatologischen Zubereitungen erfolgt nach üblichen, dem Fachmann bekannten Verfahren.

Bevorzugt liegen die hautkosmetischen und dermatologischen Mittel in Form von Emulsionen insbesondere als Wasser-in-Öl-(W/O)- oder Öl-in-Wasser(O/W)-Emulsionen vor. Es ist aber auch möglich, andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen, usw.

Die Herstellung von Emulsionen erfolgt nach bekannten Methoden. Die Emulsionen enthalten neben wenigstens

einem Polymer A in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion, z.B. für eine Hautcreme etc., enthält im Allgemeinen eine wässrige Phase, die mittels eines geeigneten Emulgatorsystems in einer Öl- oder Fettphase emulgiert ist.

Der Anteil des Emulgatorsystems beträgt in diesem Emulsionstyp bevorzugt etwa von 4 bis 35 Gew.-%, bezogen auf das Gesamtgewicht der Emulsion. Vorzugsweise beträgt der Anteil der Fettphase etwa 20 bis 60 Gew.-%. Vorzugsweise beträgt der Anteil der wässrigen Phase etwa 20 und 70 %, jeweils bezogen auf das Gesamtgewicht der Emulsion. Bei den Emulgatoren handelt es sich um solche, die in diesem Emulsionstyp üblicherweise verwendet werden. Sie werden z. B. ausgewählt unter: $C_{12}$-$C_{18}$-SorbitanFettsäureestern; Estern von Hydroxystearinsäure und $C_{12}$-$C_{30}$-Fettalkoholen; Mono- und Diestern von $C_{12}$-$C_{18}$-Fettsäuren und Glycerin oder Polyglycerin; Kondensaten von Ethylenoxid und Propylenglykolen; oxypropylenierten/ oxyethylierten $C_{12}$-$C_{18}$-Fettalkoholen; polycyclischen Alkoholen, wie Sterolen; aliphatischen Alkoholen mit einem hohen Molekulargewicht, wie Lanolin; Mischungen von oxypropylenierten/polyglycerinierten Alkoholen und Magnesiumisostearat; Succinestern von polyoxyethylenierten oder polyoxypropylenierten Fettalkoholen; und Mischungen von Magnesium-, Calcium-, Lithium-, Zink- oder Aluminiumlanolat und hydriertem Lanolin oder Lanolinalkohol.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karite-Öl, Hoplostethus-Öl; mineralische Öle, deren Destillationsbeginn unter Atmosphärendruck bei ca. 250°C und deren Destillationsendpunkt bei 410 oC liegt, wie z. B. Vaselinöl; Ester gesättigter oder ungesättigter Fettsäuren, wie Alkylmyristate, z. B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglykol-Copolymer, Fettsäuren und Fettalkohole enthalten.

Um die Retention von Ölen zu begünstigen, können neben den Polymeren A auch Wachse verwendet werden, wie z. B. Carnaubawachs, Candilillawachs, Bienenwachs, mikrokristallines Wachs, Ozokeritwachs und Ca-, Mg- und Al-Oleate, -Myristate,-Linoleate und -Stearate.

Im Allgemeinen werden die Wasser-in-Öl-Emulsionen so hergestellt, dass die Fettphase und der Emulgator in einen Ansatzbehälter gegeben werden. Man erwärmt diesen bei einer Temperatur von etwa 50 bis 75°C, gibt dann die in Öl löslichen Wirkstoffe und/oder Hilfsstoffe zu und fügt unter Rühren Wasser hinzu, welches vorher etwa auf die gleiche Temperatur erwärmt wurde und worin man gegebenenfalls die wasserlöslichen Ingredienzien vorher gelöst hat. Man rührt, bis man eine Emulsion der gewünschten Feinheit erhält und lässt dann auf Raumtemperatur abkühlen, wobei gegebenenfalls weniger gerührt wird.

Weiterhin kann eine erfindungsgemäße Pflegeemulsion als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt.

Die wässrige Phase der O/W-Emulsion der erfindungsgemäßen Zubereitungen enthält gegebenenfalls:

- Alkohole, Diole oder Polyole sowie deren Ether, vorzugsweise Ethanol, Isopropanol, Propylenglycol, Glycerin, Ethylenglycolmonoethylether;
- übliche Verdickungsmittel bzw. Gelbildner, wie z. B. vernetzte Polyacrylsäuren und deren Derivate, Polysaccharide wie Xanthan Gum oder Alginate, Carboxymethylcellulose oder Hydroxycarboxymethylcellulose, Fettalkohole, Polyvinylalkohol und Polyvinylpyrrolidon.

Die Ölphase enthält in der Kosmetik übliche Ölkomponenten, wie beispielsweise:

- Ester aus gesättigten und/oder ungesättigten, verzweigten und/oder unverzweigten $C_3$-$C_{30}$-Alkancarbonsäuren und gesättigten und/oder ungesättigten, verzweigten und/oder unverzweigten $C_3$-$C_{30}$-Alkoholen, aus aromatischen Carbonsäuren und gesättigten und/oder ungesättigten, verzweigten und/oder unverzweigten $C_3$-$C_{30}$-Alkoholen, beispielhaft Isopropylmyristat, Isopropylstearat, Hexyldecylstearat, Oleyloleat; außerdem synthetische, halbsynthetische und natürliche Gemische solcher Ester, wie Jojobaöl;
- verzweigte und/oder unverzweigte Kohlenwasserstoffe und -wachse;

- Silikonöle wie Cyclomethicon, Dimethylpolysiloxan, Diethylpolysiloxan, Octamethylcyclotetrasiloxan sowie Mischungen daraus;
- Dialkylether;
- Mineralöle und Mineralwachse;
- Triglyceride gesättigter und/oder ungesättigter, verzweigter und/oder unverzweigter $C_8$-$C_{24}$-Alkancarbonsäuren; sie können ausgewählt werden aus synthetischen, halbsynthetischen oder natürlichen Ölen, wie Olivenöl, Palmöl, Mandelöl oder Mischungen.

Als Emulgatoren kommen vorzugsweise O/W-Emulgatoren, wie Polyglycerinester, Sorbitanester oder teilveresterte Glyceride, in Betracht.
Die Herstellung kann durch Aufschmelzen der Ölphase bei ca. 80°C erfolgen; die wasserlöslichen Bestandteile werden in heißem Wasser gelöst, langsam und unter Rühren zur Ölphase zugegeben; homogenisiert und kaltgerührt.
Die nachfolgenden nicht einschränkenden Beispiele sollen den Gegenstand der Erfindung näher erläutern.

Messmethoden

Bestimmung des K-Wertes

Die K-Werte werden nach Fikentscher, Cellulosechemie, Bd. 13, S. 58 bis 64 (1932) bei 25°C in N-Methylpyrrolidon-(NMP)-Lösung gemessen und stellen ein Maß für das Molgewicht dar. Die NMP-Lösung der Polymere enthält 1g Polymer in 100 ml Lösung. Für den Fall, dass die Polymere in Form von wässrigen Dispersionen vorliegen, werden in Abhängigkeit vom Polymergehalt der Dispersion entsprechende Mengen der Dispersion mit NMP auf 100 ml aufgefüllt, so dass die Konzentration von 1g Polymer in 100 ml Lösung entstehen.
Die Messung des K-Wertes erfolgt in einer Micro-Ubbelohde-Kapillare Typ M Ic der Fa. Schott.
Bestimmumg der Tröpfchengrößenverteilung (TGV) mittels Malvern®-Streulichtanalyse Die Bestimmung der Tröpfchengrößenverteilung wurde mit Partikelgrößenmeßsystem zur Erfassung von Flüssigkeits-Aerosolen "Malvern®Master Sizer X" vorgenommen (Malvern Instruments Inc., Southborough MA, USA).

Messprinzip:

Das Messsystem beruht auf der Methode der Laserlicht-Beugung am Partikel, die sich außer zur Spray-Analyse (Aerosole, Pumpsprays) auch zur Größenbestimmung von Feststoffen, Suspensionen und Emulsionen im Größenbereich von 0,1 μm bis 2000μm eignet.
Ein Partikelkollektiv (=Tröpfchen) wird von einem Laser beleuchtet. An jedem Tröpfchen wird ein Teil des einfallenden Laserlichtes gestreut. Dieses Licht wird an einem Multielement-Detektor empfangen und die dazugehörige Lichtenergieverteilung bestimmt. Aus diesen Daten wird über die Auswertesoftware die dazugehörige Partikelverteilung berechnet.

Durchführung:

Die Aerosole wurden in einem Abstand von 29,5 cm zum Laserstrahl eingesprüht. Der Sprühkegel trat rechtwinklig zum Laserstrahl ein.
Die Aerosoldosen wurden vor jeder Messung an einer fest installierten Haltevorrichtung fixiert, somit wurde erreicht, dass alle zu prüfenden Aerosole im exakt gleichen Abstand vermessen wurden.
Vor der eigentlichen Partikelmessung wurde eine "Hintergrundmessung" durchgeführt. Dadurch wurden die Auswirkungen von Staub und anderen Verschmutzungen im Messbereich eliminiert.
Anschließend wurde das Aerosol in den Prüfraum eingesprüht. Das gesamte Partikel-volumen wurde über eine Prüfdauer von 2s erfasst und ausgewertet.

Auswertung:

Die Auswertung enthält eine tabellarische Darstellung über 32 Klassenbreiten von 0,5 μm bis 2000 μm und zusätzlich eine graphische Darstellung der Partikelgrößenverteilung.
Da es sich bei den Sprühversuchen um eine annähernd gleichmäßige Verteilung handelt, wird der mittlere Durchmesser "Mean Diameter" D(v,0.5) angegeben. Dieser Zahlenwert gibt an, dass 50 % des gesamt gemessenen

Partikelvolumens unterhalb dieses Wertes liegt.

Bei gut sprühbaren Aerosolsystemen im kosmetischen Bereich liegt dieser Wert, je nach Polymergehalt, Ventil- und Sprühkopfgeometrie, Lösemittelverhältnis und Treibgasmengen im Bereich von 30 μm bis 80 μm.

Bestimmung der Festigung (Biegesteifigkeit):

Die Festigung von polymeren Filmbildnern wurde außer durch subjektive Beurteilung auch objektiv physikalisch als Biegesteifigkeit von dünnen Haarsträhnen gemessen, die mit der Polymerlösung behandelt und wieder getrocknet wurden. Dabei bestimmt ein Kraftaufnehmer die zum Biegen erforderliche Kraft, während die gesamte Messung unter standardisierten Bedingungen in einem Klimaraum bei 65 % relativer Luftfeuchte abläuft.

Zur Messung der Biegesteifigkeit wurden 3,0 gew.-%ige Lösungen der erfindungsgemäßen Polymere hergestellt. Die Messung der Biegesteifigkeit wurde an 5 bis 10 Haarsträhnen (jeweils ca. 3 g und 24 cm Länge) bei 20°C und 65 % relativer Feuchte durchgeführt. Die gewogenen, trockenen Haarsträhnen wurden in die 3,0 gew.-%ige Polymerlösung getaucht, wobei durch dreimaliges Eintauchen und Herausnehmen anschließend durch Ausdrücken zwischen Filterpapier sorgfältig ausgedrückt. Danach eine gleichmäßige Verteilung sichergestellt wurde. Die überschüssige Filmbildnerlösung wurde dann zwischen Daumen und Zeigefinger abgestreift und die Haarsträhnen wurden die Strähnen von Hand so geformt, dass sie einen runden Querschnitt erhielten.

Bei 20°C und 65 % relativer Feuchte wurde über Nacht im Klimaraum getrocknet.

Die Prüfungen wurden in einem Klimaraum bei 20°C und 65 % relativer Feuchte mittels eines Zug/Druck-Prüfgerätes durchgeführt. Die Haarsträhne wurde symmetrisch auf zwei zylindrische Rollen der Probenaufnahme gelegt. Genau in der Mitte wurde die Strähne nun von oben mit einem abgerundetem Stempel 40 mm durchgebogen (Brechen des Polymerfilms). Die dafür erforderliche Kraft wurde mit einer Wägezelle (50 N) gemessen und in Newton angegeben. Die so ermittelten Werte wurden zu denen eines handelsüblichen Vergleichspolymers (Amphomer®LV 71 als Standard 100%, wie angegeben) in Relation gesetzt.

Bestimmung der Auswaschbarkeit:

Eine analog zur Bestimmung der Festigung mit Polymer behandelte Haarsträhne wurde in einer ca. 37°C warmen Texapon®NSO - Lösung (6ml Texa-pon®NSO (28 %ig) in 1l warmes Wasser) ca. 15 Sekunden durch 5maliges Eintauchen und Ausdrücken gewaschen. Anschließend wurde die Haarsträhne klargespült und nochmals in der gleichen Art behandelt. Danach wurde die Haarsträhne auf Filterpapier gut ausgedrückt und über Nacht trocknen gelassen. Die trockene Haarsträhne wurde eingedreht und auf Rückstände untersucht.

Bestimmung der Curl Retention

Grundrezeptur: (ohne 10 % Treibgas)

2,0 Gew.-% Wirkstoff zu prüfendes Polymer
2,0 Gew.-% Luviquat®Mono LS
0,2 Gew.-% Parfümöl
0,1 Gew.-% Euxyl®K 100
ad 90,0 Gew.-% VE-Wasser.

Für jedes zu prüfende Polymer wurden 180 g einer solchen Lösung hergestellt. Für die Bestimmung der Curl Retention wurden Haarsträhnen von ca. 2 g Gewicht und 15,5 cm Länge und aus mittelbraunem, kaukasischem Menschenhaar verwendet.

Behandlung der Haarsträhnen:

Die Haarsträhnen wurden zweimal mit einer wässrigen Texapon®NSO-Lösung gewaschen. Anschließend wurden die Haarsträhnen mit warmem Wasser ausgespült, bis keine Schaumbildung mehr erkennbar war und mit VE-Wasser nachgespült, gekämmt und auf Filterpapier zum Trocknen gelegt. Vor der Messung wurden die Haarsträhnen für ca. 1 Stunde in kaltem VE-Wasser gelagert.

Die feuchte Haarsträhne wurde zwischen Filterpapier ausgedrückt, in die Polymerlösung (siehe oben) eingetaucht, mit den Fingern abgestreift und wieder zwischen Filterpapier ausgedrückt. Dieser Prozess wurde insgesamt dreimal durchgeführt.

Danach wurde das Haar um einen Teflonstab von 12 mm Durchmesser gewickelt und mit Filterpapier und Gummiring befestigt. Anschließend wurden die Haarsträhnen ca. 90 min bei 70 - 80°C im Wärmeschrank getrocknet.

Nach Abkühlen auf Raumtemperatur wurden die Locken unter Erhalt der Form abgestreift und an einem Gestell aufgehängt und die Lockenlänge ($L_0$) bestimmt.

Für die Bestimmung eines Curl-Retention-Wertes wurden 5 Haarlocken verwendet. Die Locken wurden hängend in eine Klimakammer mit 20°C und 75 % bzw. 90 % rel. Luftfeuchte gestellt. Nach 5 Stunden wurde die Länge ($L_t$) abgelesen.

Die Curl Retention errechnet sich wie folgt:

$$\text{Curl Retention in \%} = \frac{L - L_t}{L - L_o} * 100$$

L = Länge der Haare (15,5 cm)

$L_0$ = Länge der Haarlocke nach dem Trocknen

$L_t$ = Länge der Haarlocke nach Klimabehandlung

Als Curl Retention wird der Mittelwert aus den 5 Einzelmessungen nach 5 h bei 20°C und 75 % bzw. 90% rel. Feuchte angegeben.

Bestimmung der Klebrigkeit

Es wird zunächst eine klare, 20 Gew.-%ige ethanolische oder ethanolisch/wässrige Lösung des zu charakterisierenden Polymers hergestellt. Um eine klare Lösung zu erhalten, ist es gegebenenfalls notwendig, das Polymer zu neutralisieren. Aus der e-thanolischen oder ethanolisch/wässrigen Lösung wird dann mit einem Rakel (120 µm Spaltbreite) ein Film des Polymers auf einer Glasplatte aufgebracht. Diese rechteckige Glasplatte hat eine Länge von ca. 20 cm und eine Breite von ca. 6,5 m. Der darauf aufgebrachte Polymerfilm hat eine Länge von ca. 16 bis 18 cm und eine Breite von ca. 5,5 cm.

Der Film wird dann ca. 10 Stunden an der Luft getocknet und anschließend im Klimaschrank bei 20°C und 80% relativer Feuchte weitere 12 Stunden gelagert.

Bei diesen Bedingungen wird dann im Klimaschrank ein sich auf einem runden Gummistempel (Durchmesser 400 mm, Shore A-Härte 60 ± 5) befindendes Plastic-Carbon-Band (z.B. Pelikan®2060, 50 mm breit) mit einer Kraft von ca. 250 N für 10 Sekunden auf den Polymerfilm gepresst.

Die Menge des nach Entfernen des Stempels auf dem Polymerfilm haftend verbliebenen Schwarz-Pigmentes entspricht der Klebrigkeit des Filmes. Es erfolgt eine visuelle Beurteilung der Schwarzfärbung des Filmes. Die Beurteilungsskala reicht von 0 bis 5, wobei 0 nicht klebrig und 5 sehr stark klebrig bedeuten.

Bestimmung des Aussehens der Aerosol-Formulierung

Die Zubereitung aus 5 Gew.-% des jeweiligen Polymers neutralisiert mit AMP, 40 Gew.-% DME, 15 Gew.-% Ethanol und 40 Gew.-% Wasser wurden in einen transparenten Glas-Aerosolbehälter gefüllt. Das Treibgas wurde zugegeben und die Klarheit des resultierenden Flüssigkeits/Treibgasgemisches visuell beurteilt.

Beispiele

Die folgenden Beispiele erläutern die Erfindung, ohne sie darauf zu beschränken. Verwendete Abkürzungen:

| | |
|---|---|
| t-BA | tert.-Butylacrylat |
| MAS | Methacrylsäure |
| AS | Acrylsäure |
| ITS | Itaconsäure |
| EA | Ethylacrylat |
| MMA | Methylmethacrylat |
| EMA | Ethylmethacrylat |
| t-BMA | tert.-Butylmethacrylat |

(fortgesetzt)

| | |
|---|---|
| i-BMA | Isobutylmethacrylat |
| VE | voll entsalzt |

Herstellung von Urethanacrylat c26 (Komponente c)

In einem Rundkolben wurden 672,0g eines Polyesters aus Adipinsäure und Neopentylglykol mit einer OH-Zahl von ca. 200, 140,0g Hydroxyethylacrylat, 0,6 g Hydrochinon-monomethylether, 1,20g 2,6, Di-tert. Butyl-4-methylphenol, 0,12 g Tetrabutylortho-titanat vorgelegt und auf 50°C aufgeheizt. Dann wurden 400, 0 g Isophorondiisocyanat innerhalb 30 Minuten zugetropft. Man ließ weitere 7 Stunden bei 90-95°C reagieren, wobei der NCO-Gehalt auf 0,56% abfiel. Es wurde auf 60°C abgekühlt, dann wurden 520,0 g Ethanol zugegeben und ca. 2 Stunden bei 65-70°C weiterreagieren lassen bis der Isocyanatgehalt (NCO-Wert) auf 0 abgefallen war. Das erhaltene Harz wurde über einen 50 µm Filter filtriert und abgefüllt.

Herstellung von Urethanacrylat c27 (Komponente c)

In einem Rundkolben wurden 672,0g eines Polyesters aus Adipinsäure und Neopentylglykol mit einer OH-Zahl von ca. 200, 140,0g Hydroxyethylacrylat, 0,6g Hydrochinonmonomethylether, 1,21g 2,6, Di-tert. Butyl-4-methylphenol, 0,12g Cesium acetat vorgelegt und auf 50°C aufgeheizt. Dann wurden 400,0g Isophorondiisocyanat innerhalb 30 Minuten zugetropft. Man ließ weitere 7 Stunden bei 90-95°C reagieren, wobei der NCO-Gehalt auf 0,56% abfiel. Es wurde auf 60°C abgekühlt, dann wurden 520,0g Ethanol zugegeben und ca. 2 Stunden bei 65-70°C weiterreagieren lassen bis der Isocyanatgehalt (NCO-Wert) auf 0 abgefallen war. Das erhaltene Harz wurde über einen 50 µm Filter filtriert und abgefüllt.

Herstellung von Urethanacrylat c28 (Komponente c)

In einem Rundkolben wurden 672,0g eines Polyesters aus Adipinsäure und Neopentylglykol mit einer OH Zahl von ca. 200, 140,0g Hydroxyethylacrylat, 0,6g Hydrochinonmonomethylether, 1,21g 2,6, Di-tert. Butyl-4-methylphenol vorgelegt und auf 50°C aufgeheizt. Dann wurden 400,0g Isophorondiisocyanat innerhalb 30 Minuten zugetropft. Man ließ weitere 20 Stunden bei 90-95°C reagieren, wobei der NCO-Gehalt auf 0,1 % abfiel. Es wurde auf 60°C abgekühlt, dann wurden 10,0 g Methanol zugegeben und ca. 4 Stunden bei 90-95°C weiterreagieren lassen bis der Isocyanatgehalt (NCO-Wert) auf 0 abgefallen war. Das erhaltene Harz wurde bei Raumtemperatur mit 510,0g Tripropylenglykoldiacrylat gemischt und über einen 50 µm Filter filtriert und abgefüllt.

I. Polymere A: Herstellung und Charakterisierung

Polymer 10:

| | | |
|---|---|---|
| Zulauf 1 | 109,50 g | Methylmethacrylat |
| | 22,50 g | Methacrylsäure |
| | 15,00 g | Acrylsäure |
| | 3,00 g | Laromer® UA 19 T |
| | 100,00 g | Ethanol kosm. |
| Zulauf 2 | 2,25 g | Wako®V 59 |
| | 50,00 g | Ethanol kosm. |

Als Vorlage wurden in einem 1-l-Glasreaktor 37,50 g von Zulauf 1 und 13,13 g von Zulauf 2 mit 150,00 g kosmetischen Ethanol gemischt. Diese Vorlage wurde unter Stickstoffatmosphäre zum Rückfluss erhitzt. Nach Erreichen der Rückflusstemperatur wurden die Zuläufe 1 und 2 gemeinsam gestartet. Zulauf 1 wurde in 3 Stunden unter Rückfluss und Zulauf 2 in 4 Stunden unter Rückfluss zudosiert. Die Reaktionsmischung wurde 1 Stunde unter Rückfluss weiter polymerisiert. Anschließend wurde Zulauf 3 (0,68g Wako®V 59 und 50,00g Ethanol kosm.) in 30 Minuten zudosiert und 3 Stunden unter Rückfluss nachpolymerisiert.

Polymer 12:

| | | | |
|---|---|---|---|
| Zulauf 1 | 117,00g | Methylmethacrylat |
| | 15,00g | Methacrylsäure |
| | 15,00g | Acrylsäure |
| | 3,00g | Laromer®UA 19 T |
| | 100,00g | Ethanol kosm. |
| Zulauf 2 | 3,00g | Wako®V 50 |
| | 50,00g | VE-Wasser |

Als Vorlage wurden in einem 1-I-Glasreaktor 37,50 g von Zulauf 1 und 13,1g von Zulauf 2 mit 100,00 g kosmetischem Ethanol gemischt. Diese Vorlage wurde unter Stickstoffatmosphäre zum Rückfluss erhitzt. Nach Erreichen der Rückflusstemperatur wurden die Zuläufe 1 und 2 gemeinsam gestartet. Zulauf 1 wurde in 3 Stunden und Zulauf 2 in 3,5 Stunden zudosiert. Die Reaktionsmischung wurde 1 Stunde unter Rückfluss weiter polymerisiert. Danach wurde Zulauf 3 (0.60g Wako® V 50 und 25.00g VE-Wasser) in 30 Minuten zudosiert und 1,5 Stunden unter Rückfluss nachpolymerisiert. Anschließend wurde Zulauf 4 (0,60g Wako®V-50 und 25,00g VE-Wasser) in 30 Minuten zudosiert und nochmals 1,5 Stunden unter Rückfluss nachpolymerisiert

Polymer 13:

| | | |
|---|---|---|
| Zulauf1 | 109,50g | Methylmethacrylat |
| | 30,00g | Methacrylsäure |
| | 7,50g | Acrylsäure |
| | 3,00g | Laromer®UA 19 T |
| | 122,50g | Ethanol kosm. |
| Zulauf 2 | 3,75g | Wako® V 50 |
| | 18,00g | VE-Wasser |

Als Vorlage wurden in einem 1-Lter Glasreaktor 13,6 g von Zulauf 1 und 1,1 g von Zulauf 2 mit 39,90 g kosmetischen Ethanol und 82,10 g VE-Wasser gemischt. Diese Vorlage wurde unter Stickstoffatmosphäre auf 80°C erhitzt. Nach Erreichen der Rückflusstemperatur wurden die Zuläufe 1 und 2 gemeinsam gestartet. Zulauf 1 wurde in 3 Stunden unter Rückfluss und Zulauf 2 in 4 Stunden unter Rückfluss zudosiert. Die Reaktionsmischung wurde 2 Stunden unter Rückfluss weiter polymerisiert. Danach wurde Zulauf 3 (0,75 g tert.-Butylperpivalat und 43,75 g Ethanol kosm.) in 30 Minuten zudosiert und 2 Stunden unter Rückfluss nachpolymerisiert. Anschließend wurde Zulauf 4 (0,75 g tert.-Butylperpivalat und 43,75 g Ethanol kosm) in 30 Minuten zudosiert und nochmals 2 Stunden unter Rückfluss nachpolymerisiert.

Polymer 15:

| | | |
|---|---|---|
| Zulauf 1 | 109,50g | Methylmethacrylat |
| | 30,00g | Methacrylsäure |
| | 7,50g | Acrylsäure |
| | 100,00g | Ethanol kosm. |
| Zulauf 2 | 3,00g | Laromer® UA 19 T |
| | 22,50g | Ethanol kosm. |

(fortgesetzt)

| | | |
|---|---|---|
| Zulauf 3 | 3,75g | Wako®V-50 |
| | 18,00g | VE-Wasser |

Als Vorlage wurden in einem 1-I-Glasreaktor 12,3 g von Zulauf 1 und 1,1 g von Zulauf 3 mit 39,90 g kosmetischen Ethanol und 82,10g VE-Wasser gemischt. Diese Vorlage wurde unter Stickstoffatmosphäre zum Rückfluss erhitzt. Nach Erreichen der Rückflusstemperatur wurden die Zuläufe 1, 2 und 3 gemeinsam gestartet. Die Zuläufe 1 und 2 wurden in 3 Stunden unter Rückfluss und Zulauf 3 in 4 Stunden unter Rückfluss zudosiert. Die Reaktionsmischung wurde 2 Stunden unter Rückfluss weiter polymerisiert. Danach wurde Zulauf 4 (0,75 g tert.-Butylperpivalat und 43,75 g Ethanol kosm.) in 30 Minuten zudosiert und 2 Stunden unter Rückfluss nachpolymerisiert. Anschließend wurde Zulauf 5 (0',75 g tert.-Butylperpivalat und 43,75 g Ethanol kosm.) in 30 Minuten zudosiert und nochmals 2 Stunden unter Rückfluss nachpolymerisiert.

Polymer 16:

| | | |
|---|---|---|
| Zulauf 1 | 234,0g | Methylmethacrylat |
| | 30,00g | Methacrylsäure |
| | 30,00g | Acrylsäure |
| | 100,00g | Ethanol kosm. |
| | 6,00g | Urethanacrylat c26 |
| Zulauf 2 | 6,0g | Wako®V-59 |
| | 412,50g | Ethanol kosm. |
| | 68,40g | VE-Wasser |

Als Vorlage wurden in einem 2-I-Glasreaktor 15,0 g von Zulauf 1 und 24,3g von Zulauf 2 mit 171,50 g kosmetischen Ethanol und 28,60g VE-Wasser gemischt. Diese Vorlage wurde unter Stickstoffatmosphäre zum Rückfluss erhitzt. Nach Erreichen der Rückflusstemperatur wurden die Zuläufe 1 und 2 gemeinsam gestartet. Zulauf 1 wurde in 3 Stunden unter Rückfluss und Zulauf 2 in 4 Stunden unter Rückfluss zudosiert. Die Reaktionsmischung wurde 2 Stunden unter Rückfluss weiter polymerisiert. Danach wurde Zulauf 3 (1,50 g tert.-Butylperpivalat, 3,0 g VE-Wasser und 17,0 g Ethanol kosm.) in 30 Minuten zudosiert und 2 Stunden unter Rückfluss nachpolymerisiert. Anschließend wurde Zulauf 4 (1,50 g tert.-Butylperpivalat, 3,0 g VE-Wasser und 17,0 g Ethanol kosm.) in 30 Minuten zudosiert und nochmals 2 Stunden unter Rückfluss nachpolymerisiert.

Polymer 17:

| | | |
|---|---|---|
| Zulauf 1 | 234,0g | Methylmethacrylat |
| | 30,00g | Methacrylsäure |
| | 30,00g | Acrylsäure |
| | 6,00g | Urethanacrylat c26 |
| Zulauf 2 | 6,0g | Wako®V-59 |
| | 412,50g | Ethanol kosm. |

Als Vorlage wurden in einem 2-I-Glasreaktor 15,0 g von Zulauf 1 und 21,0 g von Zulauf 2 mit 171,50 g kosmetischen Ethanol und 103,0 g VE-Wasser gemischt. Diese Vorlage wurde unter Stickstoffatmosphäre zum Rückfluss erhitzt. Nach Erreichen der Rückflusstemperatur wurden die Zuläufe 1 und 2 gemeinsam gestartet. Zulauf 1 wurde in 3 Stunden unter Rückfluss und Zulauf 2 in 4 Stunden unter Rückfluss zudosiert. Die Reaktionsmischung wurde 2 Stunden unter Rückfluss weiter polymerisiert. Danach wurde Zulauf 3 (1,50 g tert.-Butylperpivalat und 17,0 g Ethanol kosm.) in

30 Minuten zudosiert und 2 Stunden unter Rückfluss nachpolymerisiert. Anschließend wurde Zulauf 4 (1,50 g tert.-Butylperpivalat und 17,0 g Ethanol kosm.) in 30 Minuten zudosiert und nochmals 2 Stunden unter Rückfluss nachpolymerisiert.

Die Prozentangaben bedeuten Gew.-%, sofern nicht anderweitig bezeichnet. Die Herstellung der Polymere 1-9 und 11 in der folgenden Tabelle erfolgte analog der zuvor beschriebenen Herstellung von Polymer 10. Die Herstellung von Polymere 14, 18, 19 und 20 erfolgte analog der beschriebenen Herstellung von Polymer15.

| Polymer | (Meth)Acrylat Gew.-% | MAA Gew.-% | AA Gew.-% | Komponente c) Gew.-% | K-Wert [1% in NMP] |
|---|---|---|---|---|---|
| 1 | MMA 75 | 10 | 10 | Ebecryl®CL 1039 5 | 31,2 |
| 2 | EMA 75 | 10 | 10 | Ebecryl®CL 1039, 5 | 30,3 |
| 3 | MMA 75 | 10 | 10 | Monomer C22 5 | 31,7 |
| 4 | EMA 75 | 10 | 10 | Monomer C22 5 | 31,6 |
| 5 | MMA 78 | 10 | 10 | Laromer®UA 19T 2 | 36,4 |
| 6 | EMA 78 | 10 | 10 | Laromer®UA 19T 2 | 34,5 |
| *7 | MMA 70 | - | 25 | Monomer C22 5 | 35,8 |
| 8 | EMA 70 | 15 | 10 | Monomer C22 5 | 31,7 |
| 9 | MMA 72 | 15 | 10 | Ebecryl®CL 1039 3 | 31,6 |
| 10 | MMA 73 | 15 | 10 | Laromer®UA 19T 2 | 37,1 |
| 11 | MMA 74 | 15 | 10 | Plex 6661-0® 1 | 35,8 |
| 12 | MMA 78 | 10 | 10 | Laromer®UA 19T 2 | 34,1 |
| 13 | MMA 73 | 20 | 5 | Laromer®UA 19T 2 | 30,5 |
| 14 | MMA 78 | 10 | 10 | Laromer®UA 19T 2 | 32,2 |
| 15 | MMA 73 | 20 | 5 | Laromer®UA 19T 2 | 31,6 |
| 16 | MMA 78 | 10 | 10 | Urethanacrylat c26 2 | 33,2 |
| 17 | MMA 78 | 10 | 10 | Urethanacrylat c26 2 | 34,0 |
| 18 | MMA 73 | 20 | 5 | Photomer® 6891, 2 | 32,0 |
| 19 | MMA 73 | 20 | 5 | Sartomer® CN981, 2 | 30,5 |
| 20 | MMA 74 | 20 | 5 | Ebecryl® 265, 1 | - |
| * Vergleichspolymer | | | | | |

Anwendungstechnische Eigenschaften der erfindungsgemäßen Polymere

| Polymer | Aussehen Aerosol | Festigung, relativ zu Amphomer® LV 71 [%] | Teilchengröße beim Versprühen eines VOC 55 Aerosols [mm] a) | Curl Retention [%] | Auswaschbarkeit | Klebrigkeit (Kempf) |
|---|---|---|---|---|---|---|
| 1 | klar | 88 | 39 | 64 | gut | 0 |
| 2 | klar | 89 | 50 | 52 | gut | 0 |
| 5 | klar | 106 | 41 | 60 | gut | 0 |
| 6 | klar | 108 | 53 | 62 | gut | 0 |
| *7 | klar | 97 | 37 | 35 | noch gut | 2 |
| 8 | klar | 83 | 51 | 66 | noch gut | 0-1 |
| 9 | klar | 115 | 50 | 78 | gut | 0-1 |

(fortgesetzt)

| Polymer | Aussehen Aerosol | Festigung, relativ zu Amphomer® LV 71 [%] | Teilchengröße beim Versprühen eines VOC 55 Aerosols [mm] a) | Curl Retention [%] | Auswaschbarkeit | Klebrigkeit (Kempf) |
|---|---|---|---|---|---|---|
| 10 | leicht trüb | 105 | 42 | 57 | gut | 0 |
| 11 | klar | 115 | 39 | 65 | noch gut | 0 |
| 12 | klar | 125 | 39 | 74 | sehr gut | 0 |
| 13 | klar | 116 | 35 | 74 | gut | 1 |
| 14 | fast klar | 105 | 34 | 79 | gut | 0 |
| 15 | fast klar | 109 | 35 | 71 | gut | 0 |
| 16 | fast klar | 104 | 36 | 89 | gut | 0 |
| 17 | fast klar | 108 | 37 | 92 | gut | 0 |
| 18 | blaustichig | 83 | 32 | 75 | noch gut | 0-1 |
| 19 | blaustichig | 75 | 34 | 62 | noch gut | 0 |
| 20 | fast klar | 84 | 33 | 69 | noch gut | 0 |
| | | | | | | |

* Zum Vergleich
a) VOC55 Aerosol:
5% des jeweiligen Polymers, vollständig neutralisiert mit AMP,
40% DME,
15% Ethanol,
40% Wasser;
Sprühvorrichtung:
Sprühkopf: Kosmos .020D Wirbel .018" 21-6443-20,
Ventil: DPV 33876 (Fa. Precision Valve)

II. Anwendungstechnische Beispiele

Falls nicht anders angegeben, werden alle eingesetzten Säuregruppen haltigen Polymere zu 100% neutralisiert mit AMP eingesetzt. "Wasser ad 100" bedeutet, dass zu der jeweiligen Zubereitung die zum Erreichen einer Gesamtmenge von 100 Gew.-% notwendige Restmenge an Wasser zugegeben wird.

Die Mengenangaben % sind Gew.-% sofern nicht anderweitig bestimmt.

Die Abkürzung "q.s." bedeutet "quatum satis", d.h. so viel eines Inhaltsstoffes zugeben, wie notwendig ist, um einen gewünschten Effekt zu erreichen.

Beispiel 1:

VOC 55-Aerosol-Haarspray

| | [%] |
|---|---|
| Polymer aus Beispiel Nr. 1 (fest) | 5,00 |
| Dimethylether | 40,00 |
| Ethanol | 15,00 |
| Wasser | ad 100 |

weitere Zusätze: Silikon, Parfüm, Entschäumer, UV-Absorber

Das Beispiel lässt sich jeweils mit den erfindungsgemäßen Polymeren 2 bis 20 wiederholen. Es wird jeweils ein VOC 55-Aerosol-Haarspray mit guten Eigenschaften erhalten.

Beispiel 2:

VOC 55-Aerosol-Haarspray

| | [%] |
|---|---|
| Polymer aus Beispiel Nr. 1 (fest) | 5,00 |
| Dimethylether | 35,00 |
| Propan/Butan | 5,00 |
| Ethanol | 15,00 |
| Wasser | ad 100 |

weitere Zusätze: Silikon, Parfüm, Entschäumer, UV-Absorber

Das Beispiel lässt sich jeweils mit den Polymeren 2 bis 20 wiederholen. Es wird jeweils ein VOC 55-Aerosol-Haarspray mit guten Eigenschaften erhalten.

Beispiel 3:

Aerosol-Haarspray mit Fluorcarbon-Treibmitteln

| | [%] |
|---|---|
| Polymer aus Beispiel Nr. 1 (fest) | 5,00 |
| Ethanol abs. | ad 100 |
| HFC 152A | 40,00 |

weitere Zusätze: Silikon, Parfüm, Entschäumer, UV-Absorber

Das Beispiel lässt sich jeweils mit den Polymeren 2 bis 20 wiederholen. Es wird jeweils ein LowVOC Aerosol-Haarspray mit guten Eigenschaften erhalten.

Beispiel 4:

Aerosol-Haarspray mit Fluorcarbon-Treibmitteln

| | [%] |
|---|---|
| Polymer aus Beispiel Nr. 1 (fest) | 5,00 |
| Dest. Wasser | ad 100 |
| HFC 152A | 10,00 |
| Dimethylether | 30,00 |
| Ethanol abs. | 30,00 |

weitere Zusätze: Silikon, Parfüm, Entschäumer, UV-Absorber

Das Beispiel lässt sich jeweils mit den Polymeren 2 bis 20 wiederholen. Es wird jeweils ein LowVOC Aerosol-Haarspray mit guten Eigenschaften erhalten.

Beispiel 5:

VOC 55-Aerosol-Haarspray

| | [%] |
|---|---|
| Polymer aus Beispiel Nr. 1 (fest) | 3,00 |
| Ultrahold® Strong (fest, Fa. BASF) | 1,00 |
| Dimethylether | 40,00 |
| Ethanol | 15,00 |
| + AMP | auf pH 8,3 |
| Wasser | ad 100 |

weiterer Zusatz: Silikon, Parfüm, Entschäumer

Das Beispiel lässt sich jeweils mit den erfindungsgemäßen Polymeren 2 bis 20 wiederholen. Es wird jeweils ein VOC 55-Aerosol-Haarspray mit guten Eigenschaften erhalten.

Beispiel 6:

VOC 55-Aerosol-Haarspray

| | [%] |
|---|---|
| Polymer aus Beispiel Nr. 1 (fest) | 3,00 |
| Luvimer® Pro55 (fest, Fa. BASF) | 1,00 |
| Dimethylether | 40,00 |
| Ethanol | 15,00 |
| Wasser | ad 100 |

weiterer Zusatz: Silikon, Parfüm, Entschäumer

Das Beispiel lässt sich jeweils mit den erfindungsgemäßen Polymeren 2 bis 20 wiederholen. Es wird jeweils ein VOC 55-Aerosol-Haarspray mit guten Eigenschaften erhalten.

Beispiel 7:

VOC 55-Aerosol-Haarspray

| | [%] |
|---|---|
| Polymer aus Beispiel Nr. 1 (fest) | 3,00 |
| Luvimer® P.U.R (fest, Fa. BASF) | 1,00 |
| Dimethylether | 40,00 |
| Ethanol | 15,00 |
| Wasser | ad 100 |

weiterer Zusatz: Silikon, Parfüm, Entschäumer

Das Beispiel lässt sich jeweils mit den erfindungsgemäßen Polymeren 2 bis 20 wiederholen. Es wird jeweils ein VOC 55-Aerosol-Haarspray mit guten Eigenschaften erhalten.

Beispiel 8:

VOC 55-Aerosol-Haarspray

| | [%] |
|---|---|
| Polymer aus Beispiel Nr. 1 (fest) | 3,00 |
| Resyn® 28-2930 (fest, Fa. National Starch) | 1,00 |
| Dimethylether | 40,00 |
| Ethanol | 15,00 |
| Wasser | ad 100 |

weiterer Zusatz: Silikon, Parfüm, Entschäumer

Das Beispiel lässt sich jeweils mit den erfindungsgemäßen Polymeren 2 bis 20 wiederholen. Es wird ebenfalls ein VOC 55-Aerosol-Haarspray mit guten Eigenschaften erhalten.

Beispiel 9:

VOC 55-Aerosol-Haarspray

| | [%] |
|---|---|
| Polymer aus Beispiel Nr. 1 (fest) | 2,00 |
| Stepanhold®R-1 *) (Fa. Stepan Chemical Co.) | 1,00 |
| Dimethylether | 40,00 |
| Ethanol | 15,00 |
| + AMP | auf pH 8,3 |
| Wasser | ad 100 |

*) Stepanhold®R-1 = Poly(Vinylpyrrolidon/Ethylmethacrylat/ Methacrylsäure)

weiterer Zusatz: Silikon, Parfüm, Entschäumer

Das Beispiel lässt sich jeweils mit den erfindungsgemäßen Polymeren 2 bis 20 wiederholen. Es wird jeweils ein VOC 55-Aerosol-Haarspray mit guten Eigenschaften erhalten.

Beispiel 10:

VOC 55-Handpumpenspray

| | [%] |
|---|---|
| Polymer aus Beispiel Nr. 1 (fest) | 7,00 |
| Ethanol | 55,00 |
| Wasser | ad 100 |

weiterer Zusatz: Silikon, Parfüm, Entschäumer

Das Beispiel lässt sich jeweils mit den erfindungsgemäßen Polymeren 2 bis 20 wiederholen. Es wird jeweils ein VOC 55-Handpumpenspray mit guten Eigenschaften erhalten.

Beispiel 11:

VOC 80-Aerosol-Haarspray

| | [%] |
|---|---|
| Polymer aus Beispiel Nr. 1 (fest) | 12,00 |
| Dimethylether | 40,00 |
| Ethanol | 40,00 |
| Wasser | ad 100 |

weiterer Zusatz: Silikon, Parfüm, Entschäumer

Das Beispiel lässt sich jeweils mit den erfindungsgemäßen Polymeren 2 bis 20 wiederholen. Es wird jeweils ein VOC 80-Aerosol-Haarspray mit guten Eigenschaften erhalten.

Beispiel 11:

| Wässriges Handpumpenspray | [%] |
|---|---|
| Polymer aus Beispiel Nr. 1 (fest) | 4,00 |
| Luviset®Clear *) (fest) | 1,00 |
| Wasser | ad 100 |

*) Luviset®Clear: Poly(Vinylpyrrolidon/ Methacrylsäureamid/Vinylimidazol), Fa. BASF

weiterer Zusatz: wasserlösliches Silikon, Parfüm, Entschäumer.

Das Beispiel lässt sich jeweils mit den erfindungsgemäßen Polymeren 2 bis 20 wiederholen. Es wird jeweils ein wässriges Handpumpenspray mit guten Eigenschaften erhalten.

Beispiel 12:

| Wässrig/ethanolische Festiger-Lösung | [%] |
|---|---|
| Polymer aus Beispiel Nr. 1 (fest) | 7,00 |
| dest. Wasser | ad 100 |
| Ethanol | 52,00 |

weiterer Zusatz: Silikon, Parfüm, Entschäumer

Das Beispiel lässt sich jeweils mit den erfindungsgemäßen Polymeren 2 bis 20 wiederholen. Es wird jeweils eine Festiger-Lösung mit guten Eigenschaften erhalten.

Beispiel 13:

| Ethanolische Festiger-Lösung | [%] |
|---|---|
| Polymer aus Beispiel Nr. 1 (fest) | 7,0 |
| Ethanol | ad 100 |

weiterer Zusatz: Silikon, Parfüm, Entschäumer ...

Das Beispiel lässt sich jeweils mit den erfindungsgemäßen Polymeren 2 bis 20 wiederholen. Es wird jeweils eine Festiger-Lösung mit guten Eigenschaften erhalten.

Beispiel 14:

| Haargel mit Aculyn 28: | [%] |
|---|---|
| Phase 1: | |
| Polymer aus Beispiel Nr. 1 (fest) | 6,00 |
| Aminomethylpropanol (38 %ige Lösung) | 1,0 |
| Wasser, dest. | ad 50 |

weiterer Zusatz: Konservierungsmittel, lösliches ethoxiliertes Silikon, Parfüm ...

| Phase 2: | |
|---|---|
| Aculyn 28 (1 %ige wässrige Suspension) | 50,00 |

Herstellung:

Die Phasen 1 und 2 werden getrennt eingewogen und homogenisiert. Dann wird Phase 2 langsam in Phase 1 eingerührt. Es bildet sich ein im Wesentlichen klares, stabiles Gel.
Das Beispiel lässt sich jeweils mit den erfindungsgemäßen Polymeren 2 bis 20 wiederholen. Es wird jeweils ein Haargel mit Aculyn 28 mit guten Eigenschaften erhalten.

Beispiel 15:

| Haargel mit Hydroxyethylcellulose: | [%] |
|---|---|
| Phase 1: | |
| Polymer aus Beispiel Nr. 1 (fest) | 6,00 |
| Wasser, dest. | ad 50 |

weiterer Zusatz: Konservierungsmittel, lösliches ethoxiliertes Silikon, Parfüm

| Phase 2: | |
|---|---|
| Natrosol HR 250 (5 %ige Lösung) | 50,00 |
| Hydroxyethylcellulose (Fa. Hercules) | |

Herstellung:

Die Phasen 1 und 2 werden getrennt eingewogen und homogenisiert. Dann wird Phase 2 langsam in Phase 1 eingerührt. Es bildet sich ein im Wesentlichen klares, stabiles Gel.
Das Beispiel lässt sich jeweils mit den erfindungsgemäßen Polymeren 2 bis 20 wiederholen. Es wird jeweils ein Haargel mit Hydroxyethylcellulose mit guten Eigenschaften erhalten.

Beispiel 16:

| Schaumconditioner | [%] |
|---|---|
| Polymer aus Beispiel Nr. 1 (fest) | 0,50 |
| Cremophor®A 25 (Ceteareth 25/Fa. BASF) | 0,20 |

(fortgesetzt)

| Schaumconditioner | [%] |
|---|---|
| Comperlan®KD (Coamide DEA/Fa. Henkel) | 0,10 |
| Propan/Butan | 10,00 |

weiterer Zusatz: Parfüm, Konservierungsmittel

| | |
|---|---|
| Wasser | ad 100 |

Herstellung: Einwiegen und unter Rühren lösen. Abfüllen und Treibgas zusetzen.

Das Beispiel lässt sich jeweils mit den erfindungsgemäßen Polymeren 2 bis 20 wiederholen. Es wird jeweils ein Schaumconditioner mit guten Eigenschaften erhalten.

Beispiel 17:

| Conditionershampoo: | [%] |
|---|---|
| A) Texapon®NSO 28 %ig (Natriumlaurethsulfat/Fa. Henkel) | 50,00 |
| Comperlan®KS (Coamid DEA/Fa. Henkel) | 1,00 |
| Polymer aus Beispiel Nr. 1 (fest) | 3,00 |
| q. s. Parfümöl | |
| | |
| B) Wasser | 44,5 |
| Natriumchlorid | 1,5 |
| q. s. Konservierungsmittel | |

Herstellung:

Die Phasen 1 und 2 werden getrennt eingewogen und homogenisiert. Dann wird Phase 2 langsam in Phase 1 eingerührt. Es bildet sich ein im Wesentlichen klares, stabiles Gel.

Das Beispiel lässt sich jeweils mit den erfindungsgemäßen Polymeren 2 bis 20 wiederholen. Es wird jeweils ein Conditionershampoo mit guten Eigenschaften erhalten.

Beispiel 18:

Standard O/W-Creme:

| Ölphase: | [%] | CTFA-Name | |
|---|---|---|---|
| Cremophor®A6 | 3,5 | Ceteareth-6 (and) Stearyl Alcohol | |
| Cremophor®A25 | 3,5 | Ceteareth-25 | |
| Glycerinmonostearat s.e. | 2,5 | Glycerylstearat | |
| Paraffinöl | | 7,5 | Paraffin Oil |
| Cetylalkohol | | 2,5 | Cetyl Alcohol |
| Luvitol®EHO | | 3,2 | Cetearyl Octanoate |
| Vitamin-E-Acetat | 1,0 | Tocopheryl Acetate | |
| Nip-Nip | | 0,1 | Methyl- and Propyl-4-hydroxybenzoate (7:3) |

Wasserphase:

Polymer aus Beispiel Nr. 1

| | | |
|---|---|---|
| (fest) | | 0,6 |
| Wasser | | 77,0 |
| 1,2-Propylenglykol | 1,5 | Propylenglykol |
| Germall II | 0,1 | Imidazolidinyl-Harnstoff |

Herstellung:

Die Öl- und Wasserphasen werden getrennt eingewogen und bei einer Temperatur von ca. 80°C homogenisiert. Dann wird die Wasserphase langsam in die Ölphase eingerührt und unter Rühren langsam auf Raumtemperatur abgekühlt.

Das Beispiel lässt sich jeweils mit den erfindungsgemäßen Polymeren 2 bis 20 wiederholen. Es wird jeweils eine Standard O/W-Creme mit guten Eigenschaften erhalten.

Beispiel 19: Flüssiges Makeup

| | |
|---|---|
| A | |
| 1,70 | Glycerylstearat |
| 1,70 | Cetylalkohol |
| 1,70 | Ceteareth-6 |
| 1,70 | Ceteareth-25 |
| 5,20 | Capryl/Caprin-Triglycerid |
| 5,20 | Mineralöl |
| B | |
| q.s. | Konservierungsmittel |
| 4,30 | Propylenglykol |
| 2,50 | Polymer aus Beispiel 1 (fest) |
| ad 100 | dest. Wasser |
| C | |
| q.s. | Parfumöl |
| D | |
| 2,00 | Eisenoxid |
| 12,00 | Titandioxid |

Herstellung:

Phase A und Phase B getrennt voneinander auf 80°C erwärmen. Dann Phase B in Phase A mit einem Rührer mischen. Alles auf 40°C abkühlen lassen und Phase C und Phase D zugeben. Wiederholt homogenisieren.

Das Beispiel lässt sich jeweils mit den erfindungsgemäßen Polymeren.2 bis 20 wiederholen. Es wird jeweils ein flüssiges Makeup mit guten Eigenschaften erhalten.

Beispiel 20: Ölfreies Makeup

| | |
|---|---|
| A | |
| 0,35 | Veegum |
| 5,00 | Butylenglykol |
| 0,15 | Xanthangummi |
| B | |
| 34,0 | dest. Wasser |
| q.s. | Konservierungsmittel |
| 0,2 | Polysorbate-20 |
| 1,6 | Tetrahydroxypropylethylenediamin |
| C | |
| 1,0 | Siliciumdioxid |
| 2,0 | Nylon-12 |
| 4,15 | Glimmer (mica) |
| 6,0 | Titandioxid |
| 1,85 | Eisenoxid |
| D | |
| 4,0 | Stearinsäure |
| 1,5 | Glycerylstearat |
| 7,0 | Benzyllaurat |
| 5,0 | Isoeicosan |
| q.s. | Konservierungsmittel |
| E | |
| 0,5 | Panthenol |
| 0,1 | Imidazolidinyl-Harnstoff |
| 5,0 | Polymer aus Beispiel 1 (fest) |

Herstellung:

Phase A mit Butylenglykol benetzen, in Phase B hineingeben und gut mischen. Phase AB auf 75°C erwärmen. Phase C Einsatzstoffe pulverisieren, in Phase AB hineingeben und gut homogenisieren. Einsatzstoffe von Phase D mischen, auf 80°C erwärmen und zu Phase ABC geben. Einige Zeit mischen, bis alles homogen ist. Alles in ein Gefäß mit Propellermischer übertragen. Einsatzstoffe von Phase E mischen, in Phase ABCD hineingeben und gut vermischen.

Das Beispiel lässt sich jeweils mit den erfindungsgemäßen Polymeren 2 bis 20 wiederholen. Es wird jeweils ein ölfreies Makeup mit guten Eigenschaften erhalten.

Beispiel 21: Schimmerndes Gel

| | |
|---|---|
| A | |
| 32,6 | dest. Wasser |
| 0,1 | Dinatrium-EDTA |
| 25,0 | Natrosol (4 %ige wässrige Lösung) |
| 0,3 | Konservierungsmittel |
| B | |
| 0,5 | dest. Wasser |

(fortgesetzt)

| | |
|---|---|
| B | |
| 0,5 | Triethanolamin |
| C | |
| 2,0 | Polymer aus Beispiel 1 (fest) |
| ad 100 | dest. Wasser |
| 1,0 | Polyquaternium-46 (20 %ige wässrige Lösung) |
| 5,0 | Eisenoxid |
| D | |
| 15,0 | dest. Wasser |
| 1,0 | D-Panthenol 50 P (Panthenol und Propylenglykol) |

Herstellung:

Mit einem Propellermischer die Einsatzstoffe von Phase A in der angegebenen Reihenfolge gut mischen. Dann Phase B in Phase A hineingeben. Langsam rühren bis alles homogen ist. Phase C gut homogenisieren, bis die Pigmente gut verteilt sind. Phase C und Phase D zu Phase AB geben und gut mischen.

Das Beispiel lässt sich jeweils mit den erfindungsgemäßen Polymeren 2 bis 20 wiederholen. Es wird jeweils ein schimmerndes Gel mit guten Eigenschaften erhalten.

Beispiel 22: Sonnenschutz-Gel

| | |
|---|---|
| Phase A | |
| 1,00 | hydriertes Ricinusöl-PEG-40 |
| 8,00 | Octyl Methoxycinnamate (Uvinul®MC 80) |
| 5,00 | Octocrylene (Uvinul®N 539) |
| 0,80 | Octyl Triazone (Uvinul®T 150) |
| 2,00 | Butyl Methoxydibenzoylmethane (Uvinul®BMBM) |
| 2,00 | Tocopherylacetat |
| q.s. | Parfümöl |
| Phase B | |
| 2,50 | Polymer aus Beispiel 1 (fest) |
| ad 100 | dest. Wasser |
| 0,30 | Acrylat/$C_{10-30}$ Alkylacrylat-Copolymer |
| 0,20 | Carbomer |
| 5,00 | Glycerin |
| 0,20 | Dinatrium-EDTA |
| q.s. | Konservierungsmittel |
| 62,80 | dest. Wasser |
| Phase C | |
| 0,20 | Natriumhydroxid |

Herstellung:

Die Komponenten der Phase A mischen. Phase B quellen lassen und unter Homogenisieren in Phase A einrühren. Mit Phase C neutralisieren und erneut homogenisieren.

Das Beispiel lässt sich jeweils mit den erfindungsgemäßen Polymeren 2 bis 20 wiederholen. Es wird jeweils

ein Sonnenschutz-Gel mit guten Eigenschaften erhalten.

Beispiel 23: Sonnenschutzemulsion mit $TiO_2$ und $ZnO_2$

| | |
|---|---|
| Phase A | |
| 6,00 | hydriertes Ricinusöl-PEG-7 |
| 2,00 | PEG-45/Dodecyl Glycol Copolymer |
| 3,00 | Isopropylmyristat |
| 8,00 | Jojobaöl (Buxus Chinensis) |
| 4,00 | Octyl Methoxycinnamate (Uvinul®MC 80) |
| 2,00 | 4-Methylbenzylidene Camphor (Uvinul®MBC 95) |
| 3,00 | Titandioxid, Dimethicon |
| 1,00 | Dimethicon |
| 5,00 | Zinkoxid, Dimethicon |
| Phase B | |
| 2,0 | Polymer aus Beispiel 1 (fest) |
| ad 100 | dest. Wasser |
| 0,20 | Dinatrium-EDTA |
| 5,00 | Glycerin |
| q.s. | Konservierungsmittel |
| 50,80 | dest. Wasser |
| Phase C | |
| q.s. | Parfümöl |

Herstellung:

Die Phasen A und B getrennt auf ca. 85°C erwärmen. Phase B in Phase A einrühren und homogenisieren. Abkühlen auf ca. 40°C, Phase C hinzugeben und nochmals kurz homogenisieren.

Das Beispiel lässt sich jeweils mit den erfindungsgemäßen Polymeren 2 bis 20 wiederholen. Es wird jeweils eine Sonnenschutzemulsion mit $TiO_2$ und $ZnO_2$ mit guten Eigenschaften erhalten.

Beispiel 24: Sonnenschutz-Lotion

| | |
|---|---|
| Phase A | |
| 6,00 | Octyl Methoxycinnamate (Uvinul®MC 80) |
| 2,50 | 4-Methylbenzylidene Camphor (Uvinul®MBC 95) |
| 1,00 | Octyl Triazone (Uvinul®T 150) |
| 2,00 | Butyl Methoxydibenzoylmethane (Uvinul®BMBM) |
| 2,00 | PVP/Hexadecen-Copolymer |
| 5,00 | PPG-3 Myristyl Ether |
| 0,50 | Dimethicon |
| 0,10 | BHT, Ascorbylpalmitat, Citronensäure, Glycerylstearat Propylenglykol |
| 2,00 | Cetylalkohol |
| 2,00 | Kaliumcetylphosphat |
| Phase B | |
| 0,50 | Polymer aus Beispiel 1 (fest) |

(fortgesetzt)

| | |
|---|---|
| Phase B | |
| ad 100 | dest. Wasser |
| 5,00 | Propylenglykol |
| 0,20 | Dinatrium-EDTA |
| q.s. | Konservierungsmittel |
| 63,92 | dest. Wasser |
| Phase C | |
| 5,00 | Mineralöl |
| 0,20 | Carbomer |
| Phase D | |
| 0,08 | Natriumhydroxid |
| Phase E | |
| q.s. | Parfümöl |

Herstellung:

Die Phasen A und B getrennt auf ca. 80°C erwärmen. Phase B unter Homogenisieren in Phase A einrühren, kurz nachhomogenisieren. Phase C anschlämmen, in Phase AB einrühren, mit Phase D neutralisieren und nachhomogenisieren. Abkühlen auf ca. 40°C, Phase E zugeben, nochmals homogenisieren.

Das Beispiel lässt sich jeweils mit den erfindungsgemäßen Polymeren 2 bis 20 wiederholen. Es wird jeweils eine Sonnenschutz-Lotion mit guten Eigenschaften erhalten.

Beispiel 25: Abziehbare Gesichtsmaske

| | |
|---|---|
| Phase A | |
| 57,10 | dest. Wasser |
| 6,00 | Polyvinylalkohol |
| 5,00 | Propylenglykol |
| Phase B | |
| 20,00 | Alkohol |
| 4,00 | PEG-32 |
| q.s | Parfümöl |
| Phase C | |
| 5,00 | Polyquaternium-44 |
| 0,50 | Polymer aus Beispiel 1 (fest) |
| ad 100 | dest. Wasser |
| 0,20 | Allantoin |

Herstellung:

Phase A auf mind. 90°C erwärmen und rühren bis gelöst. Phase B bei 50°C lösen und in Phase A einrühren. Bei ca. 35°C den Ethanolverlust ausgleichen. Phase C zugeben und unterrühren.

Das Beispiel lässt sich jeweils mit den erfindungsgemäßen Polymeren 2 bis 20 wiederholen. Es wird jeweils eine abziehbare Gesichtsmaske mit guten Eigenschaften erhalten.

Beispiel 26: Gesichtsmaske

| | |
|---|---|
| Phase A | |
| 3,00 | Ceteareth-6 |
| 1,50 | Ceteareth-25 |
| 5,00 | Cetearylalkohol |
| 6,00 | Cetearyloctanoat |
| 6,00 | Mineralöl |
| 0,20 | Bisabolol |
| 3,00 | Glycerylstearat |
| Phase B | |
| 2,00 | Propylenglykol |
| 5,00 | Panthenol |
| 2,50 | Polymer aus Beispiel 1 (fest) |
| ad 100 | dest. Wasser |
| q.s. | Konservierungsmittel |
| 53,80 | dest. Wasser |
| Phase C | |
| q.s. | Parfümöl |
| 0,50 | Tocopherylacetat |

Herstellung:

Phase A und B getrennt auf ca. 80°C erwärmen. Phase B in Phase A unter Homogenisieren einrühren, kurz nachhomogenisieren. Abkühlen auf ca. 40°C, Phase C hinzugeben, nochmals homogenisieren.

Das Beispiel lässt sich jeweils mit den erfindungsgemäßen Polymeren 2 bis 20 wiederholen. Es wird jeweils eine Gesichtsmaske mit guten Eigenschaften erhalten.

Beispiel 27: Körperlotion-Schaum

| | |
|---|---|
| Phase A | |
| 1,50 | Ceteareth-25 |
| 1,50 | Ceteareth-6 |
| 4,00 | Cetearylalkohol |
| 10,00 | Cetearyloctanoat |
| 1,00 | Dimethicon |
| Phase B | |
| 0,50 | Polymer aus Beispiel 1 (fest) |
| ad 100 | dest. Wasser |
| 2,00 | Panthenol |
| 2,50 | Propylenglykol |
| q.s. | Konservierungsmittel |
| 74,50 | dest. Wasser |

(fortgesetzt)

| | |
|---|---|
| Phase C | |
| q.s. | Parfümöl |

Herstellung:

Die Phasen A und B getrennt auf ca. 80°C erwärmen. Phase B in Phase A einrühren und homogenisieren. Abkühlen auf ca. 40°C, Phase C hinzugeben und nochmals kurz homogenisieren. Abfüllung: 90 % Wirkstoff und 10 % Propan/Butan bei 3,5 bar (20°C).

Das Beispiel lässt sich jeweils mit den erfindungsgemäßen Polymeren 2 bis 20 wiederholen. Es wird jeweils ein Körperlotion-Schaum mit guten Eigenschaften erhalten.

Beispiel 28: Gesichtswasser für trockene und empfindliche Haut

| | |
|---|---|
| Phase A | |
| 2,50 | hydriertes Rizinusöl-PEG-40 |
| q.s. | Parfümöl |
| 0,40 | Bisabolol |
| | |
| Phase B | |
| 3,00 | Glycerin |
| 1,00 | Hydroxyethylcetyldimoniumphosphat |
| 5,00 | Zaubernuss (Hamamelis Virginiana) Destillat |
| 0,50 | Panthenol |
| 0,1 | Polymer aus Beispiel 1 (fest) |
| ad 100 | dest. Wasser |
| | |
| Phase C | |
| q.s. | Konservierungsmittel |
| 87,60 | dest. Wasser |

Herstellung:

Phase A klar lösen. Phase B in Phase A einrühren.

Das Beispiel lässt sich jeweils mit den erfindungsgemäßen Polymeren 2 bis 20 wiederholen. Es wird jeweils ein Gesichtswasser für trockene und empfindliche Haut mit guten Eigenschaften erhalten.

Beispiel 29: Gesichtswaschpaste mit Peelingeffekt

| | |
|---|---|
| Phase A | |
| 58,00 | dest. Wasser |
| 2,50 | Polymer aus Beispiel 1 (fest) |
| ad 100 | dest. Wasser |
| 1,50 | Carbomer |
| q.s. | Konservierungsmittel |
| | |
| Phase B | |
| q.s. | Parfümöl |

(fortgesetzt)

| | |
|---|---|
| Phase B | |
| 7,00 | Potassium Cocoyl Hydrolyzed Protein |
| 4,00 | Cocamidpropylbetain |
| Phase C | |
| 1,50 | Triethanolamin |
| Phase D | |
| 13,00 | Polyethylen (Luwax®A) |

Herstellung:

Phase A quellen lassen. Phase B klar lösen. Phase B in Phase A einrühren. Mit Phase C neutralisieren. Danach Phase D einrühren.

Das Beispiel lässt sich jeweils mit den erfindungsgemäßen Polymeren 2 bis 20 wiederholen. Es wird jeweils eine Gesichtswaschpaste mit Peelingeffekt mit guten Eigenschaften erhalten.

Beispiel 30: Gesichtsseife

| | |
|---|---|
| Phase A | |
| 25,0 | Kaliumcocoat |
| 20,0 | Disodium Cocoamphodiacetate |
| 2.0 | Lauramide DEA |
| 1,0 | Glykolstearat |
| 0,50 | Polymer aus Beispiel 1 (fest) |
| ad 100 | dest. Wasser |
| 50,0 | dest. Wasser |
| q.s. | Citronensäure |
| Phase B | |
| q.s. | Konservierungsmittel |
| q.s. | Parfumöl |

Herstellung:

Phase A unter Rühren auf 70°C erwärmen, bis alles homogen ist, pH-Wert auf 7,0-7,5 mit Zitronensäure einstellen, alles auf 50°C abkühlen lassen und Phase B zugeben.

Das Beispiel lässt sich jeweils mit den erfindungsgemäßen Polymeren 2 bis 20 wiederholen. Es wird jeweils eine Gesichtsseife mit guten Eigenschaften erhalten.

Beispiel 31: Gesichtsreinigungsmilch, Typ O/W

| | |
|---|---|
| Phase A | |
| 1,50 | Ceteareth-6 |
| 1,50 | Ceteareth-25 |
| 2,00 | Glycerylstearat |
| 2,00 | Cetylalkohol |
| 10,00 | Mineralöl |

(fortgesetzt)

| | |
|---|---|
| Phase A | |
| | |
| Phase B | |
| 5,00 | Propylenglykol |
| q.s. | Konservierungsmittel |
| 1,0 | Polymer aus Beispiel 1 (fest) |
| ad 100 | dest. Wasser |
| 62,30 | dest. Wasser |
| | |
| Phase C | |
| 0,20 | Carbomer |
| 10,00 | Cetearyloctanoat |
| | |
| Phase D | |
| 0,40 | Tetrahydroxypropylethylendiamin |
| | |
| Phase E | |
| q.s. | Parfümöl |
| 0,10 | Bisabolol |

Herstellung:

Die Phasen A und B getrennt auf ca. 80°C erwärmen. Phase B unter Homogenisieren in Phase A einrühren, kurz nachhomogenisieren. Phase C anschlämmen, in Phase AB einrühren, mit Phase D neutralisieren und nachhomogenisieren. Abkühlen auf ca. 40°C, Phase E zugeben, nochmals homogenisieren.

Das Beispiel lässt sich jeweils mit den erfindungsgemäßen Polymeren 2 bis 20 wiederholen. Es wird jeweils eine Gesichtsreinigungsmilch, Typ O/W mit guten Eigenschaften erhalten.

Beispiel 32: Peeling-Creme, Typ O/W

| | |
|---|---|
| Phase A | |
| 3,00 | Ceteareth-6 |
| 1,50 | Ceteareth-25 |
| 3,00 | Glycerylstearat |
| 5,00 | Cetearylalkohol, Sodium Cetearyl Sulfate |
| 6,00 | Cetearyloctanoat |
| 6,00 | Mineralöl |
| 0,20 | Bisabolol |
| | |
| Phase B | |
| 2,00 | Propylenglykol |
| 0,10 | Dinatrium-EDTA |
| 0,50 | Polymer aus Beispiel 1 (fest) |
| ad 100 | dest. Wasser |
| q.s. | Konservierungsmittel |
| 59,70 | dest. Wasser |

(fortgesetzt)

| | |
|---|---|
| Phase C | |
| 0,50 | Tocopherylacetat |
| q.s. | Parfümöl |
| Phase D | |
| 10,00 | Polyethylen |

Herstellung:

Die Phasen A und B getrennt auf ca. 80°C erwärmen. Phase B in Phase A einrühren und homogenisieren. Abkühlen auf ca. 40°C, Phase C hinzugeben und nochmals kurz homogenisieren. Anschließend Phase D unterrühren.

Das Beispiel lässt sich jeweils mit den erfindungsgemäßen Polymeren 2 bis 20 wiederholen. Es wird jeweils eine Peeling-Creme, Typ O/W mit guten Eigenschaften erhalten.

Beispiel 33: Rasierschaum

| | |
|---|---|
| 6,00 | Ceteareth-25 |
| 5,00 | Poloxamer 407 |
| 52,00 | dest. Wasser |
| 1,00 | Triethanolamin |
| 5,00 | Propylenglykol |
| 1,00 | Lanolinöl-PEG-75 |
| 1,0 | Polymer aus Beispiel 1 (fest) |
| ad 100 | dest. Wasser |
| q.s. | Konservierungsmittel |
| q.s. | Parfümöl |
| 25,00 | Sodium Laureth Sulfate |

Herstellung:

Alles zusammen wiegen, danach rühren bis gelöst. Abfüllung: 90 Teile Wirksubstanz und 10 Teile Propan/Butan-Mischung 25:75.

Das Beispiel lässt sich jeweils mit den erfindungsgemäßen Polymeren 2 bis 20 wiederholen. Es wird jeweils ein Rasierschaum mit guten Eigenschaften erhalten.

Beispiel 34: After Shave Balsam

| | |
|---|---|
| Phase A | |
| 0,25 | Acrylat/$C_{10-30}$ Alkylacrylat-Copolymer |
| 1,50 | Tocopherylacetat |
| 0,20 | Bisabolol |
| 10,00 | Capryl/Caprin-Triglycerid |
| q.s. | Parfümöl |
| 1,00 | hydriertes Rizinusöl-PEG-40 |
| Phase B | |
| 1,00 | Panthenol |

(fortgesetzt)

| | |
|---|---|
| Phase B | |
| 15,00 | Alkohol |
| 5,00 | Glycerin |
| 0,05 | Hydroxyethylcellulose |
| 0,50 | Polymer aus Beispiel 1 (fest) |
| ad 100 | dest. Wasser |
| 64,00 | dest. Wasser |
| Phase C | |
| 0,08 | Natriumhydroxid |

Herstellung:

Die Komponenten der Phase A mischen. Phase B unter Homogenisieren in Phase A einrühren, kurz nachhomogenisieren. Mit Phase C neutralisieren und erneut homogenisieren.

Das Beispiel lässt sich jeweils mit den erfindungsgemäßen Polymeren 2 bis 20 wiederholen. Es wird jeweils ein After Shave Balsam mit guten Eigenschaften erhalten.

Beispiel 35: Körperpflegecreme

| | |
|---|---|
| Phase A | |
| 2,00 | Ceteareth-6 |
| 2,00 | Ceteareth-25 |
| 2,00 | Cetearylalkohol |
| 3,00 | Glycerylstearat SE |
| 5,00 | Mineralöl |
| 4,00 | Jojobaöl (Buxus Chinensis) |
| 3,00 | Cetearyloctanoat |
| 1,00 | Dimethicon |
| 3,00 | Mineralöl, Lanolinalkohol |
| Phase B | |
| 5,00 | Propylenglykol |
| 0,50 | Veegum |
| 1,00 | Panthenol |
| 1,70 | Polymer aus Beispiel1 (fest) |
| ad 100 | dest. Wasser |
| 6,00 | Polyquaternium-44 (10 %ige wässrige Lösung) |
| q.s. | Konservierungsmittel |
| 54,00 | dest. Wasser |
| Phase C | |
| q.s. | Parfümöl |

Herstellung:

Die Phasen A und B getrennt auf ca. 80°C erwärmen. Phase B homogenisieren. Phase B unter Homogenisieren in Phase A einrühren, kurz nachhomogenisieren. Abkühlen auf ca. 40°C, Phase C hinzugeben und nochmals kurz homogenisieren.

Das Beispiel lässt sich jeweils mit den erfindungsgemäßen Polymeren 2 bis 20 wiederholen. Es wird jeweils

eine Körperpflegecreme mit guten Eigenschaften erhalten.

Beispiel 36: Zahnpasta

| | |
|---|---|
| Phase A | |
| 34,79 | dest. Wasser |
| 0,50 | Polymer aus Beispiel 1 (fest) |
| ad 100 | dest. Wasser |
| 0,30 | Konservierungsmittel |
| 20,00 | Glycerin |
| 0,76 | Natriummonofluorphosphat |
| Phase B | |
| 1,20 | Natriumcarboxymethylcellulose |
| Phase C | |
| 0,80 | Aromaöl |
| 0,06 | Saccharin |
| 0,10 | Konservierungsmittel |
| 0,05 | Bisabolol |
| 1,00 | Panthenol |
| 0,50 | Tocopherylacetat |
| 2,80 | Siliciumdioxid |
| 1,00 | Natriumlaurylsulfat |
| 7,90 | Dicalciumphosphat, wasserfrei |
| 25,29 | Dicalciumphosphat-Dihydrat |
| 0,45 | Titandioxid |

Herstellung:

Phase A lösen. Phase B in Phase A einstreuen und lösen. Phase C zugeben und unter Vakuum bei RT ca. 45 Min. rühren lassen.

Das Beispiel lässt sich jeweils mit den erfindungsgemäßen Polymeren 2 bis 20 wiederholen. Es wird jeweils eine Zahnpasta mit guten Eigenschaften erhalten.

Beispiel 37: Mundwasser

| | |
|---|---|
| Phase A | |
| 2,00 | Aromaöl |
| 4,00 | hydriertes Rizinusöl-PEG-40 |
| 1,00 | Bisabolol |
| 30,00 | Alkohol |
| Phase B | |
| 0,20 | Saccharin |
| 5,00 | Glycerin |
| q.s. | Konservierungsmittel |
| 5,00 | Poloxamer 407 |

(fortgesetzt)

| | |
|---|---|
| Phase B | |
| 0,50 | Polymer aus Beispiel 1 (fest) |
| ad 100 | dest. Wasser |

Herstellung:

Phase A und Phase B getrennt klar lösen. Phase B in Phase A einrühren.

Das Beispiel lässt sich jeweils mit den erfindungsgemäßen Polymeren 2 bis 20 wiederholen. Es wird jeweils ein Mundwasser mit guten Eigenschaften erhalten.

Beispiel 38: Prothesenhaftmittel

| | |
|---|---|
| Phase A | |
| 0,20 | Bisabolol |
| 1,00 | Betacarotin |
| q.s. | Aromaöl |
| 20,00 | Cetearyloctanoat |
| 5,00 | Siliciumdioxid |
| 33,80 | Mineralöl |
| | |
| Phase B | |
| 1,0 | Polymer aus Beispiel 1 (fest) |
| ad 100 | dest. Wasser |
| 35,00 | PVP (20 %ige Lösung in Wasser) |

Herstellung:

Phase A gut mischen. Phase B in Phase A einrühren.

Das Beispiel lässt sich jeweils mit den erfindungsgemäßen Polymeren 2 bis 20 wiederholen. Es wird jeweils ein Prothesenhaftmittel mit guten Eigenschaften erhalten.

Beispiel 39: Hautpflegecreme, Typ O/W

| | |
|---|---|
| Phase A | |
| 8,00 | Cetearylalkohol |
| 2,00 | Ceteareth-6 |
| 2,00 | Ceteareth-25 |
| 10,00 | Mineralöl |
| 5,00 | Cetearyloctanoat |
| 5,00 | Dimethicon |
| | |
| Phase B | |
| 0,50 | Polymer aus Beispiel 1 (fest) |
| ad 100 | dest. Wasser |
| 2,00 | Panthenol, Propylenglykol |
| q.s. | Konservierungsmittel |

(fortgesetzt)

| | |
|---|---|
| Phase C | |
| q.s. | Parfümöl |

Herstellung:

Phase A und B getrennt auf ca. 80°C erwärmen. Phase B in Phase A unter Homogenisieren einrühren, kurz nachhomogenisieren. Abkühlen auf ca. 40°C, Phase C hinzugeben, nochmals homogenisieren.

Das Beispiel lässt sich jeweils mit den erfindungsgemäßen Polymeren 2 bis 20 wiederholen. Es wird jeweils eine Hautpflegecreme, Typ O/W mit guten Eigenschaften erhalten.

Beispiel 40: Hautpflegecreme, Typ W/O

| | |
|---|---|
| Phase A | |
| 6,00 | hydriertes Rizinusöl-PEG-7 |
| 8,00 | Cetearyloctanoat |
| 5,00 | Isopropylmyristat |
| 15,00 | Mineralöl |
| 2,00 | PEG-45/Dodecyl Glycol Copolymer |
| 0,50 | Magnesiumstearat |
| 0,50 | Aluminumstearat |
| Phase B | |
| 3,00 | Glycerin |
| 0,60 | Polymer aus Beispiel 1 (fest) |
| ad 100 | dest. Wasser |
| 0,70 | Magnesiumsulfat |
| 2,00 | Panthenol |
| q.s. | Konservierungsmittel |
| Phase C | |
| 1,00 | Tocopherol |
| 5,00 | Tocopherylacetat |
| q.s. | Parfümöl |

Herstellung:

Die Phasen A und B getrennt auf ca. 80°C erwärmen. Phase B in Phase A einrühren und homogenisieren. Abkühlen auf ca. 40°C, Phase C hinzugeben und nochmals kurz homogenisieren.

Das Beispiel lässt sich jeweils mit den erfindungsgemäßen Polymeren 2 bis 20 wiederholen. Es wird jeweils eine Hautpflegecreme, Typ W/O mit guten Eigenschaften erhalten.

Beispiel 41: Lippenpflegecreme

| | |
|---|---|
| Phase A | |
| 10,00 | Cetearyloctanoat |
| 5,00 | Polybuten |

(fortgesetzt)

| | |
|---|---|
| Phase B | |
| 0,10 | Carbomer |
| | |
| Phase C | |
| 2,00 | Ceteareth-6 |
| 2,00 | Ceteareth-25 |
| 2,00 | Glycerylstearat |
| 2,00 | Cetylalkohol |
| 1,00 | Dimethicon |
| 1,00 | Benzophenone-3 |
| 0,20 | Bisabolol |
| 6,00 | Mineralöl |
| | |
| Phase D | |
| 1,50 | Polymer aus Beispiel 1 (fest) |
| ad 100 | dest. Wasser |
| 3,00 | Panthenol |
| 3,00 | Propylenglykol |
| q.s. | Konservierungsmittel |
| | |
| Phase E | |
| 0,10 | Triethanolamin |
| | |
| Phase F | |
| 0,50 | Tocopherylacetat |
| 0,10 | Tocopherol |
| q.s. | Parfümöl |

Herstellung:

Phase A klar lösen. Phase B dazugeben und homogenisieren. Phase C addieren und schmelzen bei 80°C. Phase D auf 80°C erwärmen. Phase D zu Phase ABC geben und homogenisieren. Abkühlen auf ca. 40°C, Phase E und Phase F zugeben, nochmals homogenisieren.

Das Beispiel lässt sich jeweils mit den erfindungsgemäßen Polymeren 2 bis 20 wiederholen. Es wird jeweils eine Lippenpflegecreme mit guten Eigenschaften erhalten.

Beispiel 42: Duschgel

| | |
|---|---|
| 50,00 | Sodium Laureth Sulfate, Magnesium Laureth Sulfate, Sodium Laureth-8 Sulfate, Magnesium Laureth-8 |
| 1,00 | Cocoamide DEA |
| 0,8 | Polymer aus Beispiel 1 (fest) |
| ad 100 | dest. Wasser |
| 2,00 | Sodium Laureth Sulfate, Glycol Distearate, Cocamide MEA, Laureth-10 |
| q.s. | Konservierungsmittel |
| q.s. | Parfümöl |
| 2,00 | Natriumchlorid |

Herstellung:

Alle Komponenten gemeinsam einwiegen und bis zur Lösung rühren.

Das Beispiel lässt sich jeweils mit den erfindungsgemäßen Polymeren 2 bis 20 wiederholen. Es wird jeweils ein Duschgel mit guten Eigenschaften erhalten.

Beispiel 43: Duschgel

| | |
|---|---|
| 30,00 | Sodium Laureth Sulfate |
| 6,00 | Sodium Cocoamphodiacetate |
| 6,00 | Cocamidopropylbetain |
| 3,00 | Sodium Laureth Sulfate, Glycol Distearate, Cocamide MEA, Laureth-10 |
| 7,70 | Polyquaternium-44 |
| 0,2 | Polymer aus Beispiel 1 (fest) |
| ad 100 | dest. Wasser |
| 1,00 | Panthenol |
| q.s. | Konservierungsmittel |
| q.s. | Parfümöl |
| q.s. | Citronensäure |
| 0,50 | Natriumchlorid |

Herstellung:

Die Komponenten der Phase A einwiegen und lösen. Den pH-Wert auf 6 bis 7 einstellen.

Das Beispiel lässt sich jeweils mit den erfindungsgemäßen Polymeren 2 bis 20 wiederholen. Es wird jeweils ein Duschgel mit guten Eigenschaften erhalten.

Beispiel 44: Klares Duschgel

| | |
|---|---|
| 40,00 | Sodium Laureth Sulfate |
| 5,00 | Decylglukosid |
| 5,00 | Cocamidopropylbetain |
| 0,50 | Polyquaternium-10 |
| 2,00 | Polymer aus Beispiel 1 (fest) |
| ad 100 | dest. Wasser |
| 1,00 | Panthenol |
| q.s. | Parfümöl |
| q.s. | Konservierungsmittel |
| q.s. | Citronensäure |
| 2,00 | Natriumchlorid |

Herstellung:

Die Komponenten der Phase A einwiegen und klar lösen.

Das Beispiel lässt sich jeweils mit den erfindungsgemäßen Polymeren 2 bis 20 wiederholen. Es wird jeweils ein klares Duschgel mit guten Eigenschaften erhalten.

Beispiel 45: Duschbad

| | |
|---|---|
| A | |
| 40,00 | Sodium Laureth Sulfate |

(fortgesetzt)

| | |
|---|---|
| A | |
| 5,00 | Sodium $C_{12-15}$ Pareth-15 Sulfonate |
| 5,00 | Decylglukosid |
| q.s. | Parfümöl |
| 0,10 | Phytantriol |
| B | |
| 0,1 | Guarhydroxypropyltrimoniumchlorid |
| 2,00 | Polymer aus Beispiel 1 (fest) |
| ad 100 | dest. Wasser |
| 1,00 | Panthenol |
| q.s. | Konservierungsmittel |
| 1,00 | Laureth-3 |
| q.s. | Citronensäure |
| 2,00 | Natriumchlorid |

Herstellung:

Die Komponenten der Phase A mischen. Die Komponenten der Phase B nacheinander zugeben und mischen. Den pH-Wert auf 6 bis 7 einstellen.

Das Beispiel lässt sich jeweils mit den erfindungsgemäßen Polymeren 2 bis 20 wiederholen. Es wird jeweils ein Duschbad mit guten Eigenschaften erhalten.

Beispiel 46: Flüssigseife

| | |
|---|---|
| A | |
| 43,26 | dest. Wasser |
| 0,34 | Aminomethylpropanol |
| 3,40 | Poly(Ethylacrylat/Methacrylsäure) (Luviflex®Soft, Fa. BASF) |
| B | |
| 40,00 | Sodium Laureth Sulfate |
| 10,00 | Cocamidopropylbetain |
| 0,2 | Polymer aus Beispiel 1 (fest) |
| ad 100 | dest. Wasser |
| q.s. | Parfümöl |
| q.s. | Konservierungsmittel |
| 2,00 | Natriumchlorid |

Herstellung:

Die Komponenten der Phase A einwiegen und klar lösen. Die Komponenten der Phase B nacheinander zugeben und mischen.

Das Beispiel lässt sich jeweils mit den erfindungsgemäßen Polymeren 2 bis 20 wiederholen. Es wird jeweils eine Flüssigseife mit guten Eigenschaften erhalten.

Beispiel 47: Flüssiges Fußbad

| | |
|---|---|
| A | |
| 1,00 | Nonoxynol-14 |
| 0,10 | Bisabolol |
| 1,00 | Pinienöl (Pinus Sylvestris) |
| B | |
| 5,00 | PEG-8 |
| 1,50 | Polymer aus Beispiel 1 (fest) |
| ad 100 | dest. Wasser |
| 0,50 | Triclosan |
| 30,00 | Sodium Laureth Sulfate |
| 3,00 | Polyquaternium-16 |
| q.s. | C. I. 19 140 + C. I. 42 051 |

Herstellung:

Phase A solubilisieren. Phase B mischen.

Das Beispiel lässt sich jeweils mit den erfindungsgemäßen Polymeren 2 bis 20 wiederholen. Es wird jeweils ein flüssiges Fußbad mit guten Eigenschaften erhalten.

Beispiel 48: Erfrischungsgel

| | |
|---|---|
| A | |
| 0,60 | Carbomer |
| 45,40 | dest. Wasser |
| B | |
| 0,50 | Bisabolol |
| 0,50 | Farnesol |
| q.s. | Parfümöl |
| 5,00 | PEG-40 Hydrogenated Castor Oil |
| 0,50 | Polymer aus Beispiel 1 (fest) |
| ad 100 | dest. Wasser |
| 1,00 | Tetrahydroxypropylethylendiamin |
| 1,50 | Menthol |
| 43,00 | Alkohol |
| q.s. | C. I. 74 180, Direct Blue 86 |

Herstellung:

Phase A quellen lassen. Phase B lösen. Phase B in Phase A einrühren.

Das Beispiel lässt sich jeweils mit den erfindungsgemäßen Polymeren 2 bis 20 wiederholen. Es wird jeweils ein Erfrischungsgel mit guten Eigenschaften erhalten.

Beispiel 49: Roll-on Antiperspirant

| | |
|---|---|
| A | |
| 0,40 | Hydroxyethylcellulose |
| 50,00 | dest. Wasser |

(fortgesetzt)

| | |
|---|---|
| A | |
| | |
| B | |
| 25,00 | Alkohol |
| 0,10 | Bisabolol |
| 0,30 | Farnesol |
| 2,00 | PEG-40 Hydrogenated Castor Oil |
| q.s. | Parfümöl |
| | |
| C | |
| 5,00 | Aluminumchlorhydrat |
| 3,00 | Propylenglykol |
| 3,00 | Dimethicon-Copolyol |
| 3,00 | Polyquaternium-16 |
| 1,50 | Polymer aus Beispiel 1 (fest) |
| ad 100 | dest. Wasser |

Herstellung:

Phase A quellen lassen. Phase B und C getrennt lösen. Phase A und B in Phase C einrühren.

Das Beispiel lässt sich jeweils mit den erfindungsgemäßen Polymeren 2 bis 20 wiederholen. Es wird jeweils ein Roll-on Antiperspirant mit guten Eigenschaften erhalten.

Beispiel 50: Transparenter Deostift

| | |
|---|---|
| 5,00 | Natriumstearat |
| 0,50 | Triclosan |
| 3,00 | Ceteareth-25 |
| 20,00 | Glycerin |
| 0,50 | Polymer aus Beispiel 1 (fest) |
| ad 100 | dest. Wasser |
| q.s. | Parfümöl |
| 60,00 | Propylenglykol |
| 0,20 | Bisabolol |

Herstellung:

Phase A zusammenwiegen, schmelzen und homogenisieren. Anschließend in die Form gießen.

Das Beispiel lässt sich jeweils mit den erfindungsgemäßen Polymeren 2 bis 20 wiederholen. Es wird jeweils ein transparenter Deostift mit guten Eigenschaften erhalten.

Beispiel 51: Wasserlösliches Badeöl

| | |
|---|---|
| 15,00 | Cetearyloctanoat |
| 15,00 | Capryl/Caprin-Triglycerid |
| 1,00 | Panthenol, Propylenglykol |
| 0,10 | Bisabolol |
| 2,00 | Tocopherylacetat |

(fortgesetzt)

| | |
|---|---|
| 2,00 | Retinylpalmitat |
| 0,10 | Tocopherol |
| 37,00 | PEG-7-Glyceryl-Cocoate |
| 0,4 | Polymer aus Beispiel 1 (fest) |
| ad 100 | dest. Wasser |
| q.s. | Parfümöl |
| 23,60 | PEG-40 Hydrogenated Castor Oil |

Herstellung:

Mischen und rühren bis alles klar gelöst ist.

Das Beispiel lässt sich jeweils mit den erfindungsgemäßen Polymeren 2 bis 20 wiederholen. Es wird jeweils ein wasserlösliches Badeöl mit guten Eigenschaften erhalten.

Beispiel 52: Tagespflege-Aerosol

| | |
|---|---|
| A | |
| 4,00 | Ethylhexyl Methoxycinnamate |
| 1,50 | Octocrylen |
| 9,00 | Capryl/Caprin-Triglycerid |
| 5,00 | Simmondsia Chinensis (Jojoba) Seed Oil |
| 1,50 | Cyclomethicon |
| 3,00 | Hydrogenated Coco-Glycerides |
| 1,00 | PVP/Hexadecen-Copolymer |
| 1,00 | Ceteareth-6, Stearylalkohol |
| B | |
| 5,00 | Zinkoxid |
| C | |
| 2,00 | Ceteareth-25 |
| 1,20 | Panthenol |
| 0,20 | Sodium Ascorbyl Phosphate |
| 0,30 | Imidazolidinyl-Harnstoff |
| 0,10 | Disodium EDTA |
| 1,50 | Polymer aus Beispiel 1 (fest) |
| ad 100 | dest. Wasser |
| D | |
| 0,50 | Tocopherylacetat |
| 0,20 | Bisabolol |
| 0,33 | Capryl/Caprin-Triglycerid, Retinol |
| q.s. | Parfümöl |

Herstellung:

Phase A auf 80°C erwärmen. Phase A klar lösen. Phase B einarbeiten und homogenisieren. Phase C zugeben, erhitzen auf 80°C, aufschmelzen und homogenisieren. Unter Rühren auf ca. 40°C abkühlen, Phase D hinzugeben und kurz homogenisieren. 90 % Wirkstofflösung: 10 % Propan/Butan mit 3,5 bar (20°C) abfüllen.

Das Beispiel lässt sich jeweils mit den erfindungsgemäßen Polymeren 2 bis 20 wiederholen. Es wird jeweils

ein Tagespflege-Aerosol mit guten Eigenschaften erhalten.

Beispiel 53: Feuchtigkeitscreme

| | |
|---|---|
| A | |
| 3,00 | Vitis Vinifera (Grape) Seed Oil |
| 1,00 | Cyclopentasiloxan, Cyclohexasiloxan |
| 1,50 | Cyclomethicon |
| 2,00 | Soybean (Glycine Soja) Oil |
| 2,00 | Ethylhexyl Methoxycinnamate |
| 1,00 | Uvinul®A Plus |
| 1,00 | Hydrogenated Lecithin |
| 1,00 | Cholesterol |
| 2,00 | PEG-40 Hydrogenated Castor Oil |
| 5,00 | Cetearyloctanoat |
| 5,00 | Capryl/Caprin-Triglycerid |
| B | |
| 3,00 | Capryl/Caprin-Triglycerid, Acrylat-Copolymer |
| C | |
| 2,50 | Polymer aus Beispiel 1 (fest) |
| ad 100 | dest. Wasser |
| 0,50 | Cocotrimoniummethsulfat |
| 2,00 | Panthenol, Propylenglykol |
| 3,00 | Glycerin |
| 0,10 | Dinatrium-EDTA |
| D | |
| 0,30 | Parfümöl |
| 0,30 | DMDM Hydantoin |
| 1,00 | Tocopherylacetat |
| 2,00 | Tocopherol |

Herstellung:

Phase A auf 80°C erwärmen. Phase B in Phase A einrühren. Phase C auf ca. 80°C erwärmen und unter Homogenisieren in Phase A+B einrühren. Unter Rühren auf ca. 40°C abkühlen, Phase D hinzugeben und kurz homogenisieren.

Das Beispiel lässt sich jeweils mit den erfindungsgemäßen Polymeren 2 bis 20 wiederholen. Es wird jeweils eine Feuchtigkeitscreme mit guten Eigenschaften erhalten.

Beispiel 54: Aerosol-Haarschaum

| | |
|---|---|
| A | |
| 2,00 | Cocotrimoniummethsulfat |
| 0,20 | Parfümöl |

(fortgesetzt)

| | |
|---|---|
| B. | |
| 1,60 | Polymer aus Beispiel 1 (fest) |
| ad 100 | dest. Wasser |
| 0,50 | Poly(Ethylacrylat/Methacrylsäure) (Luviflex®Soft) |
| 0,10 | Aminomethylpropanol |
| 0,20 | Ceteareth-25 |
| 0,20 | Trimethylsilylamodimethicon, Trideceth-10, Cetrimonium Chloride |
| 0,10 | PEG-25 PABA |
| 0,20 | Hydroxyethylcellulose |
| 0,20 | PEG-8 |
| 0,20 | Panthenol |
| 15,00 | Alkohol |
| | |
| C | |
| 10,00 | Propan/Butan 3,5 bar (20°C) |

Herstellung:

Phasen A und B mischen und mit Treibgas abfüllen.

Das Beispiel lässt sich jeweils mit den erfindungsgemäßen Polymeren 2 bis 20 wiederholen. Es wird jeweils ein Aerosol-Haarschaum mit guten Eigenschaften erhalten.

Beispiel 55: Pumpmousse

| | |
|---|---|
| A | |
| 2,00 | Cocotrimoniummethosulfat |
| q.s. | Parfümöl |
| | |
| C | |
| 7,00 | Polyquaternium-46 (10 %ige wässrige Lösung) |
| 2,50 | Polymer aus Beispiel 1 (fest) |
| ad 100 | dest. Wasser |
| 0,50 | PEG-8 |
| 1,00 | Panthenol |
| q.s. | Konservierungsmittel |
| 0,20 | PEG-25 PABA (ethoxylierte p-Aminobenzoesäure) |

Herstellung:

Die Komponenten der Phase A mischen. Die Komponenten der Phase B nacheinander zugeben und klar lösen.

Das Beispiel lässt sich jeweils mit den erfindungsgemäßen Polymeren 2 bis 20 wiederholen. Es wird jeweils ein Pumpmousse mit guten Eigenschaften erhalten.

Beispiel 56: Aerosolschaum

| | |
|---|---|
| 3,0 | Polymer aus Beispiel 1 (fest) |
| ad 100 | dest. Wasser |
| 5,00 | PVPNA-Copolymer (40 %ige wässrige Lösung) |

(fortgesetzt)

| | |
|---|---|
| 0,50 | Hydroxyethylcetyldimoniumphosphat |
| 0,20 | Ceteareth-25 |
| 0,40 | Parfümöl PC 910.781/Cremophor |
| q.s. | Konservierungsmittel |
| 10,00 | Propan/Butan 3,5 bar (20°C) |

Herstellung:

Alles zusammen wiegen, rühren bis gelöst, dann abfüllen.

Das Beispiel lässt sich jeweils mit den erfindungsgemäßen Polymeren 2 bis 20 wiederholen. Es wird jeweils ein Aerosolschaum mit guten Eigenschaften erhalten.

Beispiel 57: Farbstyling-Mousse

| | |
|---|---|
| A | |
| 2,00 | Cocotrimoniummethosulfat |
| q.s. | Parfümöl |
| B | |
| 6,50 | Polymer aus Beispiel 1 (fest) |
| ad 100 | dest. Wasser |
| 0,50 | Acrylatcopolymer (Luvimer®100 P, Fa. BASF) |
| 0,10 | Aminomethylpropanol |
| 0,20 | Ceteareth-25 |
| 0,20 | Panthenol |
| 0,20 | Hydroxyethylcellulose |
| 10,00 | Alkohol |
| 0,08 | C.I. 12245, Basic Red 76 |
| 0,05 | C.I. 42510, Basic Violet 14 |
| C | |
| 10,00 | Propan/Butan 3,5 bar (20°C) |

Herstellung:

Alles zusammen wiegen, rühren bis gelöst, dann abfüllen. Nur für dunkelblondes und braunes Haar geeignet!

Das Beispiel lässt sich jeweils mit den erfindungsgemäßen Polymeren 2 bis 20 wiederholen. Es wird jeweils ein Farbstyling-Mousse mit guten Eigenschaften erhalten.

Beispiel 58: Pumphaarschaum

| | |
|---|---|
| A | |
| 1,50 | Cocotrimoniummethosulfat |
| q.s. | Parfümöl |
| B | |
| 2,00 | Polymer aus Beispiel 1 (fest) |
| ad 100 | dest. Wasser |

(fortgesetzt)

| | |
|---|---|
| B | |
| | |
| C | |
| 0,46 | Aminomethylpropanol |
| 4,00 | PEG/PPG-25/25 Dimethicon/Acrylat-Copolymer |
| q.s. | Konservierungsmittel |

Herstellung:

Phase A mischen. Phase B in Phase A einrühren. Phase C zugeben und rühren bis gelöst.

Das Beispiel lässt sich jeweils mit den erfindungsgemäßen Polymeren 2 bis 20 wiederholen. Es wird jeweils ein Pumphaarschaum mit guten Eigenschaften erhalten.

Beispiel 59: Aquawax

| | |
|---|---|
| 10 | Polymer aus Beispiel 1 (fest) |
| ad 100 | dest. Wasser |
| q.s. | Parfümöl |
| q.s. | hydriertes Rizinusöl-PEG-40 |
| 0,10 | Diethylphthalat |
| 0,10 | Cetearylethylhexanoat |
| 0,10 | PEG-7 Glyceryl Cocoate |
| 0,10 | Konservierungsmittel |
| 2,00 | Capryl/Caprin-Triglycerid, Acrylat-Copolymer |

Herstellung:

Alles mischen und homogenisieren. 15 Minuten nachrühren.

Das Beispiel lässt sich jeweils mit den erfindungsgemäßen Polymeren 2 bis 20 wiederholen. Es wird jeweils ein Aquawax mit guten Eigenschaften erhalten.

Beispiel 60: Rinse-off Conditioner und Repair Treatment

| | |
|---|---|
| A | |
| 0,20 | Cetearyloctanoat |
| 0,10 | Phytantriol |
| 2,00 | hydriertes Rizinusöl-PEG-40 |
| | |
| B | |
| q.s. | Parfümöl |
| 2,00 | Cocotrimoniummethosulfat |
| | |
| C | |
| ad 100 | dest. Wasser |

(fortgesetzt)

| | |
|---|---|
| D | |
| 2,00 | Polyquaternium-16 (20 %ige wässrige Lösung) |
| 1,0 | Polymer aus Beispiel 1 (fest) |
| 1,00 | Dimethicon-Copolyol |
| q.s. | Konservierungsmittel |
| 10,00 | Alkohol |
| q.s. | Citronensäure |

Herstellung:

Die Phasen A und B getrennt mischen. Phase C in Phase B einrühren.

Das Beispiel lässt sich jeweils mit den erfindungsgemäßen Polymeren 2 bis 20 wiederholen. Es wird jeweils ein Rinse-off Conditioner und Repair Treatment mit guten Eigenschaften erhalten.

Beispiel 61: Haarkur

| | |
|---|---|
| A | |
| 2,00 | Ceteareth-6, Stearylalkohol |
| 1,00 | Ceteareth-25 |
| 6,00 | Cetearylalkohol |
| 6,00 | Cetearyloctanoat |
| 0,30 | Phytantriol |
| B | |
| 1,0 | Polymer aus Beispiel 1 (fest) |
| ad 100 | dest. Wasser |
| 0,70 | Guar Hydroxypropytrimonoium Chloride |
| 5,00 | Propylenglykol |
| 2,00 | Panthenol |
| 0,30 | Imidazolidinyl-Harnstoff |
| C | |
| 2,00 | Cosi Silk Soluble |
| 0,20 | Parfum |
| 0,50 | Phenoxyethanol |

Herstellung:

Die Phasen A und B getrennt auf ca 80°C erwärmen. Phase B homogenisieren.

Das Beispiel lässt sich jeweils mit den erfindungsgemäßen Polymeren 2 bis 20 wiederholen. Es wird jeweils eine Haarkur mit guten Eigenschaften erhalten.

Beispiel 62: Haar-Cocktail

| | |
|---|---|
| A | |
| 0,40 | Acrylate/$C_{10-30}$ Alkylacrylat-Crosspolymer |
| 2,00 | Dimethicon |
| 3,00 | Cyclomethicon, Dimethiconol |

(fortgesetzt)

| | |
|---|---|
| A | |
| 2,00 | Phenyltrimethicon |
| 2,00 | Amodimethicone, Cetrimonium Chloride, Trideceth-10 |
| 0,50 | Dimethicon-Copolyol |
| 1,00 | Macadamia-Nussöl (Ternifolia) |
| 0,50 | Tocopherylacetat |
| 1,00 | PEG-40 Hydrogenated Castor Oil |
| q.s. | Parfümöl |
| B | |
| 0,3 | Polymer aus Beispiel 1 (fest) |
| ad 100 | dest. Wasser |
| 0,46 | Aminomethylpropanol |
| 4,00 | PEG/PPG-25/25 Dimethicon/Acrylat-Copolymer |

Herstellung:

Die Komponenten der Phase A mischen. Phase B lösen. Phase B unter Homogenisieren in Phase A einrühren.
Das Beispiel lässt sich jeweils mit den erfindungsgemäßen Polymeren 2 bis 20 wiederholen. Es wird jeweils ein Haar-Cocktail mit guten Eigenschaften erhalten.

Beispiel 63: Dauerwelle

Well-Lösung

| | |
|---|---|
| A | |
| 0.20 | Cocamidopropylbetain |
| 0,20 | Polysorbate 20 |
| 1,55 | Polymer aus Beispiel 1 (fest) |
| ad 100 | dest. Wasser |
| 0,20 | Dinatrium-EDTA |
| 0,20 | Hydroxyethylcellulose |
| B | |
| 8,00 | Thioglykolsäure |
| C | |
| 11,00 | Ammoniumhydroxid |
| D | |
| 5,00 | Ammoniumcarbonat |

Herstellung:

Die Komponenten der Phase A einwiegen und klar lösen. Phase B in Phase A einrühren.
Das Beispiel lässt sich jeweils mit den erfindungsgemäßen Polymeren 2 bis 20 wiederholen. Es wird jeweils eine Well-Lösung mit guten Eigenschaften erhalten.

Beispiel 64: Fixierung:

| | |
|---|---|
| A | |
| 1,00 | PEG-40 Hydrogenated Castor Oil |
| 0,20 | Parfümöl |
| ad 100 | dest. Wasser |
| | |
| B | |
| 0,20 | Cocamidopropylbetain |
| 0,20 | Ceteareth-25 |
| 2,5 | Polymer aus Beispiel 1 (fest) |
| q.s. | Konservierungsmittel |
| | |
| C | |
| 2,30 | Wasserstoffperoxid |
| | |
| D | |
| q.s. | Phosphorsäure |

Herstellung:

Phase A solubilisieren. Die Komponenten der Phase B nacheinander zugeben und klar lösen.

Das Beispiel lässt sich jeweils mit den erfindungsgemäßen Polymeren 2 bis 20 wiederholen. Es wird jeweils eine Fixierung mit guten Eigenschaften erhalten.

Beispiel 65: dunkelbraune Permanenthaarfarbe (Oxidationshaarfarbe)

| | |
|---|---|
| A | |
| 0,20 | Natriumsulfit |
| 0,05 | Dinatrium-EDTA |
| 0,20 | p-Phenylendiamin |
| 0,30 | Resorcinol |
| 0,20 | 4-Amino-2-hydroxytoluol |
| 0,10 | m-Aminophenol |
| 1,50 | Oleylalkohol |
| 4,50 | Propylenglykol |
| 2,30 | Sodium $C_{12-15}$ Pareth-15 Sulfonate |
| 20,00 | Ölsäure |
| ad 100 | dest. Wasser |
| | |
| B | |
| 1,0 | Polymer aus Beispiel 1 (fest) |
| 13,70 | Ammoniumhydroxid |
| 6,00 | i-Propanol |
| q.s. | Parfum |

Herstellung:

Phase A solubilisieren. Die Komponenten der Phase B nacheinander zugeben und mischen.

Das Beispiel lässt sich jeweils mit den erfindungsgemäßen Polymeren 2 bis 20 wiederholen. Es wird jeweils eine dunkelbraune Permanenthaarfarbe (Oxidationshaarfarbe) mit guten Eigenschaften erhalten.

Beispiel 66: Entwickleremulsion (pH 3 -4 )

| | |
|---|---|
| 3,00 | Hexadecylalkohol |
| 1,0 | Polymer aus Beispiel 1 (fest) |
| ad 100 | dest. Wasser |
| 1,00 | Ceteareth-20 |
| 1,00 | Sodium $C_{12-15}$ Pareth-15 Sulfonate |
| 6,00 | Wasserstoffperoxid |
| 0,50 | Phosphorsäure |
| 0,01 | Acetanilid |

Herstellung:

Die Komponenten nacheinander zugeben und mischen.

Das Beispiel lässt sich jeweils mit den erfindungsgemäßen Polymeren 2 bis 20 wiederholen. Es wird jeweils eine Entwickleremulsion (pH 3 - 4) mit guten Eigenschaften erhalten.

Beispiel 67: hellbraune Semi-Permanenthaarfarbe

| | |
|---|---|
| 10,00 | Cocodiethanolamid |
| 4,00 | Natriumdodecylbenzylsulfonat, 50 %ig |
| 1,0 | Polymer aus Beispiel 1 (fest) |
| ad 100 | dest. Wasser |
| 6,00 | $C_{9-11}$ Pareth-3 |
| 2,50 | Natriumlaurylsulfat |
| 0,40 | 2-Nitro-p-phenylendiamin |
| 0,20 | HC Red No.3 |
| 0,20 | HC Yellow No.2 |

Herstellung:

Die Komponenten nacheinander zugeben und mischen.

Das Beispiel lässt sich jeweils mit den erfindungsgemäßen Polymeren 2 bis 20 wiederholen. Es wird jeweils eine hellbraune Semi-Permanenthaarfarbe mit guten Eigenschaften erhalten.

Beispiel 68: Shampoo

| | |
|---|---|
| 30,00 | Sodium Laureth Sulfate |
| 6,00 | Sodium Cocoamphoacetate |
| 6,00 | Cocamidopropylbetain |
| 3,00 | Sodium Laureth Sulfate, Glycol Distearate, Cocamide MEA, Laureth-10 |
| 1,0 | Polymer aus Beispiel 1 (fest) |
| 2,00 | Dimethicon |
| q.s. | Parfum |
| q.s. | Konservierungsstoff |

(fortgesetzt)

| | |
|---|---|
| q.s. | Citronensäure |
| 1,00 | Natriumchlorid |
| ad 100 | dest. Wasser |

Herstellung:

Komponenten einwiegen und lösen. pH-Wert auf 6 bis 7 einstellen.

Das Beispiel lässt sich jeweils mit den erfindungsgemäßen Polymeren 2 bis 20 wiederholen. Es wird jeweils ein Shampoo mit guten Eigenschaften erhalten.

Beispiel 69: Shampoo

| | |
|---|---|
| 30,00 | Sodium Laureth Sulfate |
| 6,00 | Sodium Cocoamphoacetate |
| 6,00 | Cocamidopropylbetain |
| 3,00 | Sodium Laureth Sulfate, Glykol Distearate, Cocamide MEA, Laureth-10 |
| 1,0 | Polymer aus Beispiel 1 (fest) |
| 2,00 | Amodimethicon |
| q.s. | Parfum |
| q.s. | Konservierungsstoff |
| q.s. | Citronensäure |
| 1,00 | Natriumchlorid |
| ad 100 | dest. Wasser |

Herstellung:

Komponenten einwiegen und lösen. pH-Wert auf 6 bis 7 einstellen.

Das Beispiel lässt sich jeweils mit den erfindungsgemäßen Polymeren 2 bis 20 wiederholen. Es wird jeweils ein Shampoo mit guten Eigenschaften erhalten.

Beispiel 70: Shampoo

| | |
|---|---|
| 40,00 | Sodium Laureth Sulfate |
| 10,00 | Cocamidopropylbetain |
| 3,00 | Sodium Laureth Sulfate, Glykol Distearate, Cocamide MEA, Laureth-10 |
| 1,0 | Polymer aus Beispiel 1 (fest) |
| 2,00 | Dow Corning 3052 |
| q.s. | Parfum |
| q.s. | Konservierungsstoff |
| q.s.. | Citronensäure |
| 2,00 | Cocamido DEA |
| ad 100 | dest. Wasser |

Herstellung:

Komponenten einwiegen und lösen. pH-Wert auf 6 bis 7 einstellen.

Das Beispiel lässt sich jeweils mit den erfindungsgemäßen Polymeren 2 bis 20 wiederholen. Es wird jeweils ein Shampoo mit guten Eigenschaften erhalten.

Beispiel 71: Antischuppen-Shampoo

| | |
|---|---|
| 40,00 | Sodium Laureth Sulfate |
| 10,00 | Cocamidopropylbetain |
| 10,00 | Disodium Laureth Sulfosuccinate |
| 2,50 | Sodium Laureth Sulfate, Glycol Distearate, Cocamide MEA, Laureth-10 |
| 1,0 | Polymer aus Beispiel 1 (fest) |
| 0,50 | Climbazol |
| q.s. | Parfum |
| q.s. | Konservierungsstoff |
| 0,50 | Natriumchlorid |
| ad 100 | dest. Wasser |

Herstellung:

Komponenten einwiegen und lösen. pH-Wert auf 6 bis 7 einstellen.

Das Beispiel lässt sich jeweils mit den erfindungsgemäßen Polymeren 2 bis 20 wiederholen. Es wird jeweils ein Antischuppen-Shampoo mit guten Eigenschaften erhalten.

Beispiel 72: Shampoo

| | |
|---|---|
| 25,00 | Sodium Laureth Sulfate |
| 5,00 | Cocamidopropylbetain |
| 2,50 | Sodium Laureth Sulfate, Glycol Distearate,Cocamide MEA, Laureth-10 |
| 1,0 | Polymer aus Beispiel 1 (fest) |
| q.s. | Parfum |
| q.s. | Konservierungsstoff |
| 2,00 | Cocamido DEA |
| ad 100 | dest. Wasser |

Herstellung:

Komponenten einwiegen und lösen. pH-Wert auf 6 bis 7 einstellen.

Das Beispiel lässt sich jeweils mit den erfindungsgemäßen Polymeren 2 bis 20 wiederholen. Es wird jeweils ein Shampoo mit guten Eigenschaften erhalten.

Beispiel 73: Shampoo

| | |
|---|---|
| 20,00 | Ammonium Laureth Sulfate |
| 15,00 | Ammonium Lauryl Sulfate |
| 5,00 | Cocamidopropylbetain |
| 2,50 | Sodium Laureth Sulfate, Glycol Distearate,Cocamide MEA, Laureth-10 |
| 1,0 | Polymer aus Beispiel 1 (fest) |
| q.s. | Parfum |
| q.s. | Konservierungsstoff |
| 0,50 | Natriumchlorid |
| ad 100 | dest. Wasser |

Herstellung:

Komponenten einwiegen und lösen. pH-Wert auf 6 bis 7 einstellen.

Das Beispiel lässt sich jeweils mit den erfindungsgemäßen Polymeren 2 bis 20 wiederholen. Es wird jeweils ein Shampoo mit guten Eigenschaften erhalten.

Beispiel 74: Klares Duschgel

| | |
|---|---|
| 40,00 | Sodium Laureth Sulfate |
| 5,00 | Decylglukosid |
| 5,00 | Cocamidopropylbetain |
| 1,0 | Polymer aus Beispiel 1 (fest) |
| 1,00 | Panthenol |
| q.s. | Parfum |
| q.s. | Konservierungsstoff |
| q.s. | Citronensäure |
| 2,00 | Natriumchlorid |
| ad 100 | dest. Wasser |

Herstellung:

Komponenten einwiegen und lösen. pH-Wert auf 6 bis 7 einstellen.

Das Beispiel lässt sich jeweils mit den erfindungsgemäßen Polymeren 2 bis 20 wiederholen. Es wird jeweils ein klares Duschgel mit guten Eigenschaften erhalten.

Beispiel 75: Shampoo

| | |
|---|---|
| 12,00 | Sodium Laureth Sulfate |
| 1,50 | Decylglukosid |
| 2,50 | Cocamidopropylbetain |
| 5,00 | Coco-Glucoside Glyceryl Oleate |
| 2,00 | Sodium Laureth Sulfate, Glycol Distearate,Cocamide MEA,Laureth-10 |
| 1,0 | Polymer aus Beispiel 1 (fest) |
| q.s. | Konservierungsstoff |
| q.s. | Sunset Yellow C. I. 15985 |
| q.s. | Parfum |
| 1,00 | Natriumchlorid |
| ad 100 | dest. Wasser |

Herstellung:

Komponenten einwiegen und lösen. pH-Wert auf 6 bis 7 einstellen.

Das Beispiel lässt sich jeweils mit den erfindungsgemäßen Polymeren 2 bis 20 wiederholen. Es wird jeweils ein Shampoo mit guten Eigenschaften erhalten.

Beispiel 76: Shampoo

| | |
|---|---|
| A | |
| 40,00 | Sodium Laureth Sulfate |
| 5,00 | Sodium $C_{12-15}$ Pareth-15 Sulfonate |
| 5,00 | Decylglukosid |
| q.s. | Parfum |
| 0,10 | Phytantriol |
| B | |
| 1,0 | Polymer aus Beispiel 1 (fest) |
| ad 100 | dest. Wasser |
| 1,00 | Panthenol |
| q.s. | Konservierungsstoff |
| 1,00 | Laureth-3 |
| q.s. | Citronensäure |
| 2,00 | Natriumchlorid |

Herstellung:

Komponenten der Phase A einwiegen und lösen. pH-Wert auf 6 bis 7 einstellen. Phase B zugeben und mischen.
Das Beispiel lässt sich jeweils mit den erfindungsgemäßen Polymeren 2 bis 20 wiederholen. Es wird jeweils ein Shampoo mit guten Eigenschaften erhalten.

**Patentansprüche**

1. Wässrige haut- oder haarkosmetische Zubereitung enthaltend wenigstens ein Polymer A, welches

   a) 70- 80 Gew.% wenigstens einen Ester der (Meth)acrylsäure oder Ethacrylsäure,
   b) wenigstens eine olefinisch ungesättigte, radikalisch polymerisierbare anionogene oder anionische Verbindung: bestehend aus Acrylsäure und Methacrylsäure,
   c) wenigstens eine, keine Silikongruppen enthaltende, olefinisch ungesättigte, radikalisch polymerisierbare urethangruppenhaltige Verbindung und
   d) gegebenenfalls weitere radikalisch polymerisierbare Verbindungen einpolymerisiert enthält.

2. Wässrige haut- oder haarkosmetische Zubereitung nach Anspruch 1, wobei Komponente a) ausgewählt ist aus der Gruppe bestehend aus Methyl(meth)acrylat, Ethyl(meth)acrylat, n-Propyl(meth)acrylat, i-Propyl(meth)-acrylat, Methylethacrylat, Ethylethacrylat, n-Propylethacrylat, i-Propylethacrylat, n-Butylethacrylat, tert.-Butylethacrylat, i-Butylethacrylat, n-Butyl(meth)acrylat, tert.-Butyl(meth)acrylat, i-Butyl(meth)acrylat, sec- Butyl(meth)acrylat, 2-Pentyl-(rneth)acrylat, 3-Pentyl(meth)acrylat, Isopentylacrylat, Neopentylacrylat, n-Octyl-(meth)acrylat, 1,1,3,3-Tetramethylbutyl(meth)acrylat, Ethylhexyl(meth)acrylat, n-Nonyl(meth)acrylat, n-Decyl(meth)acrylat, n-Undecyl(meth)acrylat, Tridecyl-(meth)acrylat, Myristyl(meth)acrylat, Pentadecyl(meth)acrylat, Palmityl(meth)-acrylat, Heptadecyl(meth)acrylat, Nonadecyl(meth)acrylat, Arrachinyl(meth)-acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl-(meth)acrylat, Linolenyl(meth)acrylat, Stearyl(meth)acrylat, Lauryl(meth)acrylat, Phenoxyethylacrylat, t-Butylcyclohexylacrylat, Cyclohexyl(meth)acrylat, Ureido(meth)acrylat, Tetrahydrofurfuryl(meth)acrylat und deren Mischungen.

3. Wässrige haut- oder haarkosmetische Zubereitung nach Anspruch 2, wobei Komponente a) ausgewählt ist aus der Gruppe bestehend aus Methylmethacrylat, Ethylmethacrylat, n-Propylmethacrylat, i-Propylmethacrylat, n-Butylmethacrylat, tert.-Butylmethacrylat, i-Butylmethacrylat, sec-Butylmethacrylat und deren Mischungen.

4. Wässrige haut- oder haarkosmetische Zubereitung nach einem der Ansprüche 1 bis 3, wobei Komponente c) ausgewählt ist aus urethangruppenhaltigen (Meth)acrylaten.

5. Wässrige haut- oder haarkosmetische Zubereitung nach einem der Ansprüche 1 bis 4, wobei das wenigstens eine Polymer A

a) 70-80 Gew.% der Komponente a),
b) 4-30 Gew.-% der Komponente b),
c) 0,1-20 Gew.% der Komponente c) und
d) 0-30 Gew.-% der Komponente d)
einpolymerisiert enthält, mit der Maßgabe, dass sich die Mengen der Komponenten a) bis d) zu 100 Gew.-% addieren.

**6.** Wässrige haut- oder haarkosmetische Zubereitung nach Anspruch 5, wobei das wenigstens eine Polymer A

a) 70-80 Gew.-% der Komponente a),
b) 10-30 Gew.% der Komponente b),
c) 0,5-10 Gew.% der Komponente c) und
d) 0-30 Gew.-% der Komponente d)
einpolymerisiert enthält, mit der Maßgabe, dass sich die Mengen der Komponenten a) bis d) zu 100 Gew.% addieren.

**7.** Wässrige haut- oder haarkosmetische Zubereitung nach einem der Ansprüche 1 bis 6, wobei das wenigstens eine Polymer A

a) 70-80 Gew.-% der Komponente a), ausgewählt aus der Gruppe bestehend aus Methylmethacrylat, Ethylmethacrylat und deren Mischungen,
b) 15-28 Gew.-% der Komponente b), bestehend aus Acrylsäure und Methacrylsäure,
c) 0,5-5 Gew.-% urethangruppenhaltige (Meth)acrylate
d) 0-30 Gew.-% der Komponente d)
einpolymerisiert enthält, mit der Maßgabe, dass sich die Mengen der Komponenten a) bis d) zu 100 Gew.% addieren.

**8.** Wässrige haut- oder haarkosmetische Zubereitung nach einem der Ansprüche 1 bis 7, wobei die Zubereitung außer Wasser wenigstens einen weiteren kosmetisch akzeptablen Träger B) aufweist, der ausgewählt ist unter

i. wassermischbaren organischen Lösungsmitteln, bevorzugt $C_2$-$C_4$-Alkanolen, besonders bevorzugt Ethanol,
ii. Ölen, Fetten, Wachsen,
iii. von ii) verschiedenen Estern von $C_6$-$C_{30}$-Monocarbonsäuren mit ein-, zwei- oder dreiwertigen Alkoholen,
iv. gesättigten acyclischen und cyclischen Kohlenwasserstoffen,
v. Fettsäuren,
vi. Fettalkoholen,
vii. Treibmitteln (Treibgasen) und
viii. Mischungen davon.

**9.** Wässrige haut- oder haarkosmetische Zubereitung nach einem der Ansprüche 1 bis 8 in Form eines Sprühproduktes, wobei die Zubereitung entweder in Kombination mit einer mechanischen Pumpsprühvorrichtung oder in Kombination mit mindestens einem Treibmittel ausgewählt aus der Gruppe bestehend aus Propan, Butan, Dimethylether, fluorierten Kohlenwasserstoffen und deren Mischungen vorliegt.

## Claims

**1.** An aqueous skin or hair cosmetic preparation comprising at least one polymer A which comprises, in copolymerized form,

a) 70-80% by weight of at least one ester of (meth)acrylic acid or ethacrylic acid,
b) at least one olefinically unsaturated, free-radically polymerizable, anionogenic or anionic compound: consisting of acrylic acid and methacrylic acid,
c) at least one olefinically unsaturated, free-radically polymerizable, urethane-group-containing compound which comprises no silicone groups and
d) optionally further free-radically polymerizable compounds.

**2.** The aqueous skin or hair cosmetic preparation according to claim 1, where component a) is chosen from the group

consisting of methyl (meth)acrylate, ethyl (meth)acrylate, n-propyl (meth)acrylate, isopropyl (meth)acrylate, methyl ethacrylate, ethyl ethacrylate, n-propyl ethacrylate, isopropyl ethacrylate, n-butyl ethacrylate, tert-butyl ethacrylate, isobutyl ethacrylate, n-butyl (meth)acrylate, tert-butyl (meth)acrylate, isobutyl (meth)acrylate, sec-butyl (meth)acrylate, 2-pentyl (meth)acrylate, 3-pentyl (meth)acrylate, isopentyl acrylate, neopentyl acrylate, n-octyl (meth)acrylate, 1,1,3,3-tetramethylbutyl (meth)acrylate, ethylhexyl (meth)acrylate, n-nonyl (meth)acrylate, n-decyl (meth)acrylate, n-undecyl (meth)acrylate, tridecyl (meth)acrylate, myristyl (meth)acrylate, pentadecyl (meth)acrylate, palmityl (meth) acrylate, heptadecyl (meth)acrylate, nonadecyl (meth)acrylate, arrachinyl (meth)acrylate, behenyl (meth)acrylate, lignocerenyl (meth)acrylate, cerotinyl (meth)acrylate, melissinyl (meth)acrylate, palmitoleinyl (meth)acrylate, oleyl (meth)acrylate, linolyl (meth)acrylate, linolenyl (meth)acrylate, stearyl (meth)acrylate, lauryl (meth)acrylate, phenoxyethyl acrylate, t-butylcyclohexyl acrylate, cyclohexyl (meth)acrylate, ureido (meth)acrylate, tetrahydrofurfuryl (meth) acrylate and mixtures thereof.

**3.** The aqueous skin or hair cosmetic preparation according to claim 2, where component a) is chosen from the group consisting of methyl methacrylate, ethyl methacrylate, n-propyl methacrylate, isopropyl methacrylate, n-butyl methacrylate, tert-butyl methacrylate, isobutyl methacrylate, sec-butyl methacrylate and mixtures thereof.

**4.** The aqueous skin or hair cosmetic preparation according to one of claims 1 to 3, where component c) is chosen from urethane-group-containing (meth)acrylates.

**5.** The aqueous skin or hair cosmetic preparation according to one of claims 1 to 4, where the at least one polymer A comprises, in copolymerized form,

a) 70-80% by weight of component a),
b) 4-30% by weight of component b),
c) 0.1-20% by weight of component c) and
d) 0-30% by weight of component d),
with the proviso that the amounts of the components a) to d) add up to 100% by weight.

**6.** The aqueous skin or hair cosmetic preparation according to claim 5, where the at least one polymer A comprises, in copolymerized form,

a) 70-80% by weight of component a),
b) 10-30% by weight of component b),
c) 0.5-10% by weight of component c) and
d) 0-30% by weight of component d),
with the proviso that the amounts of the components a) to d) add up to 100% by weight.

**7.** The aqueous skin or hair cosmetic preparation according to one of claims 1 to 6, where the at least one polymer A comprises, in copolymerized form,

a) 70-80% by weight of component a) chosen from the group consisting of methyl methacrylate, ethyl methacrylate and mixtures thereof,
b) 15-28% by weight of component b), consisting of acrylic acid and methacrylic acid,
c) 0.5-5% by weight of urethane-group-containing (meth)acrylates
d) 0-30% by weight of component d),
with the proviso that the amounts of the components a) to d) add up to 100% by weight.

**8.** The aqueous skin or hair cosmetic preparation according to one of claims 1 to 7, where the preparation, apart from water, has at least one further cosmetically acceptable carrier B) which is chosen from

i. water-miscible organic solvents, preferably $C_2$-$C_4$-alkanols, particularly preferably ethanol,
ii. oils, fats, waxes,
iii. esters of $C_6$-$C_{30}$-monocarboxylic acids with mono-, di- or trihydric alcohols, which are different from ii),
iv. saturated acyclic and cyclic hydrocarbons,
v. fatty acids,
vi. fatty alcohols,
vii. propellants (propellant gases) and
viii. mixtures thereof.

9. The aqueous skin or hair cosmetic preparation according to one of claims 1 to 8 in the form of a spray product, where the preparation is present either in combination with a mechanical pump spray device or in combination with at least one propellant chosen from the group consisting of propane, butane, dimethyl ether, fluorinated hydrocarbons and mixtures thereof.

## Revendications

1. Composition cosmétique aqueuse pour la peau ou les cheveux, contenant au moins un polymère A, qui contient

a) 70-80% en poids d'au moins un ester de l'acide (méth)acrylique ou de l'acide éthacrylique,
b) au moins un composé anionogène ou anionique, oléfiniquement insaturé, polymérisable par voie radicalaire constitué par l'acide acrylique et méthacrylique,
c) au moins un composé contenant des groupes uréthane, ne contenant pas de groupe de type silicone, oléfiniquement insaturé, polymérisable par voie radicalaire et
d) le cas échéant d'autres composés polymérisables par voie radicalaire, sous forme copolymérisée.

2. Composition cosmétique aqueuse pour la peau ou les cheveux selon la revendication 1, le composant a) étant choisi dans le groupe constitué par le (méth)acrylate de méthyle, le (méth)acrylate d'éthyle, le (méth)acrylate de n-propyle, le (méth)-acrylate d'i-propyle, l'éthacrylate de méthyle, l'éthacrylate d'éthyle, l'éthacrylate de n-propyle, l'éthacrylate d'i-propyle, l'éthacrylate de n-butyle, l'éthacrylate de tert-butyle, l'éthacrylate d'i-butyle, le (méth)acrylate de n-butyle, le (méth)acrylate de tert-butyle, le (méth)acrylate d'i-butyle, le (méth)acrylate de sec-butyle, le (méth) acrylate de 2-pentyle, le (méth)acrylate de 3-pentyle, l'acrylate d'isopentyle, l'acrylate de néopentyle, le (méth) acrylate de n-octyle, le (méth)acrylate de 1,1,3,3-tétraméthylbutyle, le (méth)acrylate d'éthylhexyle, le (méth)acrylate de n-nonyle, le (méth)acrylate de n-décyle, le (méth)acrylate de n-undécyle, le (méth)acrylate de tridécyle, le (méth) acrylate de myristyle, le (méth)acrylate de pentadécyle, le (méth)acrylate de palmityle, le (méth)acrylate d'heptadécyle, le (méth)acrylate de nonadécyle, le (méth)acrylate d'arachinyle, le (méth)acrylate de béhényle, le (méth) acrylate de lignocérényle, le (méth)acrylate de cérotinyle, le (méth)acrylate de mélissinyle, le (méth)acrylate de palmitoléinyle, le (méth)acrylate d'oléyle, le (méth)acrylate de linoléyle, le (méth)acrylate de linolényle, le (méth) acrylate de stéaryle, le (méth)acrylate de lauryle, l'acrylate de phénoxyéthyle, l'acrylate de t-butylcyclohexyle, le (méth)acrylate de cyclohexyle, l'uréido(méth)acrylate, le (méth)acrylate de tétrahydrofurfuryle et leurs mélanges.

3. Composition cosmétique aqueuse pour la peau ou les cheveux selon la revendication 2, le composant a) étant choisi dans le groupe constitué par le méthacrylate de méthyle, le méthacrylate d'éthyle, le méthacrylate de n-propyle, le méthacrylate d'i-propyle, le méthacrylate de n-butyle, le méthacrylate de tert-butyle, le méthacrylate d'i-butyle, le méthacrylate de sec-butyle et leurs mélanges.

4. Composition cosmétique aqueuse pour la peau ou les cheveux selon l'une quelconque des revendications 1 à 3, le composant c) étant choisi parmi les (méth)acrylates contenant des groupes uréthane.

5. Composition cosmétique aqueuse pour la peau ou les cheveux selon l'une quelconque des revendications 1 à 4, ledit au moins un polymère A contenant, sous forme copolymérisée

a) 70-80% en poids de composant a)
b) 4-30% en poids de composant b),
c) 0,1-20% en poids de composant c) et
d) 0-30% en poids de composant d)

sous réserve que les quantités des composants a) à d) s'ajoutent à 100% en poids.

6. Composition cosmétique aqueuse pour la peau ou les cheveux selon la revendication 5, ledit au moins un polymère A contenant, sous forme copolymérisée

a) 70-80% en poids de composant a)
b) 10-30% en poids de composant b),
c) 0,5-10% en poids de composant c) et
d) 0-30% en poids de composant d)

sous réserve que les quantités des composants a) à d) s'ajoutent à 100% en poids.

**7.** Composition cosmétique aqueuse pour la peau ou les cheveux selon l'une quelconque des revendications 1 à 6, ledit au moins un polymère A contenant, sous forme copolymérisée

a) 70-80% en poids de composant a), choisi dans le groupe constitué par le méthacrylate de méthyle, le méthacrylate d'éthyle et leurs mélanges,
b) 15-28% en poids de composant b), constitué par l'acide acrylique et l'acide méthacrylique,
c) 0,5-5% en poids de (méth)acrylates contenant des groupes uréthane
d) 0-30% en poids de composant d)

sous réserve que les quantités des composants a) à d) s'ajoutent à 100% en poids.

**8.** Composition cosmétique aqueuse pour la peau ou les cheveux selon l'une quelconque des revendications 1 à 7, la composition présentant, outre de l'eau, au moins un autre support B) cosmétiquement acceptable, qui est choisi parmi

i. les solvants organiques miscibles à l'eau, de préférence les $C_2$-$C_4$-alcanols, de manière particulièrement préférée l'éthanol,
ii. les huiles, les graisses, les cires,
iii. les esters différents de ii) d'acides monocarboxyliques en $C_6$-$C_{30}$ avec des alcools monovalents, divalents ou trivalents,
iv. les hydrocarbures acycliques et cycliques saturés,
v. les acides gras,
vi. les alcools gras,
vii. les agents propulseurs (gaz propulseurs) et
viii. leurs mélanges.

**9.** Composition cosmétique aqueuse pour la peau ou les cheveux selon l'une quelconque des revendications 1 à 8 sous forme d'un produit à pulvériser, la composition se trouvant soit en combinaison avec un dispositif de pulvérisation à pompe mécanique, soit en combinaison avec au moins un agent propulseur, choisi dans le groupe constitué par le propane, le butane, les hydrocarbures fluorés et leurs mélanges.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 97000664 A
- DE 19838852 A
- EP 0203161 A
- WO 9700664 A
- US 3479328 A
- US 3674838 A
- GB 1443715 A
- EP 0036813 A
- DE 4007146 A
- DE 2726041 A
- US 4260703 A
- US 4481093 A
- JP 63297369 B
- JP 59157112 B
- EP 0903363 A
- EP 0942022 A
- EP 1002818 A
- WO 0172862 A
- WO 04050888 A
- WO 9806783 A
- DE 4434554 A1
- WO 04067599 A
- US 5240835 A
- WO 04052843 A
- WO 9425537 A
- DE 10246112 A
- WO 0039183 A
- WO 9732917 A
- EP 1035144 A
- DE 4333238 A
- DE 3929973
- DE 2150557
- DE 2817369
- DE 3708451
- DE 3314742 A
- EP 1084696 A
- DE PS1165574 C
- DE PS2024051 C
- EP 934956 A


### In der Beschreibung aufgeführte Nicht-Patentliteratur


- Chemistry and Technology of UV- and EB-Formulations for Coatings, Inks and Paints. SITA Technology, 1991, vol. 11, 73-123
- Macromolecules. Plenum Press, 1984, vol. 2
- **S.P. PAPPAS.** *J. Rad. Cur.,* Juli 1987, 6
- **KARL-HEINZ SCHRADER.** Grundlagen und Rezepturen der Kosmetika. Verlag Hüthig, 319-355
- Kosmetik und Hygiene von Kopf bis Fuß. Wiley-VCH, 2004, 235-236
- **VOLUME 4.** International Cosmetic Ingredient Dictionary and Handbook. The Cosmetic, Toiletry, and Fragrance Association, 2002
- **SCHRADER.** Grundlagen und Rezepturen der Kosmetika. Auflage, 1989
- **FIKENTSCHER.** *Cellulosechemie,* 1932, vol. 13, 58-64